# EUROPEAN PATENT APPLICATION

(11) **EP 4 674 845 A1**
(43) Date of publication of application: **07.01.2026**
(21) Application number: 24797441.3
(22) Date of filing: 25.04.2024
(51) Int. Cl.: C07D 311/88, C07D 405/12, C07D 491/147, C07D 491/22, C07D 471/16, C07D 471/22, C07F 7/08, C07D 219/08, C07H 19/073, C12Q 1/6897

(54) **NOVEL COMPOUND AND USE THEREOF**

(30) Priority: 28.04.2023 KR 20230056134; 01.09.2023 KR 20230116049; 24.04.2024 KR 20240054823
(71) Applicant: SFC Co., Ltd., Cheongju-si, Chungcheongbuk-do 28122 (KR)
(72) Inventor: LEE, Do Min, Cheongju-si, Chungcheongbuk-do 28122 (KR); MASANTA, Goutam, Cheongju-si, Chungcheongbuk-do 28122 (KR); EUM, Min Su, Cheongju-si, Chungcheongbuk-do 28122 (KR); KANG, Ji Soo, Cheongju-si, Chungcheongbuk-do 28122 (KR); SI, Ho Young, Cheongju-si, Chungcheongbuk-do 28122 (KR); KOLAY, Tina, Cheongju-si, Chungcheongbuk-do 28122 (KR); JANG, Eun Chong, Cheongju-si, Chungcheongbuk-do 28122 (KR); LIM, Hyun Ju, Cheongju-si, Chungcheongbuk-do 28122 (KR); SONG, Ju Man, Cheongju-si, Chungcheongbuk-do 28122 (KR); KIM, Seo Young, Cheongju-si, Chungcheongbuk-do 28122 (KR); CHOI, Sang Don, Cheongju-si, Chungcheongbuk-do 28122 (KR); SAHU, Sudipkumar, Cheongju-si, Chungcheongbuk-do 28122 (KR)
(74) Representative: BCKIP Part mbB
(86) International application number: PCT/KR2024/005627
(87) International publication number: WO 2024/225787

(57) **Abstract**

The present invention relates to a novel compound and uses thereof, and more specifically, to a compound capable of labeling biomolecules (such as nucleic acids and proteins), a composition for labeling or detecting biomolecules containing the compound, a support for labeling or detecting biomolecules containing the compound, and a method of labeling or detecting biomolecules using the compound.

## Description

### BACKGROUND

### 1. Field of the Invention

The present invention relates to a novel compound and uses thereof, and more specifically, to a compound capable of labeling biomolecules (such as nucleic acids and proteins), a composition for labeling or detecting biomolecules containing the compound, a support for labeling or detecting biomolecules containing the compound, and a method of labeling or detecting biomolecules using the compound.

### 2. Discussion of Related Art

Fluorescent dyes are used in molecular biology, cell biology, and molecular genetics as detection labels for detecting biomolecules *in vivo* and *in vitro,* or for observing biological phenomena at the cellular level.

Labeling nucleic acids, proteins, and the like with fluorescent dyes and using them as detection labels has been widely applied in various fields. In particular, fluorescently labeled oligonucleotides are advantageously used in a variety of analyses, including DNA sequencing, fluorescence in situ hybridization (FISH), hybridization analysis for nucleic acid arrays, microarrays, and nucleic acid amplification analysis including real-time polymerase chain reaction (PCR).

### SUMMARY OF THE INVENTION

The present invention is directed to providing novel compounds that can be widely used in molecular biology, cell biology, and genetics for identifying biomolecules (for example, nucleic acids, proteins, and the like), or for detecting or diagnosing analytes.

The present invention is also directed to providing a composition for labeling or detecting biomolecules containing the compound, a support for labeling or detecting biomolecules containing the compound, and a method of labeling or detecting biomolecules using the compound, as various uses of the novel compound defined herein.

According to the prevent invention to solve the above technical problems, there is provided a compound represented by Chemical Formula 1 below. 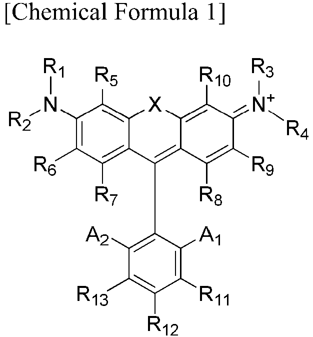
R₁ to R₄ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C3-C40 cycloalkyloxy, optionally substituted C5-C40 aryloxy, optionally substituted C2-C40 heteroaryloxy, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, Rₓ and Rₛ,
R₅ to R₁₃ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C40 cycloalkyloxy, optionally substituted C5-C40 aryloxy, optionally substituted C2-C40 heteroaryloxy, optionally substituted C5-C50 aryl, optionally substituted C2-C50 heteroaryl, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, Rₓ and Rₛ,
optionally, any two adjacent groups among R₁ to R₁₃ are bonded to each other to form an optionally substituted aliphatic ring, an optionally substituted aromatic ring, or an optionally substituted aliphatic-aromatic mixed ring,
A₁ and A₂ are each independently selected from hydrogen, halogen, optionally substituted C1-C10 alkyl, optionally substituted C3-C10 cycloalkyl, optionally substituted C1-C10 heterocycloalkyl, optionally substituted C1-C10 heteroalkyl, optionally substituted C2-C10 alkenyl, optionally substituted C2-C10 alkynyl, optionally substituted C1-C10 alkoxy, optionally substituted C5-C40 aryloxy, and cyano, provided that A₁ and A₂ are not simultaneously hydrogen,
Rₓ is a reactive group, or a group comprising a backbone including a hydrocarbon having 1 to 40 carbon atoms to which at least one reactive group is bound,
the reactive group being selected from carboxyl, carboxyl derivatives, carboxylate (-CO₂⁻), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkyne, haloacetamide, imido ester, glycol, halotriazine, hydrazine, acyl halide, alkyl halide, and aryl halide,
Rₛ is a carrier molecule, or a group comprising a backbone including a hydrocarbon having 1 to 40 carbon atoms to which at least one carrier molecule is bound,
at least one of R₁ to R₁₃ is Rₓ or Rₛ,
X is O, S, CRₐR_{b}, NRₐ, or SiRₐR_{b},
and Rₐ and R_{b} are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted C5-C50 aryl, and optionally substituted C2-C50 heteroaryl, or Rₐ and R_{b} are bonded to each other to form an optionally substituted aliphatic ring, an optionally substituted aromatic ring, or an optionally substituted aliphatic-aromatic mixed ring.

More specifically, according to the prevent invention, there is provided a compound represented by Chemical Formula 2 below. 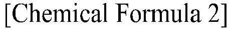 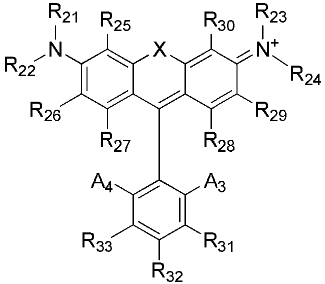 wherein
R₂₁ to R₂₄ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C3-C40 cycloalkyloxy, optionally substituted C5-C40 aryloxy, optionally substituted C2-C40 heteroaryloxy, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, and -L₃-R₃₆,
R₂₅ to R₃₃ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C40 cycloalkyloxy, optionally substituted C5-C40 aryloxy, optionally substituted C2-C40 heteroaryloxy, optionally substituted C5-C50 aryl, optionally substituted C2-C50 heteroaryl, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, and -L₃-R₃₆,
optionally, any two adjacent groups among R₂₁ to R₃₃ are bonded to each other to form an optionally substituted aliphatic ring, an optionally substituted aromatic ring, or an optionally substituted aliphatic-aromatic mixed ring,
A₃ and A₄ are each independently selected from hydrogen, halogen, optionally substituted C1-C10 alkyl, optionally substituted C3-C10 cycloalkyl, optionally substituted C1-C10 heterocycloalkyl, optionally substituted C1-C10 heteroalkyl, optionally substituted C2-C10 alkenyl, optionally substituted C2-C10 alkynyl, optionally substituted C1-C10 alkoxy, optionally substituted aryloxy, and cyano, provided that A₃ and A₄ are not simultaneously hydrogen,
X is O, S, CRₐR_{b}, NRₐ, or SiRₐR_{b},
at least one of R₂₁ to R₃₃ is -L₃-R₃₆,
L₃ is a linker comprising a hydrocarbon having 1 to 40 carbon atoms,
R₃₆ is a nucleoside selected from Chemical Formulas 3 to 5: 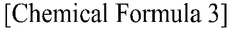 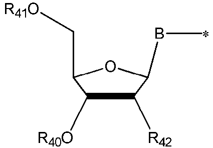 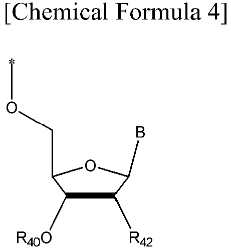 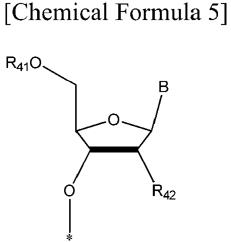 wherein
   * denotes a position at which the nucleoside is bonded to L₃,
   B is a nucleobase,
   R₄₀ is selected from hydrogen, -P(OR₄₃)(N(R₄₄R₄₅)), and -L₄-R₄₆,
   R₄₁ is an alcohol protecting group, hydrogen, or -P(OR₄₃)(N(R₄₄R₄₅)), or a nucleic acid or an optionally substituted support,
   R₄₃ to R₄₅ are each independently selected from hydrogen, optionally substituted C1-C10 alkyl, and optionally substituted C1-C10 heteroalkyl,
   L₄ is a single bond, or selected from an internucleotide phosphodiester bond, optionally substituted C1-C10 alkyl, and optionally substituted C1-C10 heteroalkyl,
   R₄₆ is hydroxy, -P(OR₄₃)(N(R₄₄R₄₅)), or a nucleic acid or an optionally substituted support,
   R₄₂ is selected from hydrogen, hydroxy, alkoxy, and -ORₚ,
   and Rₚ is a protecting group.

In another aspect of the present invention, there is provided a conjugate (for example, a nucleotide conjugate, a probe, or a primer) comprising a compound represented by Chemical Formula 1 or Chemical Formula 2 as a labeling reporter.

In yet another aspect of the present invention, there is provided a method for detecting nucleic acid, the method comprising: (a) preparing a reaction mixture comprising a target nucleic acid, reagents necessary to amplify the target nucleic acid, and the nucleotide conjugate defined herein; (b) amplifying the target nucleic acid in the reaction mixture; and (c) measuring fluorescence intensity of the reaction mixture.

The novel compound according to the present invention has the advantage of improved detectability even when a target biomolecule is present at a low concentration in a sample, as it exhibits a lower limit of detection (LOD) compared to conventional commercially available fluorescent substances.

Accordingly, the novel compound according to the present invention may be applied to the field of labeling and detecting biomolecules (for example, nucleic acids or proteins) as a substitute for conventional commercially available fluorescent substances.

### BRIEF DESCRIPTION OF THE DRAWINGS

FIG. 1 is a graph showing the results of Real-Time PCR performed in duplicate using a dual-labeled probe according to EX 1-1 and EX 1-2 for a BQCV (black queen cell virus) target.
FIG. 2 is a graph showing the results of Real-Time PCR performed in duplicate using a dual-labeled probe according to EX 1-3 and EX 1-4 for a BQCV (black queen cell virus) target.
FIG. 3 is a graph showing the results of Real-Time PCR performed in duplicate using a dual-labeled probe according to EX 1-5 and EX 1-6 for a BQCV (black queen cell virus) target.
FIG. 4 is a graph showing the results of Real-Time PCR performed in duplicate using a dual-labeled probe according to EX 1-7 and EX 1-8 for a BQCV (black queen cell virus) target.
FIGS. 5 to 12 are standard curves of Real-Time PCR experiment results obtained using dual-labeled probes according to EX 1-1 to EX 1-8, respectively.
FIG. 13 is a graph showing the results of Real-Time PCR performed in duplicate using a dual-labeled probe according to EX 2-1 and EX 2-2 for a BQCV (black queen cell virus) target.
FIG. 14 is a graph showing the results of Real-Time PCR performed in duplicate using a dual-labeled probe according to EX 2-3 and EX 2-4 for a BQCV (black queen cell virus) target.
FIG. 15 is a graph showing the results of Real-Time PCR performed in duplicate using a dual-labeled probe according to EX 2-5 and EX 2-6 for a BQCV (black queen cell virus) target.
FIG. 16 is a graph showing the results of Real-Time PCR performed in duplicate using a dual-labeled probe according to EX 2-7 and EX 2-8 for a BQCV (black queen cell virus) target.
FIGS. 17 to 24 are standard curves of Real-Time PCR experiment results obtained using dual-labeled probes according to EX 2-1 to EX 2-8, respectively.

### DETAILED DESCRIPTION OF EXEMPLARY EMBODIMENTS

For ease of understanding the present invention, certain terms are defined herein for convenience. Unless otherwise defined herein, scientific and technical terms used in the present invention shall have meanings as generally understood by those of ordinary skill in the art.

In addition, unless specifically indicated by the context, singular terms also include their plural forms, and plural terms may also include their singular forms.

Furthermore, unless otherwise defined herein, any functional group shall be construed according to the definitions commonly used in the technical field to which the present invention pertains.

### Novel compound

According to one aspect of the present invention, a novel compound represented by the following Chemical Formula 1 is provided. 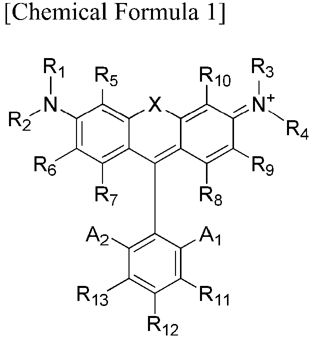 wherein,
R₁ to R₄ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C3-C40 cycloalkyloxy, optionally substituted C5-C40 aryloxy, optionally substituted C2-C40 heteroaryloxy, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, Rₓ and Rₛ,
R₅ to R₁₃ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C40 cycloalkyloxy, optionally substituted C5-C40 aryloxy, optionally substituted C2-C40 heteroaryloxy, optionally substituted C5-C50 aryl, optionally substituted C2-C50 heteroaryl, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, Rₓ and Rₛ,
optionally, any two adjacent groups among R₁ to R₁₃ are bonded to each other to form an optionally substituted aliphatic ring, an optionally substituted aromatic ring, or an optionally substituted aliphatic-aromatic mixed ring,
A₁ and A₂ are each independently selected from hydrogen, halogen, optionally substituted C1-C10 alkyl, optionally substituted C3-C10 cycloalkyl, optionally substituted C1-C10 heterocycloalkyl, optionally substituted C1-C10 heteroalkyl, optionally substituted C2-C10 alkenyl, optionally substituted C2-C10 alkynyl, optionally substituted C1-C10 alkoxy, optionally substituted C5-C40 aryloxy, and cyano, provided that A₁ and A₂ are not simultaneously hydrogen,
Rₓ is a reactive group, or a group comprising a backbone including a hydrocarbon having 1 to 40 carbon atoms to which at least one reactive group is bound,
the reactive group being selected from carboxyl, carboxyl derivatives, carboxylate (-CO₂⁻), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkyne, haloacetamide, imido ester, glycol, halotriazine, hydrazine, acyl halide, alkyl halide, and aryl halide,
Rₛ is a carrier molecule, or a group comprising a backbone including a hydrocarbon having 1 to 40 carbon atoms to which at least one carrier molecule is bound,
at least one of R₁ to R₁₃ is Rₓ or Rₛ,
X is O, S, CRₐR_{b}, NRₐ, or SiRₐR_{b},
and Rₐ and R_{b} are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted C5-C50 aryl, and optionally substituted C2-C50 heteroaryl, or Rₐ and R_{b} are bonded to each other to form an optionally substituted aliphatic ring, an optionally substituted aromatic ring, or an optionally substituted aliphatic-aromatic mixed ring.

Herein, Rₓ and Rₛ may comprise the following Structure 1: 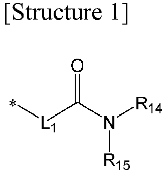 wherein
* denotes a position at which Structure 1 is bonded to the compound represented by Chemical Formula 1,
R₁₄ and R₁₅ are each independently selected from hydrogen, optionally substituted C1-C20 alkyl, optionally substituted C1-C20 heteroalkyl, optionally substituted C2-C20 alkenyl, optionally substituted C2-C20 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C20 cycloalkyloxy, optionally substituted C5-C20 aryloxy, optionally substituted C2-C20 heteroaryloxy, optionally substituted C5-C20 aryl, optionally substituted C2-C20 heteroaryl, thiol, optionally substituted C5-C20 aralkyl, optionally substituted C1-C20 alkylthio, optionally substituted C5-C20 arylthio, optionally substituted C3-C20 cycloalkylthio, optionally substituted C2-C20 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, and thioether,
or R₁₄ and R₁₅ are bonded to each other to form an optionally substituted aliphatic ring, an optionally substituted aromatic ring, or an optionally substituted aliphatic-aromatic mixed ring,
and L₁ is a single bond or a linker comprising a hydrocarbon having 1 to 20 carbon atoms.

At least one of R₁₄ and R₁₅ comprises a reactive group selected from carboxyl, carboxyl derivatives, carboxylate (-CO₂⁻), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkyne, haloacetamide, imido ester, glycol, halotriazine, hydrazine, acyl halide, alkyl halide, and aryl halide.

In case L₁ is a single bond, one of R₁₄ and R₁₅ may be hydrogen.

Also, Rₓ and Rₛ may comprise the following Structure 2: 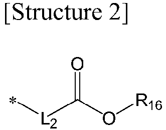 wherein
* denotes a position at which Structure 2 is bonded to the compound represented by Chemical Formula 1,
R₁₆ is selected from hydrogen, optionally substituted C1-C20 alkyl, optionally substituted C1-C20 heteroalkyl, optionally substituted C2-C20 alkenyl, optionally substituted C2-C20 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C20 cycloalkyloxy, optionally substituted C5-C20 aryloxy, optionally substituted C2-C20 heteroaryloxy, optionally substituted C5-C20 aryl, optionally substituted C2-C20 heteroaryl, thiol, optionally substituted C5-C20 aralkyl, optionally substituted C1-C20 alkylthio, optionally substituted C5-C20 arylthio, optionally substituted C3-C20 cycloalkylthio, optionally substituted C2-C20 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, and thioether,
and L₂ is a linker comprising a hydrocarbon having 1 to 20 carbon atoms.

Hereinafter, certain functional groups mentioned herein will be specifically described. Functional groups not mentioned below shall be construed according to general definitions commonly used in the technical field to which the present invention pertains.

As used herein, the term "optionally substituted" means that a given functional group may be unsubstituted, or may be substituted with at least one substituent, provided that such substitution does not interfere with or undesirably alter the efficacy of the compounds defined herein. The term "optionally substituted" is a general expression commonly used in the relevant technical field for defining functional groups, and unless otherwise defined herein, the scope of any functional group interpreted by the term "optionally substituted" shall be construed according to the general definitions commonly accepted in the technical field to which the present invention pertains.

When any functional group defined herein is a substituted functional group, any carbon contained in the functional group may be substituted with at least one substituent.

For example, the substituent may be selected from C1-C40 alkyl, C1-C40 heteroalkyl, C2-C40 alkenyl, C2-C40 alkynyl, C3-C20 cycloalkyl, C3-C20 cycloalkenyl, C2-C20 heterocycloalkyl, hydroxy, oxido (-O⁻), optionally substituted C1-C40 alkoxy, C3-C40 cycloalkyloxy, C5-C40 aryloxy, C2-C40 heteroaryloxy, optionally substituted C5-C50 aryl, optionally substituted C2-C50 heteroaryl, thiol, C5-C50 aralkyl, C1-C40 alkylthio, C5-C40 arylthio, C3-C40 cycloalkylthio, C2-C40 heteroarylthio, acylamino, acyloxy, sulfo group, ammonium, sulfonate ester, sulfonamide, ester, aminocarbonyl, nitroso (-N=O), halogen, silyl, amide, optionally substituted carbamate, phospho group, nitrile, acetal, ketal, amino, aminothiocarbonyl, aminocarbonyl-oxy, aminosulfonyl-oxy, amidino, sulfonyl-oxy, isonitrile, cyanate, imide, iminium, ether, thioether, Rx, and Rs.

As used herein, hydrogen may be protium (¹H), deuterium (²H), or tritium (³H).

As used herein, the term "hetero atom" refers to any atom other than carbon and hydrogen, and more specifically, a hetero atom is an atom that can be used to replace a carbon atom in a carbon (more specifically, hydrocarbon)-based backbone structure. Examples of hetero atoms include nitrogen, oxygen, sulfur, phosphorus, silicon, and selenium.

Rₓ is a reactive group, or a group comprising a backbone including a hydrocarbon having 1 to 40 carbon atoms to which at least one reactive group is bound.

The backbone refers to a series of bonds for linking a reactive group or a carrier molecule to the scaffold represented by Chemical Formula 1.

For example, the backbone may have an alkyl or heteroalkyl containing 1 to 40 carbon atoms as a main chain. In addition, the main chain may include a plurality of alkyl or heteroalkyl groups connected via an intermediate functional group such as an amide or an ester.

The backbone may comprise at least one hetero atom selected from O, S, N, P, and Si. The backbone may be linear or non-linear and may be composed of any combination of single, double, and triple bonds.

The backbone may, for example, be selected from alkyl, alkenyl, aryl, heteroaryl, cycloalkyl, heterocycloalkyl, -C(O)-, -C(O)O-, -NR_{L}-, -O-, -S-, -C(O)NR_{L}-, -S(O)_{O}-, - S(O)NR_{L}-, -S(O₂)NR_{L}-, -P(O)_{P}-, and Structures (a) to (o) below, or may be composed of a combination thereof.

| | | | |
|---|---|---|---|
| (a) | -(CH₂)_{q}- | | 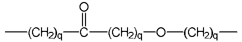 |
| (b) | -(CH₂)_{q}-O-(CH₂)_{q}- | (j) | |
| (c) | 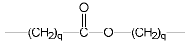 | (k) | 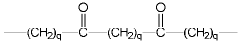 |
| (d) | 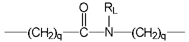 | (l) | 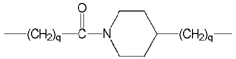 |
| (e) | 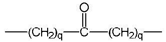 | (m) | 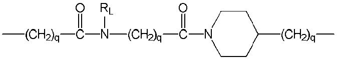 |
| (f) | 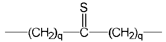 | | |
| (g) | -(CH₂)_{q}-S-(CH₂)_{q}- | (n) | 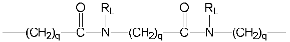 |
| (h) | -(CH₂)_{q}-O-(CH₂)_{q}-O-(CH₂)_{q}- | (o) | 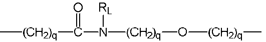 |
| (i) | -(CH₂)_{q}-S-(CH₂)_{q}-S-(CH₂)_{q}- | | |

Herein, R_{L} is selected from hydrogen, C1-C40 alkyl, and C1-C40 heteroalkyl; q is the same or different at each occurrence and is independently an integer from 0 to 10; o is an integer from 1 to 2; and p is an integer from 1 to 4.

The reactive group is a functional group selected from carboxyl, carboxyl derivatives, carboxylate (-CO₂⁻), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkyne, haloacetamide, imido ester, glycol, halotriazine, hydrazine, acyl halide, alkyl halide, and aryl halide.

If necessary, the reactive group may be protected so as not to participate in a reaction by the presence of a protecting group.

The protecting group is introduced by chemically modifying the reactive group in order to impart reaction selectivity of at least a portion of the reactive groups during a sequence of chemical or biological reaction processes.

Examples of the protecting group include hydroxyl protecting groups, amino protecting groups, carbonyl protecting groups, carboxyl protecting groups, thiol protecting groups, and phosphate protecting groups. In addition, unless otherwise defined herein, a functional group other than the above exemplified protecting groups that can be introduced to and removed from a specific reactive group may also be used as the protecting group.

For example, when the reactive group is hydroxyl, the protecting group may be selected from: alkanoyl groups such as acetyl, benzoyl, pivaloyl, chloroacetyl, trifluoroacetyl, and methoxyacetyl; alkoxycarbonyl groups such as benzyloxycarbonyl, ethoxycarbonyl, methoxybenzyloxycarbonyl, tert-butoxycarbonyl, diphenylmethoxycarbonyl, and 2,2,2-trichloroethyloxycarbonyl; aralkyl groups such as benzyl, nitrobenzyl, methoxybenzyl, trityl, methoxytrityl, 4,4-dimethoxytrityl (DMT), and diphenylmethyl; alkyl groups such as tert-butyl, methoxymethyl, methoxyethyl (MOE), methyl, 2,2,2-trichloroethyl, 2-trimethylsilylethyl, methylthiomethyl, benzyloxymethyl, 2-(trimethylsilyl)-ethoxymethyl, and 9-fluorenylmethyl; ether groups such as methoxymethyl ether (MOM), benzyl ether, tert-butyldimethylsilyl ether, p-methoxybenzyl ether (PMB), p-methoxyphenyl ether, β-methoxyethoxymethyl ether (MEM), methylthiomethyl ether, trimethylsilyl ether, methyl ether, and ethoxyethyl ether; ester groups such as acetate ester, methanesulfonate ester, and tert-butyl carbonate; silyl groups such as tert-butyldiphenylsilyl, trimethylsilyl, and triisopropylsilyl; sulfonyl groups such as methanesulfonyl; allyl; tetrahydropyranyl (THP); tetrahydrofuran (THF); or other groups described below.

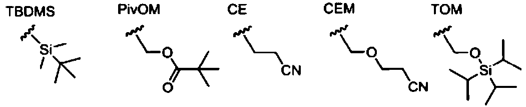

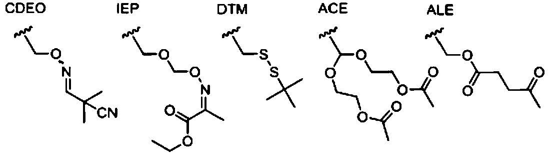

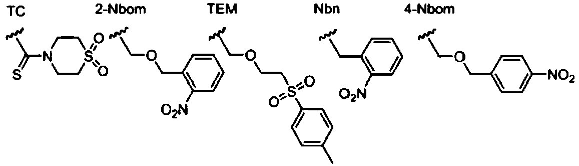

In addition, when the reactive group is amino, the protecting group may be selected from: carbamate groups such as tert-butyl carbamate and benzyl carbamate; amide groups such as acetamide, p-toluenesulfonamide, and diphenylphosphoramide; phthalimide group; carbonyl groups such as 9-fluorenylmethoxycarbonyl (Fmoc), tert-butoxycarbonyl (Boc), tert-amyloxycarbonyl, benzyloxycarbonyl (Cbz), 4-methoxybenzyloxycarbonyl, methoxycarbonyl, ethoxycarbonyl, nitrobenzyloxycarbonyl, 2,2,2-trichloroethyloxycarbonyl, aryloxycarbonyl, isobornyloxycarbonyl, and adamantyloxycarbonyl; sulfonyl groups such as 4-methylphenylsulfonyl, 2,4-dinitrobenzenesulfonyl, and 2-trimethylsilylethanesulfonyl (SES); aralkyl groups such as benzyl, trityl, 1,1-bis-(4-methoxyphenyl)methyl, 2,4-dimethoxybenzyl (DMB), and 4-methoxybenzyl (PMB); silyl groups such as trimethylsilyl (TMS) and tert-butyldimethylsilyl (TBS); alkanoyl groups such as acetyl, trifluoroacetyl, phthalyl, pivaloyl, and benzoyl; allyl; 2-nitrophenylsulfenyl; or other groups described below.

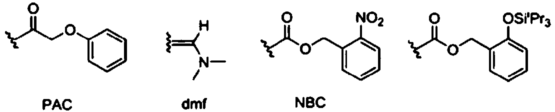

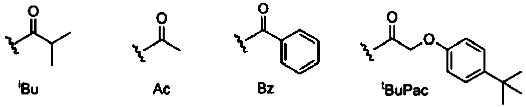

When the reactive group is carbonyl, the protecting group may be an acetal group such as dimethyl acetal, 1,3-dioxane, or thioacetal; a ketal group such as dimethyl ketal or thioketal; an oxime group such as O-methyloxime; or a hydrazone group such as N,N-dimethylhydrazone or an analog thereof.

When the reactive group is carboxyl, the protecting group may be an ester group such as methyl ester, allyl ester, or benzyl ester; an amide group such as N,N-dimethylamide; an alkyl group such as methyl, ethyl, tert-butyl, phenylsulfonylethyl, cyanoethyl, 2-trimethylsilylethyl, 2-trimethylsilylethoxymethyl, or nitroethyl; an aralkyl group such as benzyl; an aryl group such as phenyl; an allyl group; or an analog thereof.

When the reactive group is thiol, the protecting group may be an ether group such as benzylthioether; an ester group such as thioacetate ester, thiocarbonate, or thiocarbamate; or an analog thereof.

When the reactive group is phosphate, the protecting group may be an alkyl group such as tert-butyl, methyl, ethyl, cyanoethyl, trimethylsilylethyl, triphenylsilylethyl, or 2,2,2-trichloroethyl; an alkenyl group such as ethenyl, propenyl, butenyl, 2-cyanobutenyl, or 1-ethyl-2-butenyl; a cycloalkyl group such as cyclopropyl, cyclobutyl, or cyclohexyl; an aralkyl group such as benzyl, α-naphthylmethyl, trityl, dimethylphenyl, chlorobenzyl, or nitrobenzyl; an aryl group such as phenyl, naphthyl, methylphenyl, dimethylphenyl, or chlorophenyl; an allyl group; or an analog thereof.

Additional examples of protecting groups may be found in the following references: PROTECTIVE GROUPS IN ORGANIC SYNTHESIS, Third Edition, John Wiley & Sons, Inc., 1999; and https://en.wikipedia.org/wiki/Protecting_group.

As used herein, a Ca-Cb functional group refers to a functional group having a carbon number of a to b. For example, a Ca-Cb alkyl refers to a saturated aliphatic group having a carbon number of a to b, including straight-chain and branched alkyl groups. The straight or branched alkyl group may have 40 or fewer carbon atoms in its main chain (for example, C1-C10 straight-chain, C3-C10 branched).

Specifically, the alkyl group may be methyl, ethyl, n-propyl, i-propyl, n-butyl, s-butyl, i-butyl, t-butyl, pent-1-yl, pent-2-yl, pent-3-yl, 3-methylbut-1-yl, 3-methylbut-2-yl, 2-methylbut-2-yl, 2,2,2-trimethyleth-1-yl, n-hexyl, n-heptyl, or n-octyl.

As used herein, an alkoxy refers to both -O-(alkyl) groups and -O-(unsubstituted cycloalkyl) groups, which are hydrocarbons having one or more ether bonds and 1 to 10 carbon atoms in a straight or branched chain.

Specifically, the alkoxy group may include, but is not limited to, methoxy, ethoxy, propoxy, isopropoxy, n-butoxy, tert-butoxy, sec-butoxy, n-pentoxy, n-hexoxy, 1,2-dimethylbutoxy, cyclopropyloxy, cyclobutyloxy, cyclopentyloxy, and cyclohexyloxy.

As used herein, amino groups may be classified as primary, secondary, or tertiary amino groups depending on the number of hydrogen atoms bound to the nitrogen atom. In addition, if necessary, the amino group may also be provided in the form of a quaternary ammonium, which refers to a case where the nitrogen atom of the amino group carries a positive (+) charge.

Non-limiting examples of phospho groups usable herein include optionally substituted phosphine, optionally substituted phosphinate, optionally substituted phosphonate, optionally substituted phosphite, optionally substituted phosphate, phosphoric acid, phosphorous acid, and hypophosphorous acid, but are not limited thereto. The phospho group may be an optionally substituted phosphonate, an optionally substituted phosphate, and/or phosphoric acid.

Non-limiting examples of sulfo groups usable herein include optionally substituted sulfonyl, sulfonium, sulfate, sulfonic acid, optionally substituted sulfonate, optionally substituted sulfinate, sulfite, and optionally substituted sulfoxide, but are not limited thereto. The sulfo group may be sulfonic acid and/or an optionally substituted sulfonate.

As used herein, halogen refers to fluoro (-F), chloro (-Cl), bromo (-Br), or iodo (-I), and haloalkyl refers to an alkyl substituted with one or more of the above-described halogens. For example, halomethyl refers to a methyl group in which at least one hydrogen atom of methyl is replaced with a halogen (-CH₂X, -CHX₂, or -CX₃).

As used herein, aralkyl refers to a functional group in which an aryl group is substituted on a carbon of an alkyl group, and is a general term for -(CH₂)ₙAr. Examples of aralkyl include benzyl (-CH₂C₆H₅) and phenethyl (-CH₂CH₂C₆H₅).

As used herein, aryl, unless otherwise defined, refers to an unsaturated aromatic ring comprising a single ring or multiple rings (preferably 1 to 4 rings) fused together or linked by covalent bonds. Non-limiting examples of aryl include phenyl, biphenyl, o-terphenyl, m-terphenyl, p-terphenyl, 1-naphthyl, 2-naphthyl, 1-anthryl, 2-anthryl, 9-anthryl, 1-phenanthrenyl, 2-phenanthrenyl, 3-phenanthrenyl, 4-phenanthrenyl, 9-phenanthrenyl, 1-pyrenyl, 2-pyrenyl, and 4-pyrenyl.

As used herein, heteroaryl refers to a functional group in which one or more carbon atoms in the above-defined aryl group are substituted with non-carbon atoms such as nitrogen, oxygen, or sulfur. Non-limiting examples of heteroaryl include furyl, tetrahydrofuryl, pyrrolyl, pyrrolidinyl, thienyl, tetrahydrothienyl, oxazolyl, isoxazolyl, triazolyl, thiazolyl, isothiazolyl, pyrazolyl, pyrazolidinyl, oxadiazolyl, thiadiazolyl, imidazolyl, imidazolinyl, pyridyl, pyridazinyl, triazinyl, piperidinyl, morpholinyl, thiomorpholinyl, pyrazinyl, piperazinyl, pyrimidinyl, naphthyridinyl, benzofuranyl, benzothienyl, indolyl, indolinyl, indolizinyl, indazolyl, quinolizinyl, quinolyl, isoquinolyl, cinnolinyl, phthalazinyl, quinazolinyl, quinoxalinyl, pteridinyl, quinuclidinyl, carbazolyl, acridinyl, phenazinyl, phenothiazinyl, phenoxazinyl, purinyl, benzimidazolyl, benzothiazolyl, and conjugated analogs thereof.

As used herein, cycloalkyl or heterocycloalkyl (a hydrocarbon ring containing one or more hetero atoms) refers, unless otherwise defined, to a ring structure of an alkyl or heteroalkyl group, respectively.

Non-limiting examples of cycloalkyl include cyclopentyl, cyclohexyl, 1-cyclohexenyl, 3-cyclohexenyl, and cycloheptyl. Non-limiting examples of heterocycloalkyl include 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothiophen-2-yl, tetrahydrothiophen-3-yl, 1-piperazinyl, and 2-piperazinyl.

As used herein, cycloalkene or cycloalkyne refers, unless otherwise defined, to a ring structure of an alkyl group having at least one unsaturated bond (double or triple bond). At least one carbon atom of the cycloalkene or cycloalkyne may be substituted with a hetero atom. The cycloalkene or cycloalkyne may have 3 to 30, or 3 to 20 carbon atoms.

In addition, the cycloalkyl or heterocycloalkyl group may be conjugated with, or covalently linked to, another cycloalkyl, heterocycloalkyl, aryl, or heteroaryl group.

As used herein, a derivative refers to a compound that is similar to another compound, obtained by chemically modifying a part of the compound.

A carboxyl derivative refers to a functional group that can be converted to a carboxyl group, and may be an N-hydroxysuccinimide ester, N-hydroxybenzotriazole ester, acyl halide, acyl imidazole, thioester, alkyl ester, alkenyl ester, alkynyl ester, or aromatic ester.

The carboxyl derivative may be optionally substituted, and examples thereof include trifluoromethyl ester, pentafluorophenyl ester, p-nitrophenyl ester, tetrafluorophenyl ester, sulfo-succinimidyl ester, sulfo-dichlorophenyl ester, and sulfo-tetrafluorophenyl ester.

As used herein, a 1,2,4,5-tetrazine derivative may be 3,6-dimethyl-1,2,4,5-tetrazine, 3,6-diphenyl-1,2,4,5-tetrazine, or 3-methyl-6-phenyl-1,2,4,5-tetrazine.

As used herein, a cycloalkyne derivative refers to a compound that participates in a bipolar cycloaddition reaction, an inverse-electron demand Diels-Alder reaction, or a strain-promoted azide-alkyne cycloaddition (SPAAC) reaction. Examples of cycloalkyne derivatives that participate in SPAAC reactions include cyclooctynes such as OCT, COMBO (ALO), MOFO, DIFO, DIBO, BARAC, DIBAC (ADIBO), DBCO, DIMAC, BCN, or TMTH.

In addition, examples of any derivatives defined herein may be found in publications known in the relevant technical field.

As used herein, a dienophile refers to an alkene or alkyne capable of undergoing a Diels-Alder reaction with a diene, and specifically may be an alkene or alkyne in which an electron-withdrawing group is directly attached to a sp²-hybridized or sp-hybridized carbon.

As used herein, an electron-withdrawing group refers to a functional group that tends to attract electrons through an inductive effect or a resonance effect, and may also be referred to as a deactivating group. Examples of electron-withdrawing groups include trifluoromethylsulfonyl (-SO₂CF₃), substituted or unsubstituted ammonium (-NR₃⁺), nitro, sulfonic acid (-SO₃H), sulfonyl (-SO₂R), nitrile, trihalomethyl (-CF₃, -CCl₃, -CBr₃, -CI₃), haloformyl (-COCl, -COBr, -COI), formyl (-CHO), acyl (-COR), carboxyl (-CO₂H), substituted ester (-CO₂R), substituted or unsubstituted aminocarbonyl (-CONR₂), and nitroso (-N=O). Unless otherwise defined herein, the electron-withdrawing group may further include other functional groups that exhibit a tendency to attract electrons, in addition to those exemplified above.

R₁ to R₁₃ may independently be any of the functional groups defined above, or two adjacent groups among R₁ to R₁₃ may be bonded to each other to form an optionally substituted aliphatic ring, an optionally substituted aromatic ring, or an optionally substituted aliphatic-aromatic mixed ring, as long as the properties of the compound defined herein are maintained.

When two adjacent groups among R₁ to R₁₃ form an aliphatic or aromatic ring, the ring may be, for example, a single ring composed of four-membered, five-membered, six-membered, or larger (typically, seven- to ten-membered) atoms, or a fused ring in which the single ring is fused. The ring may also be a mixed ring containing both aliphatic and aromatic rings. The aliphatic ring and aromatic ring may be fused together or connected by a single bond. In addition, the ring may comprise at least one hetero atom.

Herein, optionally, at least one pair selected from R₁ and R₅, R₂ and R₆, R₃ and R₁₀, and R₄ and R₉ may be bonded to each other to form an optionally substituted aliphatic ring, an optionally substituted aromatic ring, or an optionally substituted aliphatic-aromatic mixed ring. Also, optionally, at least one pair selected from R₁ and R₂, and R₃ and R₄ may be bonded to each other to form an optionally substituted aliphatic ring, an optionally substituted aromatic ring, or an optionally substituted aliphatic-aromatic mixed ring.

Rₐ and R_{b} may each independently be any of the functional groups defined above, or Rₐ and R_{b} are bonded to each other to form an optionally substituted aliphatic ring, an optionally substituted aromatic ring, or an optionally substituted aliphatic-aromatic mixed ring, as long as the properties of the compound defined herein are maintained.

When Rₐ and R_{b} are bonded to each other to form an aliphatic or aromatic ring, the ring may be, for example, a single ring composed of four-membered, five-membered, six-membered, or larger (typically seven- to ten-membered) atoms, or a fused ring in which the single ring is fused. The ring may also be a mixed ring containing both aliphatic and aromatic rings. The aliphatic and aromatic rings may be fused together or connected by a single bond. In addition, the ring may comprise at least one hetero atom.

L₁ and L₂ may each be a linker comprising a hydrocarbon containing 1 to 40 carbon atoms.

The linker (including L₃) may have a main chain of alkyl or heteroalkyl containing carbon atoms. The main chain may further comprise multiple alkyl or heteroalkyl groups connected through an intermediate functional group such as an amide or ester.

The linker may include at least one hetero atom selected from O, S, N, P, and Si. The linker may be linear or non-linear, and may comprise any combination of single, double, and triple bonds.

Examples of the linker include alkyl, alkenyl, aryl, heteroaryl, cycloalkyl, heterocycloalkyl, -C(O)-, -C(O)O-, -NR_{L}-, -O-, -S-, -C(O)NR_{L}-, -S(O)_{O}-, -S(O)NR_{L}-, - S(O₂)NR_{L}-, -P(O)_{P}-, and those selected from structural formulas (a) to (o), or combinations thereof.

| | | | |
|---|---|---|---|
| (a) | -(CH₂)_{q}- | (j) | 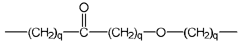 |
| (b) | -(CH₂)_{q}-O-(CH₂)_{q}- | | |
| (c) | 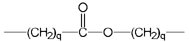 | (k) | 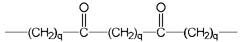 |
| (d) | 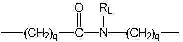 | (l) | 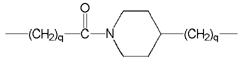 |
| (e) | 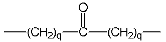 | (m) | 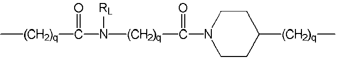 |
| (f) | 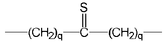 | | |
| (g) | -(CH₂)_{q}-S-(CH₂)_{q}- | (n) | 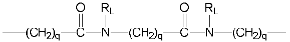 |
| (h) | -(CH₂)_{q}-O-(CH₂)_{q}-O-(CH₂)_{q}- | (o) | 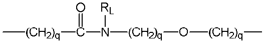 |
| (i) | -(CH₂)_{q}-S-(CH₂)_{q}-S-(CH₂)_{q}- | | |

Herein, R_{L} is selected from hydrogen, C1-C40 alkyl, and C1-C40 heteroalkyl, q is the same or different for each occurrence and is independently an integer from 0 to 10, o is an integer from 1 to 2, and p is an integer from 1 to 4.

Rₛ is a carrier molecule or a group comprising at least one carrier molecule bound to a backbone comprising a hydrocarbon containing 1 to 40 carbon atoms, and the definition of the backbone is the same as that of Rₓ.

The carrier molecule refers to a substance that carries a compound represented by Chemical Formula 1.

More specifically, the carrier molecule may be an amino acid, a polymer of amino acids, a peptide, a protein, a neurotoxin, phallotoxin, cytokine, toxin, protease substrate, protein kinase substrate, enzyme, antibody, antibody fragment, lectin, glycoprotein, histone, albumin, lipoprotein, avidin, streptavidin, protein A, protein G, protein L, phycobiliprotein, fluorescent protein, hormone, growth factor, nucleic acid base, nucleoside, nucleotide, nucleic acid polymer, nucleotide analog, nucleoside analog, nucleoside triphosphate, deoxynucleoside triphosphate (dNTP), dideoxynucleoside triphosphate (ddNTP), organic or inorganic nanoparticle, organic or inorganic microparticle, hapten, carbohydrate, polysaccharide, lipid, ion complexing moiety such as crown ether, PEG group, or an organic or inorganic polymer.

The carrier molecule may also be protected with a protecting group so as not to participate in any reaction. In this case, "the carrier molecule being protected with a protecting group" means that a functional group present on the carrier molecule is protected with the protecting group, and the definition of the protecting group for the carrier molecule is the same as that of the protecting group for the reactive group described above.

The compound represented by Chemical Formula 1 may further comprise a counter ion. The counter ion is an organic or inorganic anion and may be appropriately selected in consideration of the solubility and stability of the compound.

Examples of counter ions of the compound according to one embodiment of the present invention include inorganic acid anions such as hexafluorophosphate ion, halide ion, phosphate ion, perchlorate ion, periodate ion, hexafluoroantimonate ion, hexafluorostannate ion, tetrafluoroborate ion, and tetrafluoride ion, and organic acid anions such as thiocyanate ion, benzenesulfonate ion, naphthalenesulfonate ion, p-toluenesulfonate ion, alkylsulfonate ion, benzoate ion, alkylcarboxylate ion, trihaloalkylcarboxylate ion, alkylsulfonate ion, trihaloalkylsulfonate ion, and nicotinate ion.

Additionally, metal compound ions such as bisphenyldithiol chelate, thiobisphenol chelate and bisdiol-α-diketone, metal ions such as sodium and potassium, and quaternary ammonium salts may also be selected as counter ions.

More specifically, according to the present invention, a novel compound represented by Chemical Formula 2 below is provided. Unless otherwise defined herein, in the compound represented by Chemical Formula 2, the definitions of structures and functional groups corresponding to those in the compound represented by Chemical Formula 1 are the same as those of Chemical Formula 1. 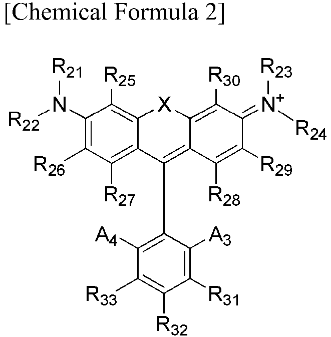 wherein
R₂₁ to R₂₄ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C3-C40 cycloalkyloxy, optionally substituted C5-C40 aryloxy, optionally substituted C2-C40 heteroaryloxy, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, and -L₃-R₃₆,
R₂₅ to R₃₃ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C40 cycloalkyloxy, optionally substituted C5-C40 aryloxy, optionally substituted C2-C40 heteroaryloxy, optionally substituted C5-C50 aryl, optionally substituted C2-C50 heteroaryl, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, and -L₃-R₃₆,
optionally, any two adjacent groups among R₂₁ to R₃₃ are bonded to each other to form an optionally substituted aliphatic ring, an optionally substituted aromatic ring, or an optionally substituted aliphatic-aromatic mixed ring,
A₃ and A₄ are each independently selected from hydrogen, halogen, optionally substituted C1-C10 alkyl, optionally substituted C3-C10 cycloalkyl, optionally substituted C1-C10 heterocycloalkyl, optionally substituted C1-C10 heteroalkyl, optionally substituted C2-C10 alkenyl, optionally substituted C2-C10 alkynyl, optionally substituted C1-C10 alkoxy, optionally substituted aryloxy, and cyano, provided that A₃ and A₄ are not simultaneously hydrogen,
X is O, S, CRₐR_{b}, NRₐ, or SiRₐR_{b},
Rₐ and R_{b} are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted C5-C50 aryl, and optionally substituted C2-C50 heteroaryl, or Rₐ and R_{b} are bonded to each other to form an optionally substituted aliphatic ring, an optionally substituted aromatic ring, or an optionally substituted aliphatic-aromatic mixed ring,
at least one of R₂₁ to R₃₃ is -L₃-R₃₆,
L₃ is a linker comprising a hydrocarbon having 1 to 40 carbon atoms,
R₃₆ is a nucleoside selected from Chemical Formulas 3 to 5: 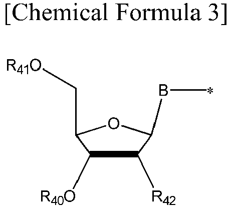 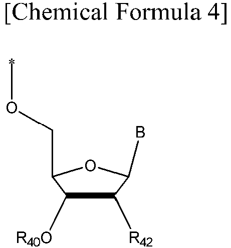 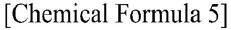 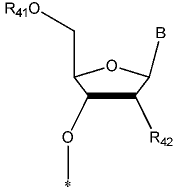 wherein
   * denotes a position at which the nucleoside is bonded to L₃,
   B is a nucleobase,
   R₄₀ is selected from hydrogen, -P(OR₄₃)(N(R₄₄R₄₅)), and -L₄-R₄₆,
   R₄₁ is an alcohol protecting group, hydrogen, or -P(OR₄₃)(N(R₄₄R₄₅)), or a nucleic acid or an optionally substituted support,
   R₄₃ to R₄₅ are each independently selected from hydrogen, optionally substituted C1-C10 alkyl, and optionally substituted C1-C10 heteroalkyl,
   L₄ is a single bond, or selected from an internucleotide phosphodiester bond, optionally substituted C1-C10 alkyl, and optionally substituted C1-C10 heteroalkyl,
   R₄₆ is hydroxy, -P(OR₄₃)(N(R₄₄R₄₅)), or a nucleic acid or an optionally substituted support,
   R₄₂ is selected from hydrogen, hydroxy, alkoxy, and -ORₚ,
   and Rₚ is a protecting group.

R₄₁ or R₄₆ may be an optionally substituted support or nucleic acid. The optionally substituted support or nucleic acid may be a support or nucleic acid substituted with at least one selected from coumarin, cyanine, BODIPY, fluorescein, rhodamine, pyrene, carbopyronin, oxazine, xanthene, thioxanthene, acridine, derivatives thereof, and Chemical Formula 1.

Specific examples of the compound defined herein are described below. However, the following exemplary compounds are provided to aid understanding of the compound defined herein and are not intended to limit the scope of the compound defined herein. It is expected that the exemplary compounds described below, as well as compounds considered equivalent according to the scope of the exemplary compounds and the compound defined herein, will exhibit the effects of the compound defined herein. Accordingly, the compound according to any embodiment of the present invention may be at least one structure or a derivative thereof selected from Compound 1 to Compound 47 described below.

In addition, the compound represented by Chemical Formula 1 or Chemical Formula 2 may be synthesized with reference to the synthesis examples of Compounds 1 to 42 disclosed in detail in the synthesis steps herein, or by referring to the disclosures herein and using known synthesis methods.

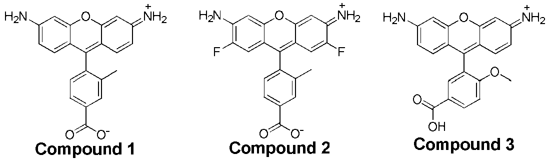

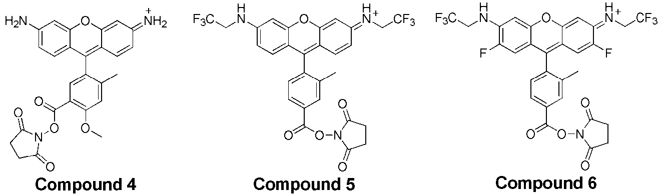

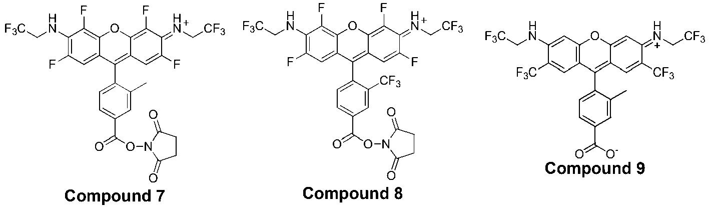

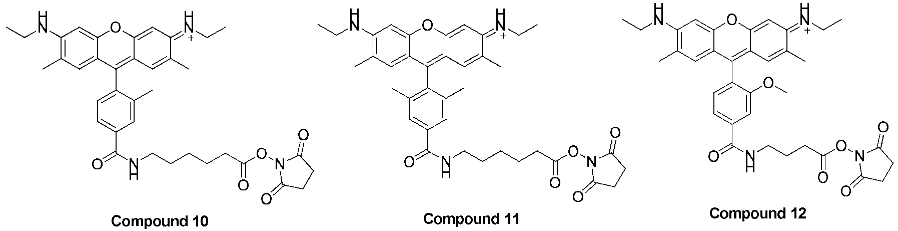

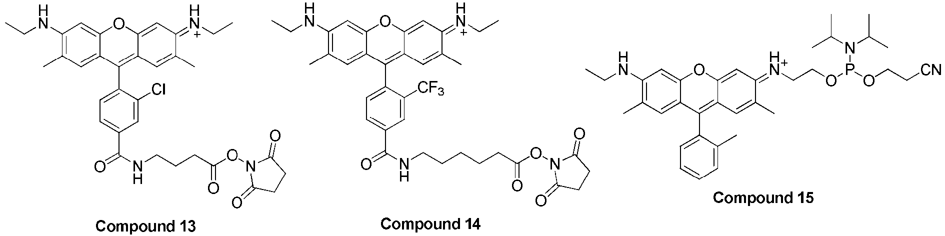

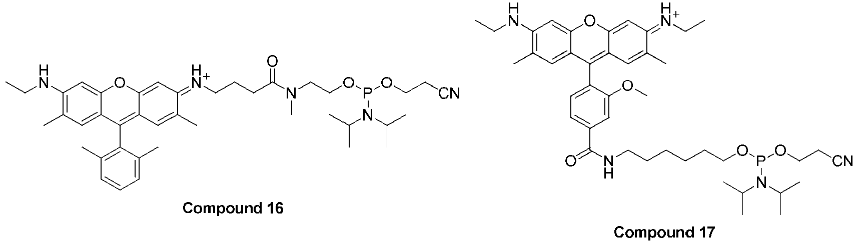

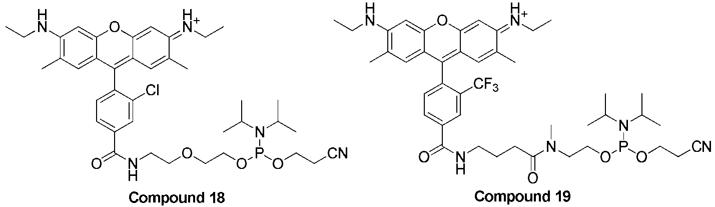

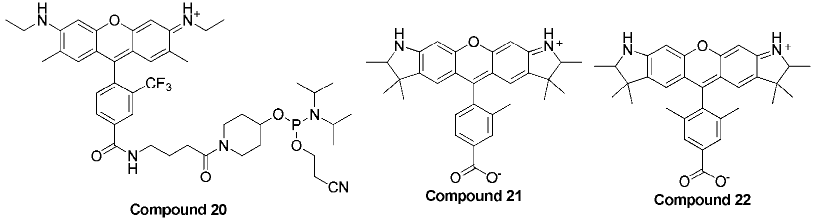

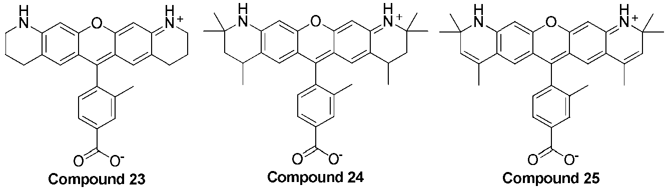

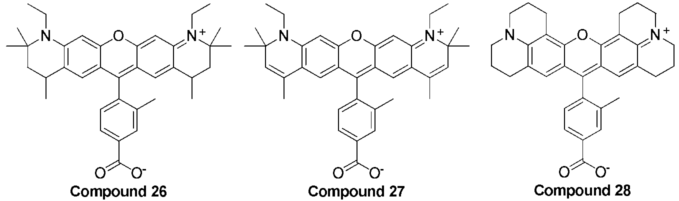

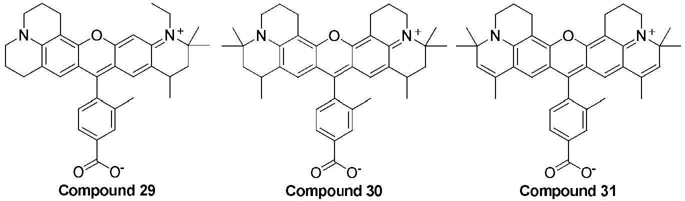

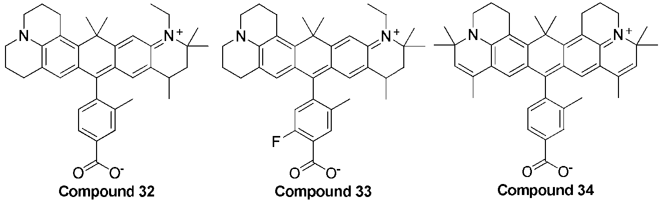

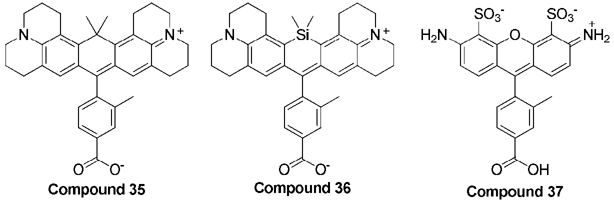

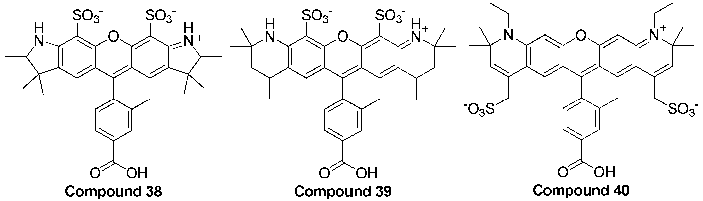

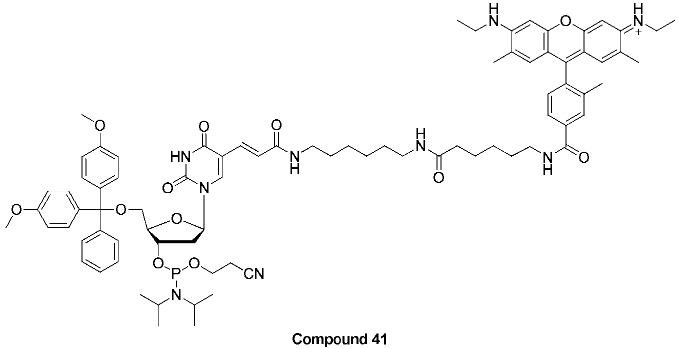

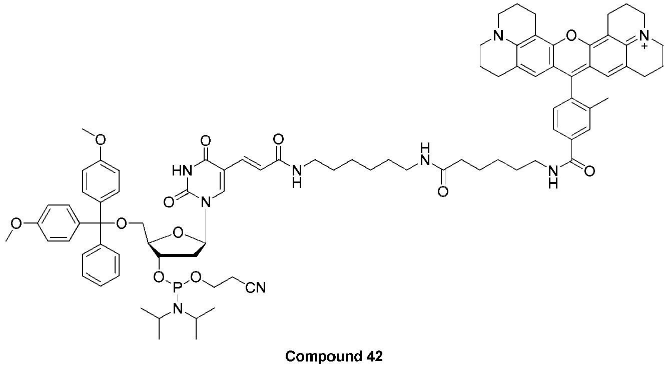

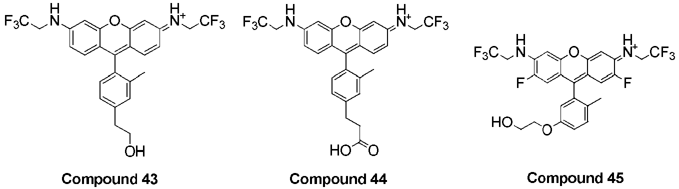

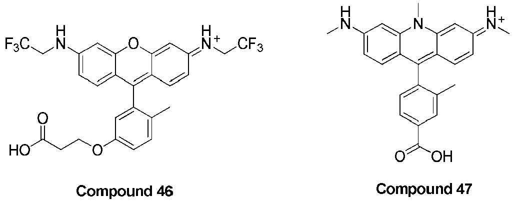

The compound defined herein can be used as a reporter for labeling or detecting biomolecules. The biomolecules include, but are not limited to, antibodies, lipids, proteins, peptides, carbohydrates, and/or nucleic acids (including DNA, RNA, or nucleotides).

Specific examples of lipids include fatty acids, phospholipids, and lipopolysaccharides, and specific examples of carbohydrates include monosaccharides, disaccharides, and polysaccharides (for example, dextran).

Furthermore, the compound defined herein can be used for labeling or detecting substances other than biomolecules, including drugs, hormones (including receptor ligands), receptors, enzymes or enzyme substrates, cells, cell membranes, toxins, microorganisms, or nano-biomaterials (such as polystyrene microspheres), which comprise at least one selected from amino, sulfhydryl, carbonyl, hydroxyl, carboxyl, phosphate, and thiophosphate groups.

### Conjugates, a Composition for Detecting Nucleic Acid, and a Support for Detecting Nucleic Acid Comprising the Novel Compound

According to another aspect of the present invention, there is provided a conjugate comprising the compound defined herein as a reporter for nucleic acid labeling. The conjugate may be a probe or primer, or a nucleotide conjugate.

When the conjugate is a probe, the probe is preferably, but not limited to, a probe capable of hybridizing complementarily to a target nucleic acid. As used herein, the probe may be selected from a nucleic acid, peptide, saccharide, oligonucleotide, protein, antibody, or a combination thereof, but is not limited thereto.

When the conjugate is a nucleotide conjugate, the conjugate may comprise an oligonucleotide.

The oligonucleotide refers to a polymer of 1 to several hundred nucleotides. In the oligonucleotide, the phosphodiester bond may be modified or replaced with methylphosphonate, phosphorothioate (PTO), boranophosphate, phosphoryl guanidine, guanidinopropyl phosphoramidate, thiazole, guanidinium, or the like. The oligonucleotide may also include one or more modified nucleotides. For example, the modified nucleotides may include PNA, BNA (e.g., LNA, ENA), HNA, ANA, CeNA, or GNA with sugar modification, or cases where any substituent (e.g., MOE, alkyl, halogen) is introduced into the sugar.

The modified nucleotides and nucleosides may include modified nucleobases derived from adenine (A), guanine (G), thymine (T), cytosine (C), and uracil (U). Examples include, but are not limited to, inosine, xanthine, hypoxanthine, neplanocin, isoguanosine, tubercidin, 2-aminoadenine, 2-haloadenine, 2-alkyladenine, 2-methylaminoadenine, 6-methyladenine, 8-haloadenine, 8-aminoadenine, 8-thioadenine, diazaadenine, 8-hydroxyadenine and other substituted adenines, 5-halouracil, 4-thiouracil, 5-trifluoromethyluracil, pseudouracil, 2-thiouracil, 5-halouracil and other substituted uracils, 5-halocytosine, 5-trifluorocytosine, 6-azacytosine and other substituted cytosines, 2-thiothymine, 6-azathymine and other substituted thymines, 8-halogenated guanine, 8-aminoguanine, 8-thioguanine, 8-thioalkylguanine, 8-hydroxyguanine, 8-azaguanine, 7-deazaguanine, 7-methylguanine and other substituted guanines, pyrazolo[3,4-d]pyrimidine, 2,6-diaminopurine, phenoxazine, 2-aminopurine, 3-nitropyrrole, 5-hydroxybutynyluridine, Super A^{®}, Super G^{®}, Super T^{®}, Super D^{™}, and the like.

Examples of modified nucleobases can also be found in the following references: U.S. Pat. No. 11,155,713; Current Topics in Medicinal Chemistry, Volume 7, Number 7, Wojciechowski, Filip, and Hudson, Robert H., 2007, pp. 667-679. As used herein, the term "nucleobase" includes modified nucleobases.

The conjugate may include a minor groove binder (MGB) for selective binding to the minor groove of a target nucleic acid. The minor groove binder is a crescent-shaped molecule capable of selectively forming non-covalent bonds with the minor groove (e.g., shallow furrow within the DNA helix) present in nucleic acids such as DNA.

The conjugate may also include at least one quencher. In this case, the quencher may be located at the 5' end, the 3' end, or at an internal position of the conjugate.

For example, the compound (reporter) represented by Chemical Formula 1 or Chemical Formula 2 may be labeled at the 5' end of the conjugate, and a quencher may be labeled at the 3' end. Between the 5' end and the 3' end, a probe or a minor groove binder capable of hybridizing complementarily with a target nucleic acid may be positioned.

The absorbance range of the quencher usable herein may be from 400 nm to 800 nm. In addition, the absorbance range of the quencher may be appropriately selected in consideration of the fluorescence characteristics of the reporter defined herein.

It is important that the probe is designed such that the reporter can be sufficiently quenched by the quencher while minimizing signal interference. Accordingly, when designing the probe, it is necessary to ensure compatibility between the reporter labeled at the 5' end and the quencher labeled at the 3' end of the probe, depending on the type of target biomolecule (for example, nucleic acid).

As the quencher, various known or commercially available quenchers, such as azo, coumarin, cyanine, BODIPY, fluorescein, rhodamine, pyrene, carbopyronin, oxazine, xanthene, thioxanthene, acridine, and derivatives thereof, may be used (for example, BHQ0, BHQ1, BHQ2, BHQ3, BBQ650, DABCYL, TAMRA, MGBEclipse, Atto540Q, Atto575Q, Atto612Q, QSY7, QSY21, etc.). In addition, the quencher disclosed in Korean Patent Publication No. 10-2020-0067733 and 10-2019-0062162 may also be used.

The conjugate defined herein can be variously utilized in chemical and biological fields. In particular, it can be advantageously used in real-time polymerase chain reaction (PCR) or microarray analysis, but is not limited thereto.

According to another aspect of the present invention, there is provided a composition for detecting nucleic acid comprising the conjugate. The composition for detecting nucleic acid may further comprise an enzyme for reaction with a target biomolecule, a solvent (such as a buffer), and other reagents, together with the conjugate containing the compound defined herein as a reporter for nucleic acid labeling and a quencher.

The solvent may include, for example, a buffer selected from the group consisting of phosphate buffer, carbonate buffer, and Tris buffer; an organic solvent selected from dimethyl sulfoxide, dimethylformamide, dichloromethane, methanol, ethanol, and acetonitrile; or water. Depending on the type of solvent, it is possible to control the solubility by introducing various functional groups into the reporter.

According to yet another aspect of the present invention, there is provided a support for detecting nucleic acid comprising: the compound (reporter) defined herein; a support; and a linker that connects the probe and the support. Accordingly, a biomolecule in a sample can be immobilized on the support through interaction with the reporter immobilized thereon.

The support may be made of at least one material selected from glass (for example, controlled pore glass (CPG)), cellulose, nylon, acrylamide gel, dextran, polystyrene, resin, alginate, collagen, peptide, fibrin, hyaluronic acid, agarose, polyhydroxyethyl methacrylate, polyvinyl alcohol, polyethylene glycol, polyethylene oxide, polyethylene glycol diacrylate, gelatin, Matrigel^{®}, polylactic acid, carboxymethyl cellulose, dextran, chitosan, latex, or Sepharose^{®}, but is not limited thereto. The support may be in the form of beads or a membrane.

The linker is a portion connecting the reporter and the support, and any substance capable of linking the reporter and the support can be used as the linker as intended herein.

For example, the linker may be selected from the group consisting of optionally substituted C1-C30 alkyl, optionally substituted C3-C30 cycloalkyl, optionally substituted C2-C30 heteroalkyl, optionally substituted C2-C30 heterocycloalkyl, optionally substituted C2-C30 alkenyl, optionally substituted C5-C50 aryl, optionally substituted C2-C50 heteroaryl, amide (-CONH-), ester (-COO-), ketone (-CO-), nucleoside, and any combination thereof.

Such a linker serves only to connect the reporter and the support, and does not affect other reactions of the reporter or fluorescent dye, nor does it interfere with fluorescence or quenching actions.

### Method for Detecting Nucleic Acid

According to another aspect of the present invention, there is provided a method for detecting nucleic acid, the method comprising: (a) preparing a reaction mixture comprising a target nucleic acid, reagents required for amplifying the target nucleic acid, and a nucleotide conjugate defined herein; (b) amplifying the target nucleic acid in the reaction mixture; and (c) measuring the fluorescence intensity of the reaction mixture.

Step (b) may comprise: (b-1) extending the nucleotide conjugate hybridized to the target nucleic acid by a polymerase; (b-2) separating the reporter and quencher of the nucleotide conjugate from the target nucleic acid by exonuclease activity of the polymerase; and (b-3) allowing the reporter separated from the quencher to emit fluorescence.

Step (b) may be performed by a method selected from the group consisting of strand displacement amplification (SDA), polymerase chain reaction (PCR), reverse transcription polymerase chain reaction (RT-PCR), real-time polymerase chain reaction (Real-time PCR), allele-specific polymerase chain reaction, ligase chain reaction (LCR), rolling circle amplification (RCA), isothermal multiple displacement amplification (IMDA), recombinase polymerase amplification (RPA), self-sustained sequence replication (3SR), single primer isothermal amplification (SPIA), multiple displacement amplification (MDA), whole genome amplification (WGA), cross-priming amplification (CPA), signal mediated amplification of RNA technology (SMART), transcription mediated amplification (TMA), nucleic acid sequence based amplification (NASBA), loop-mediated isothermal amplification (LAMP), and helicase dependent amplification (HDA).

The method may further comprise step (d) of determining the amount of amplification of the target nucleic acid based on the fluorescence intensity measured in step (c).

### DNA Microarray Method

In a DNA microarray method, a target nucleic acid to be labeled is reacted with a dye for labeling, and a single-stranded probe nucleic acid having a base sequence complementary to the target nucleic acid is prepared. The single-stranded target nucleic acid and the probe nucleic acid are hybridized on a substrate, and the fluorescence of the target nucleic acid is measured.

In this labeling method, as the probe nucleic acid immobilized on the substrate, when investigating gene expression, a cDNA library such as cDNA, a genomic library, or a product prepared by amplification using the entire genome as a template by PCR may be used.
In addition, when examining mutations in genes, various conjugates corresponding to mutations may be synthesized based on a known reference sequence and used.
The probe nucleic acid may be immobilized on the substrate by an appropriate method depending on the type of nucleic acid or substrate. For example, a method may be employed in which DNA is electrostatically bound to a substrate surface-treated with a cation such as polylysine by utilizing the charge of the DNA.

The single-stranded target nucleic acid is immobilized on the substrate and hybridized with the nucleotide conjugate. In this case, the compound defined herein is labeled at the 5' end of the nucleotide conjugate, and a quencher is labeled at the 3' end. A probe that can complementarily bind to the target nucleic acid may be positioned between the 5' and 3' ends.

Hybridization is preferably carried out at a temperature ranging from room temperature to 70°C for 2 to 48 hours. Through hybridization, the target nucleic acid having a base sequence complementary to the probe nucleic acid selectively binds to the probe nucleic acid. Then, the substrate is washed and dried at room temperature.
At this time, although the nucleotide conjugate is hybridized to the target nucleic acid via the probe, the compound at the 5' end remains quenched by the quencher at the 3' end.

Subsequently, the nucleotide conjugate hybridized to the target nucleic acid is extended by a polymerase. The nucleotide conjugate is separated and degraded from the target nucleic acid by the exonuclease activity of the polymerase, and the compound at the 5' end and the quencher at the 3' end of the nucleotide conjugate are separated from each other, thereby enabling the fluorescent dye to emit fluorescence.

At this time, by measuring the fluorescence intensity generated, the amplification amount of the target nucleic acid can be determined.

### PCR Method

In a PCR method, a probe complementary to the base sequence of a target nucleic acid to be labeled is labeled with a reporter, and the probe is reacted with the target nucleic acid either before or after amplification of the target nucleic acid, and the fluorescence of the target nucleic acid is measured.

Specifically, the extension reaction of the target nucleic acid is carried out by an enzyme (DNA polymerase or RNA polymerase). In this reaction, the enzyme recognizes a double-stranded nucleic acid sequence formed by the target nucleic acid and an oligonucleotide primer, and extension occurs from the recognized position, thereby amplifying only the desired gene region.

During synthesis by the enzyme, nucleotides (dNTPs or NTPs) are used as substrates for the synthesis reaction. By mixing nucleotides bearing a reporter with ordinary nucleotides (dNTPs or NTPs) at an arbitrary ratio, nucleic acids incorporating dyes at that ratio can be synthesized.

Alternatively, after incorporating nucleotides having an amino group into the nucleic acid by PCR, the reporter may be conjugated to synthesize a nucleic acid labeled with the reporter.

In addition, when nucleotides in which the hydroxyl group at the 3' position is replaced with hydrogen are used as substrates for synthesis by the enzyme, the extension of the nucleic acid ceases, and the reaction terminates at that point. Such a nucleotide, a dideoxynucleotide triphosphate (ddNTP), is referred to as a terminator.

When terminators are mixed with ordinary nucleotides to synthesize a nucleic acid, terminators are incorporated with a certain probability, causing termination of the reaction, thereby synthesizing nucleic acids of various lengths. By separating these products by gel electrophoresis, DNA molecules are arranged in order of length.

When each type of terminator is labeled with a reporter corresponding to its base, base-specific patterns can be observed at the 3' ends of the synthesized nucleic acids. By reading fluorescence information based on the reporters attached to the terminators, base sequence information of the target nucleic acid can be obtained.

Alternatively, instead of using terminators, a primer pre-labeled with a reporter may be hybridized with the target nucleic acid.

As a probe, a peptide nucleic acid (PNA) may also be used. PNA is a molecule in which the sugar-phosphate backbone of a nucleic acid is replaced with a polyamide backbone composed of glycine units, and it has a three-dimensional structure closely resembling that of nucleic acids. It binds very specifically and strongly to nucleic acids with complementary base sequences. This allows the probe to be applied not only to conventional DNA analysis methods such as in situ hybridization (ISH), but also as a reagent for telomere research through use of telomere-specific PNA probes.

For example, in labeling, a double-stranded DNA may be hybridized with a PNA labeled with a reporter and having a base sequence complementary to all or part of the DNA sequence. The mixture is heated to generate single-stranded DNA, slowly cooled to room temperature to form a PNA-DNA complex, and fluorescence is measured.

In the above example, a method was described in which a target nucleic acid is amplified by PCR and the fluorescence of the resulting product is measured. In this method, it is necessary to check the size of the amplified product by electrophoresis and then measure fluorescence intensity to determine the quantity of the amplified product.

To address this, probes designed to emit fluorescence upon hybridization with PCR products, by utilizing energy transfer of fluorescent dyes, can be used to measure the quantity of products in real time.

For example, DNA labeled with a donor and an acceptor can be used. Specific labeling methods include molecular beacon assays, TaqMan-PCR methods, and cycling probe methods, which can confirm the presence of a nucleic acid with a specific sequence.

### Other Labeling Methods

The reporter of the present invention can also be used in methods for labeling a target using specific binding.

Specifically, in labeling a sample containing a target or a sample modified with a modifying substance, one of a binding substance that specifically binds to the sample or a binding substance that specifically binds to the modifying substance is labeled with the reporter, and fluorescence from the labeled binding substance is measured.

Examples of combinations of the sample or modifying substance and the binding substance include antigen-antibody, hapten-anti-hapten antibody, biotin-avidin, tag antigen-tag antibody, lectin-glycoprotein, or hormone-receptor combinations.

Specifically, a binding substance such as an antibody labeled with the reporter is reacted with an antigen present on a substrate, in a solution, on beads, or in an antibody, to label a specific antigen through antigen-specific interaction of the antibody. Antigens may include proteins, polysaccharides, nucleic acids, and peptides, and haptens such as low-molecular-weight molecules like FITC or dinitrophenyl groups may also be used. Combinations of antigens (or haptens) and antibodies include GFP and anti-GFP antibody, and FITC and anti-FITC antibody.

The labeled antigens can be used in various detection methods such as immunostaining, ELISA, Western blotting, or flow cytometry.

Furthermore, the reporter of the present invention can be used to observe intracellular signaling phenomena. Various enzymes are involved in intracellular signaling or cellular responses thereto. In representative signaling pathways, specific protein kinases are activated, which induces phosphorylation of proteins and initiates signal transduction.

Binding and hydrolysis of nucleotides (e.g., ATP or ADP) play a critical role in such activation, and introduction of the reporter into nucleotide derivatives enables sensitive observation of intracellular signaling phenomena.

The reporter of the present invention can also be used to observe gene expression phenomena utilizing RNA interference (RNAi).

RNAi is a phenomenon in which the expression of a target gene is suppressed by introducing double-stranded RNA (dsRNA) into cells to degrade the mRNA of the target gene. By labeling the designed dsRNA with the reporter, RNAi phenomena can be observed.

Additionally, the reporter of the present invention may be used as a dye for confirming transcription levels of a target nucleic acid or expression levels of a target protein by including a reactive group capable of labeling the target nucleic acid or target protein in tissues or cells.

Hereinafter, specific embodiments of the present invention are provided. However, the embodiments described below are merely illustrative or explanatory of the present invention, and the present invention is not limited thereto. Furthermore, reporters defined in the claims and the detailed description of the specification, which are not synthesized in the following preparation examples, may be synthesized by reference to the preparation examples provided herein.

### Synthesis Example 1. Synthesis of Compound 1

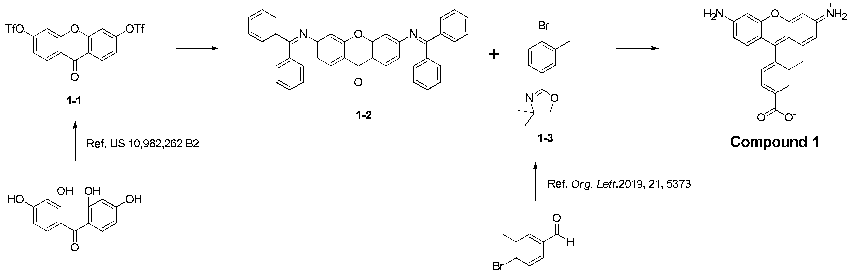

### Synthesis of Intermediate 1-1

Intermediate 1-1 was synthesized with reference to US 10,982,262 B2.

### Synthesis of Intermediate 1-2

Intermediate 1-1 (10 g, 20.3 mmol), benzophenone imine (9.2 g, 50.7 mmol), cesium carbonate (19.8 g, 60.9 mmol), Pd₂(dba)₃ (0.37 g, 0.4 mmol), Xantphos (0.7 g, 1.2 mmol), and 1,4-dioxane (100 mL) were added to a 500 mL four-neck reaction flask, and the mixture was stirred at 100°C for 24 hours. After cooling, the reaction mixture was filtered through a Celite pad. The filtrate was concentrated and purified by column chromatography to obtain Intermediate 1-2.

### Synthesis of Intermediate 1-3

Intermediate 1-3 was synthesized with reference to Org. Lett. 2019, 21, 5373.

### Synthesis of Compound 1

Intermediate 1-3 (2.18 g, 8.11 mmol) and tetrahydrofuran (20 mL) were added to a 100 mL three-neck reaction flask, and the mixture was stirred under a nitrogen atmosphere at -78°C for 5 minutes. To the reaction flask, 1.6 M n-butyllithium (5 mL, 8.11 mmol) was slowly added dropwise and stirred at -78°C for 1 hour. Intermediate 1-2 (1.5 g, 2.7 mmol) dissolved in tetrahydrofuran (15 mL) was added dropwise to the reaction flask, followed by stirring at room temperature for 1 hour. Hydrochloric acid (6 M, 15 mL) was added, and the reaction was stirred at 80°C for 16 hours. After concentration, the crude product was purified by column chromatography to obtain Compound 1 (0.3 g).

The ¹H-NMR data of the obtained Compound 1 is as follows:
¹H-NMR (400 MHz, MeOD) δ 8.11 (s, 1H), 8.11-8.09 (d, 1H), 7.41-7.39 (d, 1H), 7.10-7.06 (m, 2H), 6.89-6.86 (m, 4H), 2.14 (s, 3H).

### Synthesis Example 2. Synthesis of Compound 2

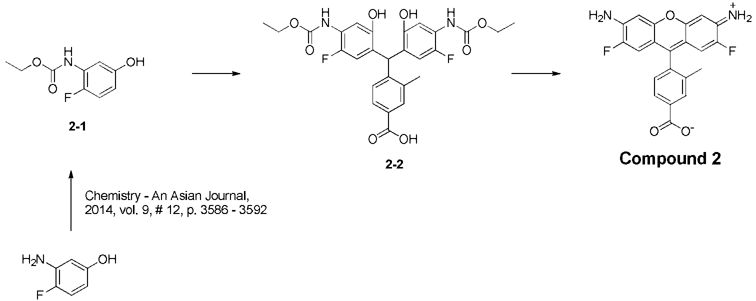

### Synthesis of Intermediate 2-1

Intermediate 2-1 was synthesized with reference to Chemistry - An Asian Journal, 2014, vol. 9, no. 12, pp. 3586-3592.

### Synthesis of Intermediate 2-2

A 100 mL single-neck round-bottom flask was charged with Intermediate 2-1 (2.67 g, 13.4 mmol), 4-formyl-3-methylbenzoic acid (1 g, 6.09 mmol), p-toluenesulfonic acid (0.21 g, 1.22 mmol), and toluene (30 mL), and the mixture was stirred at 80 °C for 16 hours. After cooling, the reaction mixture was concentrated and purified by column chromatography to obtain Intermediate 2-2.

### Synthesis of Compound 2

A 50 mL single-neck round-bottom flask was charged with Intermediate 2-2 (1 g, 1.84 mmol) and concentrated hydrobromic acid (10 mL), and the mixture was stirred under reflux for 16 hours. After cooling, the reaction mixture was concentrated, followed by the addition of chloranil (0.64 g, 2.62 mmol) and 1,4-dioxane (20 mL), and stirred at 60 °C for 4 hours. After cooling, the mixture was filtered through Celite, and the filtrate was concentrated and purified by column chromatography to obtain Compound 2 (0.3 g).

The ¹H-NMR data of the obtained Compound 2 is as follows:
¹H-NMR (400 MHz, MeOD) δ 7.95 (s, 1H), 7.90-7.88 (d, 1H), 7.16-7.14 (d, 1H), 6.98-6.96 (d, 2H), 6.86-6.83 (m, 2H), 2.05 (s, 3H).

### Synthesis Example 3. Synthesis of Compound 3

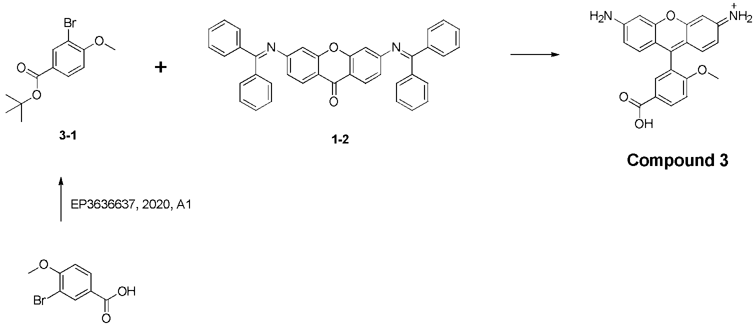

### Synthesis of Intermediate 3-1

Intermediate 3-1 was synthesized with reference to EP3636637, 2020, A1.

### Synthesis of Compound 3

A 50 mL three-neck round-bottom flask was charged with Intermediate 3-1 (0.78 g, 2.7 mmol) and tetrahydrofuran (10 mL), and the mixture was stirred under a nitrogen atmosphere at -78 °C for 5 minutes. To the reaction flask, 1.6 M n-butyllithium (1.6 mL, 2.7 mmol) was slowly added dropwise and stirred at -78 °C for 1 hour. Intermediate 1-2 (0.5 g, 0.5 mmol) dissolved in tetrahydrofuran (5 mL) was added dropwise to the reaction flask, and the reaction mixture was stirred at room temperature for 1 hour. After concentration, trifluoroacetic acid (1.2 mL) and dichloromethane (10 mL) were added to the reaction flask, and the mixture was stirred at room temperature for 16 hours. The reaction mixture was concentrated and purified by column chromatography to obtain Compound 3 (0.05 g).

The ¹H-NMR data of the obtained Compound 3 is as follows:
¹H-NMR (400 MHz, MeOD) δ 7.78 (s, 4H, NH), 7.77-7.75 (m, 2H), 7.44-7.40 (m, 1H), 7.15-7.12 (m, 2H), 6.88-6.79 (m, 2H), 6.78 (s, 2H), 3.74 (s, 3H).

### Synthesis Example 4. Synthesis of Compound 4

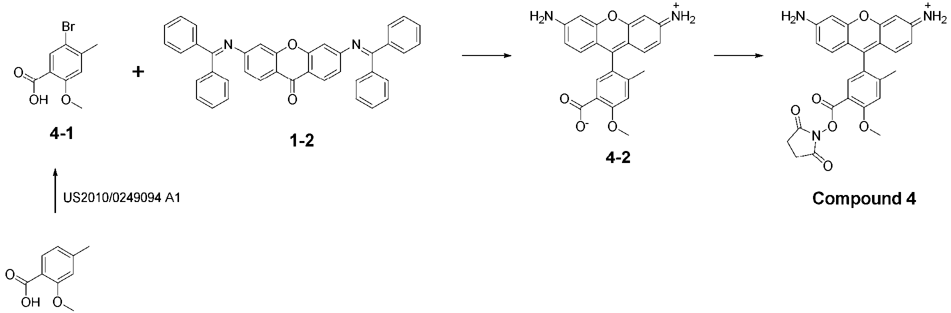

### Synthesis of Intermediate 4-1

Intermediate 4-1 was synthesized with reference to US2010/0249094 A1.

### Synthesis of Intermediate 4-2

A 100 mL three-neck round-bottom flask was charged with Intermediate 4-1 (1.24 g, 5.05 mmol) and tetrahydrofuran (15 mL), and the mixture was stirred under a nitrogen atmosphere at -78 °C for 5 minutes. To the reaction flask, 2.5 M n-butyllithium (4 mL, 10.1 mmol) was slowly added dropwise and stirred at -78 °C for 15 minutes. Intermediate 1-2 (2.0 g, 3.61 mmol) dissolved in tetrahydrofuran (20 mL) was added dropwise to the reaction flask, and the reaction mixture was stirred at room temperature for 2 hours. After concentration, trifluoroacetic acid (1.27 mL) and dichloromethane (8 mL) were added to the reaction flask, and the mixture was stirred at room temperature for 16 hours. The reaction mixture was concentrated and purified by column chromatography to obtain Intermediate 4-2.

### Synthesis of Compound 4

A 50 mL single-neck round-bottom flask was charged with Intermediate 4-2 (0.18 g, 0.48 mmol), TSTU (N,N,N',N'-Tetramethyl-O-(N-succinimidyl)uronium tetrafluoroborate) (0.22 g, 0.72 mmol), triethylamine (0.26 mL, 1.92 mmol), and dimethylformamide (3 mL), and the mixture was stirred at room temperature for 30 minutes. The reaction mixture was concentrated and purified by column chromatography to obtain Compound 4 (0.1 g).

The ¹H-NMR data of the obtained Compound 4 is as follows:
¹H-NMR (400 MHz, DMSO-d6) δ 7.75 (s, 1H), 7.52 (s, 1H), 7.35-7.32 (d, 2H), 7.14-7.12 (m, 2H), 7.04-7.02 (d, 2H), 4.12 (s, 3H), 2.90 (s, 4H), 2.14 (s, 3H).

### Synthesis Example 5. Synthesis of Compound 5

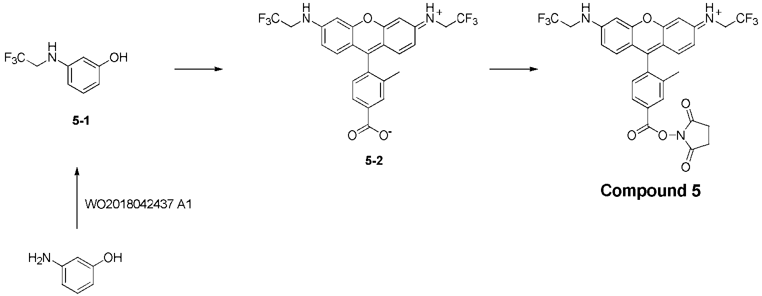

### Synthesis of Intermediate 5-1

Intermediate 5-1 was synthesized with reference to WO2018/042437 A1.

### Synthesis of Intermediate 5-2

A 100 mL single-neck round-bottom flask was charged with Intermediate 5-1 (1.16 g, 6.09 mmol), 4-formyl-3-methylbenzoic acid (0.5 g, 3.05 mmol), and 60% aqueous sulfuric acid (10 mL), and the mixture was stirred at 130 °C for 3 hours. After cooling, the reaction mixture was concentrated and purified by column chromatography. The reaction mixture was then poured into cold water, and the resulting solid was filtered to obtain Intermediate 5-2.

### Synthesis of Compound 5

A 100 mL single-neck round-bottom flask was charged with Intermediate 5-2 (1.36 g, 2.67 mmol), HSTU (N,N,N',N'-Tetramethyl-O-(N-succinimidyl)uronium hexafluorophosphate) (1.44 g, 4.01 mmol), triethylamine (1.1 mL, 8.02 mmol), and dimethylformamide (15 mL), and the mixture was stirred at room temperature for 30 minutes. The reaction mixture was concentrated and purified by column chromatography to obtain Compound 5 (0.4 g).

The ¹H-NMR data of the obtained Compound 5 is as follows:
¹H-NMR (400 MHz, DMSO-d6) δ 8.26 (s, 1H), 8.19-8.17 (d, 1H), 7.61-7.59 (d, 1H), 7.34-7.13 (m, 6H), 4.55-4.45 (m, 4H), 2.94 (s, 4H), 2.13 (s, 3H).

### Synthesis Example 6. Synthesis of Compound 6

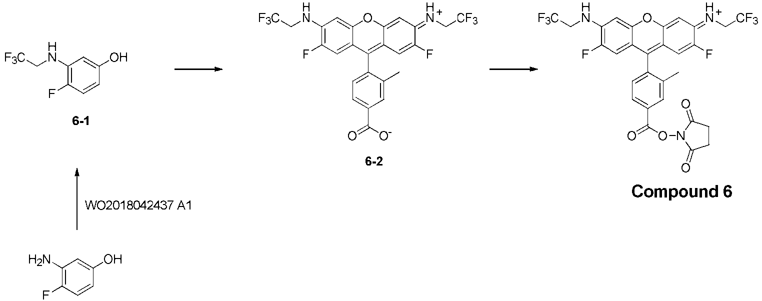

### Synthesis of Intermediate 6-1

Intermediate 6-1 was synthesized with reference to WO2018/042437 A1.

### Synthesis of Intermediate 6-2

A 100 mL single-neck round-bottom flask was charged with Intermediate 6-1 (1.27 g, 6.07 mmol), 4-formyl-3-methylbenzoic acid (0.5 g, 3.05 mmol), and 60% aqueous sulfuric acid (10 mL), and the mixture was stirred at 130 °C for 3 hours. After cooling, the reaction mixture was concentrated and purified by column chromatography. The reaction mixture was then poured into cold water, and the resulting solid was filtered to obtain Intermediate 6-2.

### Synthesis of Compound 6

A 100 mL single-neck round-bottom flask was charged with Intermediate 6-2 (0.15 g, 0.28 mmol), HSTU (N,N,N',N'-Tetramethyl-O-(N-succinimidyl)uronium hexafluorophosphate) (0.3 g, 0.84 mmol), triethylamine (0.12 mL, 0.84 mmol), and dimethylformamide (6 mL), and the mixture was stirred at room temperature for 30 minutes. The reaction mixture was concentrated and purified by column chromatography to obtain Compound 6 (0.07 g).

The ¹H-NMR data of the obtained Compound 6 is as follows:
¹H-NMR (400 MHz, DMSO-d6) δ 8.24 (s, 1H), 8.19-8.17 (d, 1H), 7.64-7.63 (d, 2H), 7.56-7.54 (d, 1H), 7.21-7.19 (d, 2H), 4.53-4.50 (m, 4H), 2.95 (s, 4H), 2.15 (s, 3H).

### Synthesis Example 7. Synthesis of Compound 7

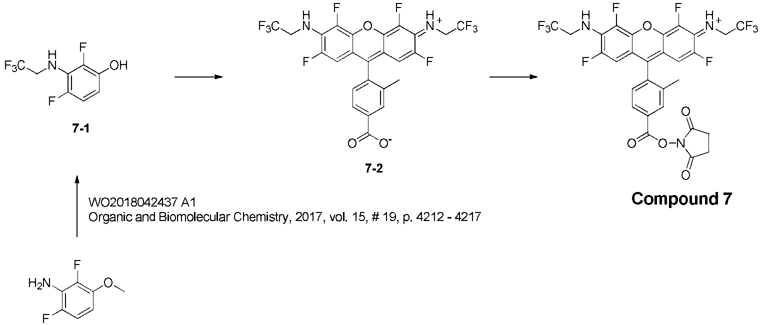

### Synthesis of Intermediate 7-1

Intermediate 7-1 was synthesized with reference to Organic and Biomolecular Chemistry, 2017, vol. 15, #19, pp. 4212-4217 and WO2018/042437 A1.

### Synthesis of Intermediate 7-2

A 100 mL single-neck round-bottom flask was charged with Intermediate 7-1 (1.28 g, 6.07 mmol), 4-formyl-3-methylbenzoic acid (0.5 g, 3.05 mmol), and 60% aqueous sulfuric acid (10 mL), and the mixture was stirred at 130 °C for 3 hours. After cooling, the reaction mixture was concentrated and purified by column chromatography. The reaction mixture was then poured into cold water, and the resulting solid was filtered to obtain Intermediate 7-2.

### Synthesis of Compound 7

A 100 mL single-neck round-bottom flask was charged with Intermediate 7-2 (0.5 g, 0.86 mmol), HSTU (N,N,N',N'-Tetramethyl-O-(N-succinimidyl)uronium hexafluorophosphate) (0.62 g, 1.74 mmol), triethylamine (0.5 mL, 3.45 mmol), and dimethylformamide (8 mL), and the mixture was stirred at room temperature for 30 minutes. The reaction mixture was concentrated and purified by column chromatography to obtain Compound 7 (0.07 g).

The ¹H-NMR data of the obtained Compound 7 is as follows:
¹H-NMR (400 MHz, DMSO-d6) δ 8.17 (s, 1H), 8.11-8.09 (d, 1H), 7.51-7.49 (d, 1H), 6.39-6.36 (d, 2H), 4.21-4.13 (m, 4H), 2.93 (s, 4H), 2.16 (s, 3H).

### Synthesis Example 8. Synthesis of Compound 8

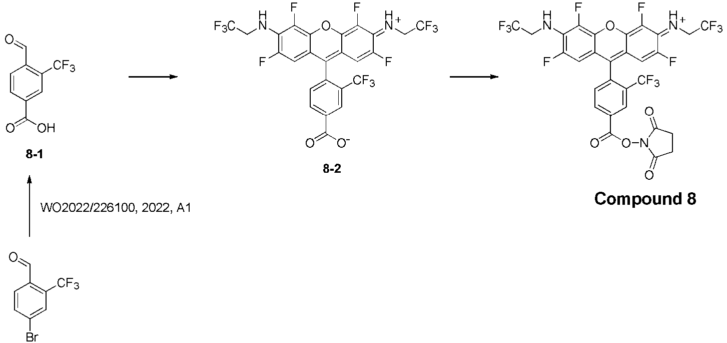

### Synthesis of Intermediate 8-1

Intermediate 8-1 was synthesized with reference to WO2022/226100, 2022, A1.

### Synthesis of Intermediate 8-2

A 100 mL single-neck round-bottom flask was charged with Intermediate 6-1 (0.96 g, 4.58 mmol), Intermediate 8-1 (0.5 g, 2.29 mmol), and 60% aqueous sulfuric acid (10 mL), and the mixture was stirred at 130 °C for 3 hours. After cooling, the reaction mixture was concentrated and purified by column chromatography. The reaction mixture was then poured into cold water, and the resulting solid was filtered to obtain Intermediate 8-2.

### Synthesis of Compound 8

A 100 mL single-neck round-bottom flask was charged with Intermediate 8-2 (0.6 g, 1.0 mmol), HSTU (N,N,N',N'-Tetramethyl-O-(N-succinimidyl)uronium hexafluorophosphate) (0.9 g, 2.51 mmol), triethylamine (0.5 mL, 4.0 mmol), and dimethylformamide (10 mL), and the mixture was stirred at room temperature for 30 minutes. The reaction mixture was concentrated and purified by column chromatography to obtain Compound 8 (0.14 g).

The ¹H-NMR data of the obtained Compound 8 is as follows:
¹H-NMR (400 MHz, DMSO-d6) δ 8.51 (s, 1H), 8.42-8.40 (d, 1H), 7.59-7.57 (d, 1H), 6.89-6.87 (d, 2H), 6.68-6.65 (d, 2H), 4.15-4.05 (m, 4H), 2.94 (s, 4H).

### Synthesis Example 9. synthesis of Compound 9

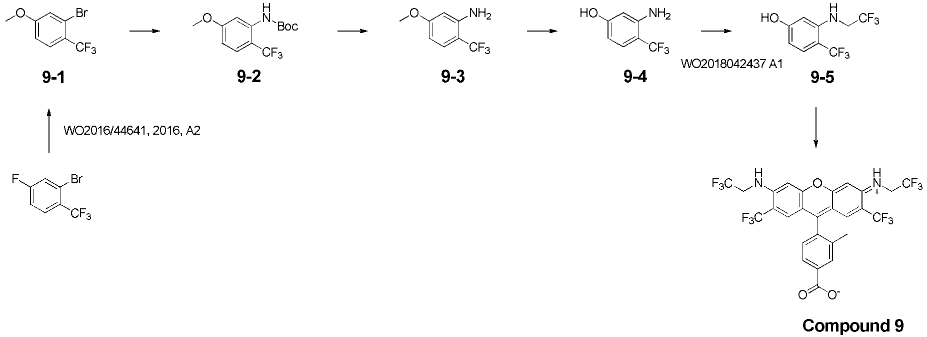

### synthesis of Intermediate 9-1

Intermediate 9-1 was synthesized with reference to WO2016/44641, 2016, A2.

### synthesis of Intermediate 9-2

A 1 L four-neck round-bottom flask was charged with Intermediate 9-1 (42 g, 165 mmol), tert-butyl carbamate (21.3 g, 182 mmol), cesium carbonate (108 g, 331 mmol), Pd(OAc)2 (3.7 g, 16.5 mmol), Xantphos (9.6 g, 16.5 mmol), and 1,4-dioxane (420 mL), and the mixture was stirred at 100°C for 24 hours. After cooling, the mixture was filtered through celite. The filtrate was concentrated and purified by column chromatography to obtain Intermediate 9-2.

### synthesis of Intermediate 9-3

A 250 mL single-neck round-bottom flask was charged with Intermediate 9-2 (7 g, 28 mmol), trifluoroacetic acid (14 mL), and dichloromethane (56 mL), and the mixture was stirred at room temperature for 2 hours. The reaction mixture was concentrated, followed by the addition of dichloromethane (100 mL) and 5% aqueous sodium bicarbonate solution (100 mL), and vigorously stirred. The organic layer was separated, dried over anhydrous sodium sulfate for 5 minutes, and filtered. The filtrate was concentrated to obtain Intermediate 9-3.

### synthesis of Intermediate 9-4

A 500 mL single-neck round-bottom flask was charged with Intermediate 9-3 (6.6 g, 23 mmol), boron tribromide (2.60 mL, 0.028 mmol), and dichloromethane (66 mL), and the mixture was stirred at room temperature for 6 hours. After adding 5% aqueous sodium bicarbonate solution (200 mL), the mixture was vigorously stirred. The organic layer was separated, dried over anhydrous sodium sulfate for 5 minutes, and filtered. The filtrate was concentrated and purified by column chromatography to obtain Intermediate 9-4.

### synthesis of Intermediate 9-5

Intermediate 9-5 was synthesized with reference to WO2018/042437 A1.

### synthesis of Compound 9

A 100 mL single-neck round-bottom flask was charged with Intermediate 9-5 (2.1 g, 8 mmol), 4-formyl-3-methylbenzoic acid (0.66 g, 4 mmol), and 60% aqueous sulfuric acid (5 mL), and the mixture was stirred at 130 °C for 3 hours. The reaction mixture was cooled, poured into cold water, and neutralized. The resulting solid was filtered, vacuum dried at 60 °C, and Compound 9 was obtained (1.5 g).

The ¹H-NMR data of the obtained Compound 9 is as follows:
¹H-NMR (400 MHz, DMSO-d6) δ 8.22 (s, 1H), 8.16-8.11 (d, 1H), 7.51-7.47 (d, 1H), 7.44-7.23 (m, 4H), 4.55-4.45 (m, 4H), 2.13 (s, 3H).

### Synthesis Example 10. synthesis of Compound 10

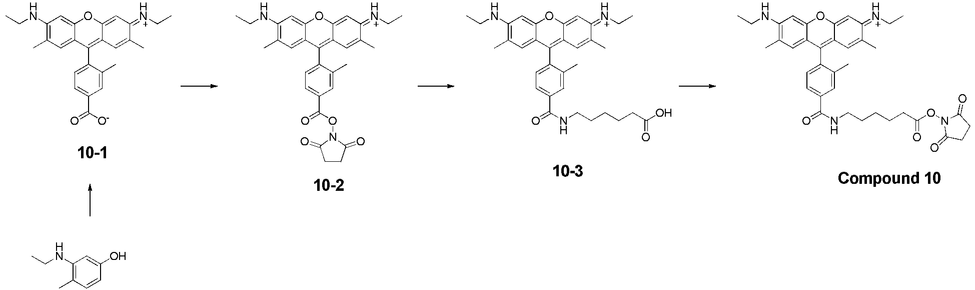

### synthesis of Intermediate 10-1

A 250 mL single-neck round-bottom flask was charged with 3-ethylamino-p-cresol (27.0 g, 0.178 mol), 4-formyl-3-methylbenzoic acid (14.7 g, 0.089 mol), and 70% sulfuric acid (110 mL), and the mixture was stirred at 130 °C for 3 hours. After cooling, the reaction mixture was poured into cold water and neutralized. The resulting solid was filtered and dried. The solid was refluxed in MeOH (1 L) with chloranil (11 g, 0.045 mol) for 1 hour, cooled, and concentrated. The solid was filtered and vacuum dried at 60 °C to obtain Intermediate 10-1.

### synthesis of Intermediate 10-2

A 1 L single-neck round-bottom flask was charged with Intermediate 10-1 (23 g, 0.054 mol), HSTU (N,N,N',N'-Tetramethyl-O-(N-succinimidyl)uronium hexafluorophosphate) (23.1 g, 0.06 mol), triethylamine (22 mL, 0.161 mol), and DMF (230 mL), and the mixture was stirred at room temperature for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 10-2.

### synthesis of Intermediate 10-3

A 500 mL single-neck round-bottom flask was charged with Intermediate 10-2 (18.5 g, 0.0275 mol), 6-aminohexanoic acid (5.68 g, 0.055 mol), triethylamine (11.5 mL, 0.083 mol), and DMF (185 mL), and the mixture was stirred at 40 °C for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 10-3.

### synthesis of Compound 10

A 250 mL single-neck round-bottom flask was charged with Intermediate 10-3 (1 g, 1.5 mmol), HSTU (N,N,N',N'-Tetramethyl-O-(N-succinimidyl)uronium hexafluorophosphate) (0.64 g, 1.8 mmol), triethylamine (0.62 mL, 4 mmol), and dichloromethane (100 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 10 (0.8 g).

The ¹H-NMR data of the obtained Compound 10 is as follows:
¹H-NMR (400 MHz, CDCl₃) δ 8.74 (m, 1H), 8.01 (s, 1H), 7.94 (m, 1H), 7.78 (t, 2H, J = 6.0 Hz), 7.37 (d, 1H, J = 7.6 Hz), 6.95 (s, 2H), 6.84 (s, 2H), 3.52-3.41 (m, 6H), 2.80 (m, 6H), 2.12 (s, 6H), 2.05 (s, 3H), 1.94 (m, 6H), 1.27 (t, 6H, J = 6.8 Hz).

### Synthesis Example 11. synthesis of Compound 11

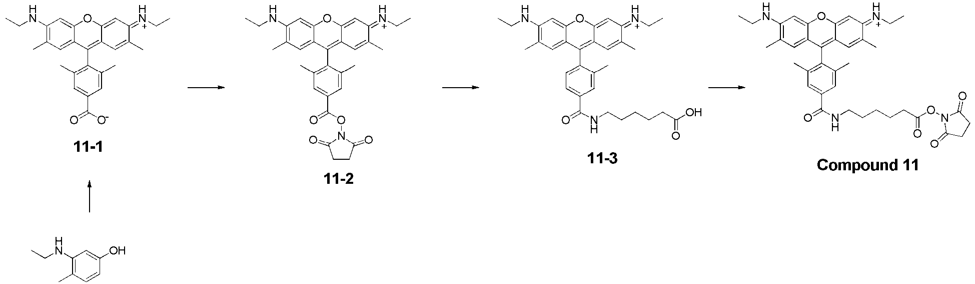

### synthesis of Intermediate 11-1

A 250 mL single-neck round-bottom flask was charged with 3-ethylamino-p-cresol (25.0 g, 0.165 mol), 4-formyl-3,5-methylbenzoic acid (14.7 g, 0.083 mol), and 70% sulfuric acid (110 mL), and the mixture was stirred at 130 °C for 3 hours. After cooling, the reaction mixture was poured into cold water and neutralized. The resulting solid was filtered and dried. The solid was refluxed in MeOH (1 L) with chloranil (11 g, 0.045 mol) for 1 hour, cooled, and concentrated. The solid was filtered and vacuum dried at 60 °C to obtain Intermediate 11-1.

### synthesis of Intermediate 11-2

A 1 L single-neck round-bottom flask was charged with Intermediate 11-1 (20 g, 0.045 mol), HSTU (N,N,N',N'-Tetramethyl-O-(N-succinimidyl)uronium hexafluorophosphate) (19.5 g, 0.054 mol), triethylamine (19 mL, 0.136 mol), and DMF (200 mL), and the mixture was stirred at room temperature for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 11-2.

### synthesis of Intermediate 11-3

A 500 mL single-neck round-bottom flask was charged with Intermediate 11-2 (17.0 g, 0.0275 mol), 4-aminohexanoic acid (5.11 g, 0.050 mol), triethylamine (10.4 mL, 0.074 mol), and DMF (170 mL), and the mixture was stirred at 40 °C for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 11-3.

### synthesis of Compound 11

A 250 mL single-neck round-bottom flask was charged with Intermediate 11-3 (9.0 g, 0.013 mol), HSTU (N,N,N',N'-Tetramethyl-O-(N-succinimidyl)uronium hexafluorophosphate) (7.2 g, 0.02 mol), triethylamine (5.5 mL, 0.04 mol), and dichloromethane (90 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 11 (8.2 g).

The ¹H-NMR data of the obtained Compound 11 is as follows:
¹H-NMR (400 MHz, DMSO-d₆) δ 8.77 (m, 1H), 7.98 (d, 2H, J = 7.8 Hz), 7.78 (t, 2H, J = 6.0 Hz), 6.97 (s, 2H), 6.82 (s, 2H), 3.49-3.40 (m, 6H), 2.77 (m, 6H), 2.11 (s, 6H), 2.06 (s, 6H), 1.94 (m, 2H), 1.28 (t, 6H, J = 6.8 Hz).

### Synthesis Example 12. synthesis of Compound 12

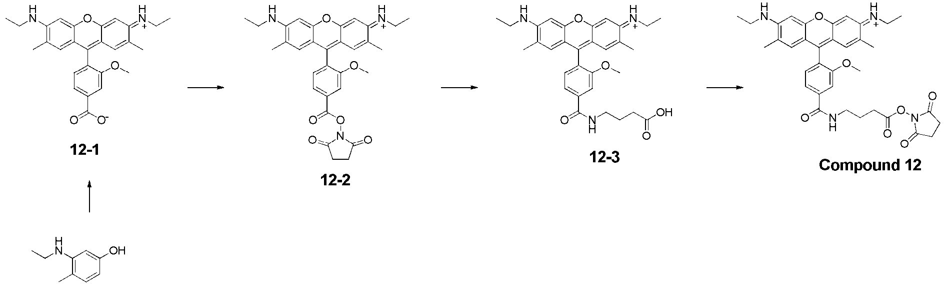

### synthesis of Intermediate 12-1

A 250 mL single-neck round-bottom flask was charged with 3-ethylamino-p-cresol (25.0 g, 0.165 mol), 4-formyl-3-methoxy-methylbenzoic acid (14.7 g, 0.083 mol), and 70% sulfuric acid (110 mL), and the mixture was stirred at 130 °C for 3 hours. After cooling, the reaction mixture was poured into cold water and neutralized. The resulting solid was filtered and dried. The solid was refluxed in MeOH (1 L) with chloranil (11.0 g, 0.045 mol) for 1 hour, cooled, and concentrated. The solid was filtered and vacuum dried at 60 °C to obtain Intermediate 12-1.

### synthesis of Intermediate 12-2

A 1 L single-neck round-bottom flask was charged with Intermediate 12-1 (10.0 g, 0.023 mol), HSTU (N,N,N',N'-Tetramethyl-O-(N-succinimidyl)uronium hexafluorophosphate) (9.7 g, 0.027 mol), triethylamine (9.4 mL, 0.067 mol), and DMF (100 mL), and the mixture was stirred at room temperature for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 12-2.

### synthesis of Intermediate 12-3

A 500 mL single-neck round-bottom flask was charged with Intermediate 12-2 (10.0 g, 0.015 mol), 4-aminobutanoic acid (3.07 g, 0.030 mol), triethylamine (6.22 mL, 0.045 mol), and DMF (100 mL), and the mixture was stirred at 40 °C for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 12-3.

### synthesis of Compound 12

A 250 mL single-neck round-bottom flask was charged with Intermediate 12-3 (5.0 g, 0.0074 mol), HSTU (N,N,N',N'-Tetramethyl-O-(N-succinimidyl)uronium hexafluorophosphate) (4.0 g, 0.011 mol), triethylamine (3.1 mL, 0.02 mol), and dichloromethane (50 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 12 (4.0 g).

The ¹H-NMR data of the obtained Compound 12 is as follows:
¹H-NMR (400 MHz, DMSO-d₆) δ 8.72 (m, 1H), 8.03 (s, 1H), 7.91 (m, 1H), 7.76 (t, 2H, J = 6.0 Hz), 7.34 (d, 1H, J = 7.6 Hz), 6.99 (s, 2H), 6.82 (s, 2H), 3.89 (s, 3H), 3.55-3.40 (m, 6H), 2.77 (m, 6H), 2.12 (s, 6H), 1.94 (m, 2H), 1.29 (t, 6H, J = 6.8 Hz).

### Synthesis Example 13. synthesis of Compound 13

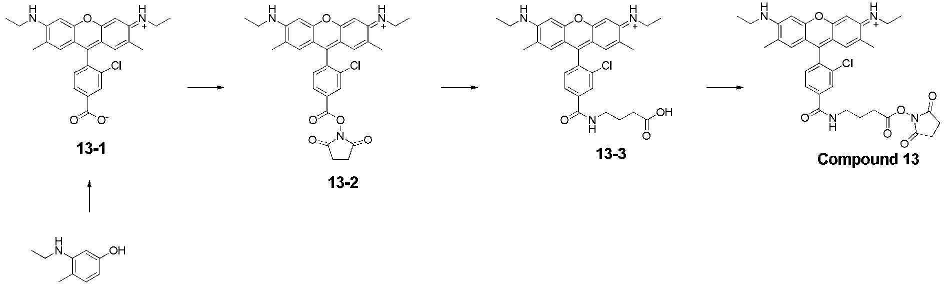

### synthesis of Intermediate 13-1

A 250 mL single-neck round-bottom flask was charged with 3-ethylamino-p-cresol (25.0 g, 0.165 mol), 3-chloro-4-formylmethylbenzoic acid (15.3 g, 0.083 mol), and 70% sulfuric acid (110 mL), and the mixture was stirred at 130 °C for 3 hours. After cooling, the reaction mixture was poured into cold water and neutralized. The resulting solid was filtered and dried. The solid was refluxed in MeOH (1 L) with chloranil (11.0 g, 0.045 mol) for 1 hour, cooled, and concentrated. The solid was filtered and vacuum dried at 60 °C to obtain Intermediate 13-1.

### synthesis of Intermediate 13-2

A 1 L single-neck round-bottom flask was charged with Intermediate 13-1 (10.0 g, 0.023 mol), HSTU (N,N,N',N'-Tetramethyl-O-(N-succinimidyl)uronium hexafluorophosphate) (9.7 g, 0.027 mol), triethylamine (9.4 mL, 0.067 mol), and DMF (100 mL), and the mixture was stirred at room temperature for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 13-2.

### synthesis of Intermediate 13-3

A 500 mL single-neck round-bottom flask was charged with Intermediate 13-2 (10.0 g, 0.015 mol), 4-aminobutanoic acid (3.07 g, 0.030 mol), triethylamine (6.22 mL, 0.045 mol), and DMF (100 mL), and the mixture was stirred at 40 °C for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 13-3.

### synthesis of Compound 13

A 250 mL single-neck round-bottom flask was charged with Intermediate 13-3 (5.0 g, 0.0074 mol), HSTU (N,N,N',N'-Tetramethyl-O-(N-succinimidyl)uronium hexafluorophosphate) (4.0 g, 0.011 mol), triethylamine (3.1 mL, 0.02 mol), and dichloromethane (50 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 13 (4.2 g).

The ¹H-NMR data of the obtained Compound 13 is as follows:
¹H-NMR (400 MHz, DMSO-d₆) δ 8.73 (m, 1H), 8.00 (s, 1H), 7.87 (m, 1H), 7.76 (t, 2H, J = 6.0 Hz), 7.37 (d, 1H, J = 7.6 Hz), 6.90 (s, 2H), 6.83 (s, 2H), 3.50-3.42 (m, 6H), 2.79 (m, 6H), 2.11 (s, 6H), 1.97 (m, 2H), 1.27 (t, 6H, J = 6.8 Hz).

### Synthesis Example 14. synthesis of Compound 14

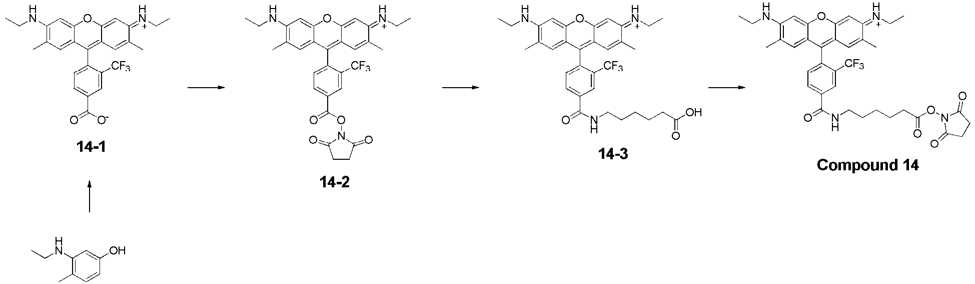

### synthesis of Intermediate 14-1

A 250 mL single-neck round-bottom flask was charged with 3-ethylamino-p-cresol (5.0 g, 0.033 mol), 4-formyl-3-trifluorobenzoic acid (3.6 g, 0.016 mol), and 70% sulfuric acid (25 mL), and the mixture was stirred at 130 °C for 3 hours. After cooling, the reaction mixture was poured into cold water and neutralized. The resulting solid was filtered and dried. The solid was refluxed in MeOH (1 L) with chloranil (2.0 g, 0.008 mol) for 1 hour, cooled, and concentrated. The solid was filtered and vacuum dried at 60 °C to obtain Intermediate 14-1.

### synthesis of Intermediate 14-2

A 1 L single-neck round-bottom flask was charged with Intermediate 14-1 (3.5 g, 7.3 mmol), HSTU (N,N,N',N'-Tetramethyl-O-(N-succinimidyl)uronium hexafluorophosphate) (3.13 g, 8.7 mmol), triethylamine (3.0 mL, 2.2 mmol), and DMF (35 mL), and the mixture was stirred at room temperature for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 14-2.

### synthesis of Intermediate 14-3

A 500 mL single-neck round-bottom flask was charged with Intermediate 14-2 (4.0 g, 5.5 mmol), 6-aminohexanoic acid (1.14 g, 11.0 mmol), triethylamine (2.3 mL, 17.0 mmol), and DMF (40 mL), and the mixture was stirred at 40 °C for 1 hour. The reaction mixture was concentrated, water was added, and the mixture was vigorously stirred, followed by extraction with dichloromethane. The organic layer was dried over anhydrous sodium sulfate for 5 minutes and filtered. The filtrate was concentrated and purified by column chromatography to obtain Intermediate 14-3.

### synthesis of Compound 14

A 250 mL single-neck round-bottom flask was charged with Intermediate 14-3 (3.0 g, 4.2 mmol), HSTU (N,N,N',N'-Tetramethyl-O-(N-succinimidyl)uronium hexafluorophosphate) (2.27 g, 6.3 mmol), triethylamine (1.8 mL, 12.6 mmol), and dichloromethane (30 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 14 (2.2 g).

The ¹H-NMR data of the obtained Compound 14 is as follows:
¹H-NMR (400 MHz, DMSO-d₆) δ 8.75 (bs, 1H), 7.95 (s, 1H), 7.86 (m, 1H), 7.76 (t, 2H, J = 6.0 Hz), 7.35 (d, 1H, J = 7.6 Hz), 6.91 (s, 2H), 6.82 (s, 2H), 3.50-3.42 (m, 6H), 2.79 (m, 6H), 2.11 (s, 6H), 1.97 (m, 6H), 1.27 (t, 6H, J = 6.8 Hz).

### Synthesis Example 15. synthesis of Compound 15

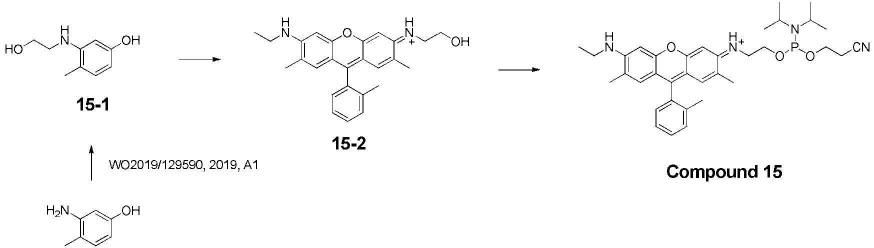

### synthesis of Intermediate 15-1

Intermediate 15-1 was synthesized with reference to WO2019/129590, 2019, A1.

### synthesis of Intermediate 15-2

A 50 mL single-neck round-bottom flask was charged with Intermediate 15-1 (2.41 g, 0.016 mol), 3-ethylamino-p-cresol (2.5 g, 0.016 mol), o-tolualdehyde (1.92 g, 0.016 mol), and 70% sulfuric acid (15 mL), and the mixture was stirred at 130 °C for 3 hours. After cooling, the reaction mixture was poured into cold water and neutralized. The resulting solid was filtered and vacuum dried at 60 °C to obtain Intermediate 15-2.

### synthesis of Compound 15

A 25 mL single-neck round-bottom flask was charged with Intermediate 15-2 (1.0 g, 1.8 mmol), 2-cyanoethyl N,N'-diisopropylchlorophosphoramidite (0.56 g, 2.4 mmol), triethylamine (0.76 mL, 5.5 mmol), and dichloromethane (10 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 15 (1.4 g).

The ¹H-NMR data of the obtained Compound 15 is as follows:
¹H-NMR (400 MHz, DMSO-d₆) δ 7.76 (bs, 2H), 7.35-7.10 (m, 4H), 6.90 (s, 2H), 6.77 (s, 2H), 3.50-3.42 (m, 10H), 2.69 (t, 2H, J = 5.6 Hz), 2.15 (s, 6H), 2.06 (s, 3H), 1.48 (t, 3H, J = 6.8 Hz), 1.20-1.18 (m, 12H).

### Synthesis Example 16. synthesis of Compound 16

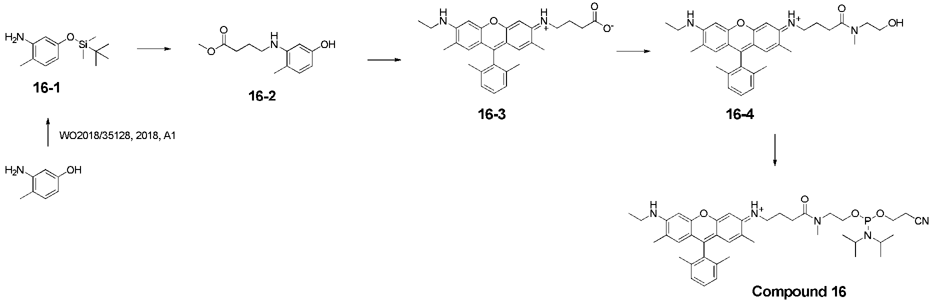

### synthesis of Intermediate 16-1

Intermediate 16-1 was synthesized with reference to WO2018/35128, 2018, A1.

### synthesis of Intermediate 16-2

A 1 L single-neck round-bottom flask was charged with Intermediate 16-1 (50.0 g, 0.211 mol), methyl 4-butylate (45.5 g, 0.253 mol), potassium carbonate (58.3 g, 0.422 mol), and dimethylformamide (500 mL), and the mixture was stirred at 60 °C for 24 hours. After cooling, the mixture was concentrated. Dichloromethane (500 mL) and water (200 mL) were added, stirred for 5 minutes, and the organic layer was separated. 6N aqueous hydrochloric acid (100 mL) was added, stirred for 5 minutes, and the organic layer was separated. The organic layer was dried over anhydrous sodium sulfate for 5 minutes and filtered. The filtrate was concentrated and purified by column chromatography to obtain Intermediate 16-2.

### synthesis of Intermediate 16-3

A 50 mL single-neck round-bottom flask was charged with Intermediate 16-2 (3.57 g, 0.016 mol), 3-ethylamino-p-cresol (2.5 g, 0.016 mol), o-tolualdehyde (1.92 g, 0.016 mol), and 70% sulfuric acid (15 mL), and the mixture was stirred at 130 °C for 3 hours. After cooling, the reaction mixture was poured into cold water and neutralized. The resulting solid was filtered and vacuum dried at 60 °C to obtain Intermediate 16-3.

### synthesis of Intermediate 16-4

A 100 mL single-neck round-bottom flask was charged with Intermediate 16-3 (3.19 g, 0.0070 mol), HSTU (N,N,N',N'-Tetramethyl-O-(N-succinimidyl)uronium hexafluorophosphate) (3.0 g, 0.0084 mol), triethylamine (3.0 mL, 0.021 mol), and dimethylformamide (32 mL), and the mixture was stirred at room temperature for 1 hour. 2-(Methylamino)ethanol (1.0 g, 0.014 mol) was added and stirred at room temperature for 2 hours. The mixture was concentrated, water was added, and the mixture was vigorously stirred and extracted with dichloromethane. The organic layer was dried over anhydrous sodium sulfate for 5 minutes and filtered. The filtrate was concentrated and purified by column chromatography to obtain Intermediate 16-4.

### synthesis of Compound 16

A 25 mL single-neck round-bottom flask was charged with Intermediate 16-4 (1.0 g, 1.5 mmol), 2-cyanoethyl N,N'-diisopropylchlorophosphoramidite (0.47 g, 2.0 mmol), triethylamine (0.86 mL, 4.5 mmol), and dichloromethane (10 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 16 (0.7 g).

The ¹H-NMR data of the obtained Compound 16 is as follows:
¹H-NMR (400 MHz, DMSO-d₆) δ 7.86 (bs, 2H), 7.42 (d, 2H, J = 7.8 Hz), 7.32 (t, 1H, J = 7.8 Hz), 6.90 (s, 2H), 6.77 (s, 2H), 3.50-3.42 (m, 15H), 2.69 (t, 2H, J = 5.6 Hz), 2.48 (t, 2H, J = 6.0 Hz), 2.11 (s, 6H), 2.07 (s, 6H), 1.48 (m, 5H), 1.20-1.18 (m, 12H).

### Synthesis Example 17. synthesis of Compound 17

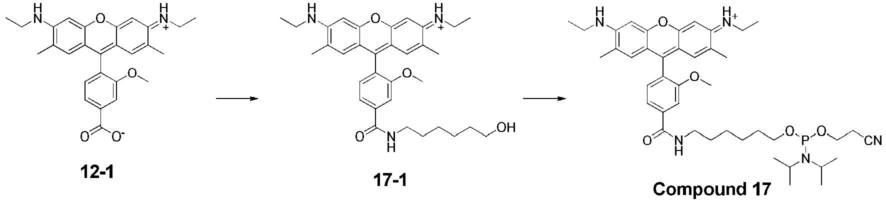

### synthesis of Intermediate 17-1

A 100 mL single-neck round-bottom flask was charged with Intermediate 12-1 (3.19 g, 0.0070 mol), HSTU (N,N,N',N'-Tetramethyl-O-(N-succinimidyl)uronium hexafluorophosphate) (3.0 g, 0.0084 mol), triethylamine (3.0 mL, 0.021 mol), and dimethylformamide (32 mL), and the mixture was stirred at room temperature for 1 hour. 6-Amino-1-hexanol (1.0 g, 0.014 mol) was added and the mixture was stirred at room temperature for 2 hours. After concentration, water was added to the reaction mixture, which was vigorously stirred and extracted with dichloromethane. The organic layer was dried over anhydrous sodium sulfate for 5 minutes and filtered. The filtrate was concentrated and purified by column chromatography to obtain Intermediate 17-1.

### synthesis of Compound 17

A 25 mL single-neck round-bottom flask was charged with Intermediate 17-1 (1.0 g, 1.5 mmol), 2-cyanoethyl N,N'-diisopropylchlorophosphoramidite (0.48 g, 2.0 mmol), triethylamine (0.65 mL, 4.5 mmol), and dichloromethane (10 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 17 (0.8 g).

The ¹H-NMR data of the obtained Compound 17 is as follows:
¹H-NMR (400 MHz, DMSO-d₆) δ 8.72 (bs, 1H), 8.03 (s, 1H), 7.91 (m, 1H), 7.76 (t, 2H, J = 6.0 Hz), 7.34 (d, 1H, J = 7.6 Hz), 6.99 (s, 2H), 6.82 (s, 2H), 3.89 (s, 3H), 3.55-3.40 (m, 12H), 2.77 (t, 2H, J = 5.8 Hz), 2.12 (s, 6H), 1.28-1.22 (m, 14H), 1.20-1.18 (m, 12H).

### Synthesis Example 18. synthesis of Compound 18

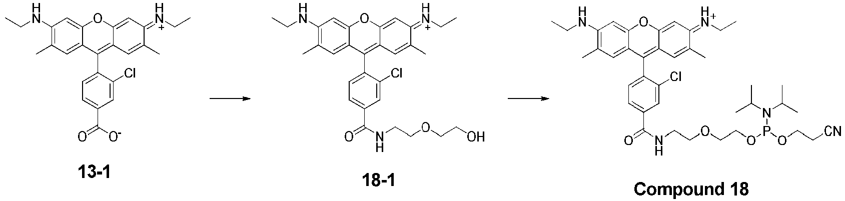

### synthesis of Intermediate 18-1

A 100 mL single-neck round-bottom flask was charged with Intermediate 13-1 (2.0 g, 4.5 mmol), HSTU (N,N,N',N'-Tetramethyl-O-(N-succinimidyl)uronium hexafluorophosphate) (2.4 g, 6.7 mmol), triethylamine (1.2 mL, 8.9 mmol), and dimethylformamide (32 mL), and the mixture was stirred at room temperature for 1 hour. 2-(2-Aminoethoxy)ethanol (0.5 g, 4.5 mmol) was added and the mixture was stirred at room temperature for 2 hours. After concentration, water was added to the reaction mixture, which was vigorously stirred and extracted with dichloromethane. The organic layer was dried over anhydrous sodium sulfate for 5 minutes and filtered. The filtrate was concentrated and purified by column chromatography to obtain Intermediate 18-1.

### synthesis of Compound 18

A 25 mL single-neck round-bottom flask was charged with Intermediate 18-1 (1.0 g, 1.5 mmol), 2-cyanoethyl N,N'-diisopropylchlorophosphoramidite (0.45 g, 2.0 mmol), triethylamine (0.61 mL, 4.5 mmol), and dichloromethane (10 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 18 (0.9 g).

The ¹H-NMR data of the obtained Compound 18 is as follows:
¹H-NMR (400 MHz, DMSO-d₆) δ 8.74 (m, 1H), 8.02 (s, 1H), 7.90 (m, 1H), 7.76 (t, 2H, J = 6.0 Hz), 7.36 (d, 1H, J = 7.6 Hz), 6.91 (s, 2H), 6.82 (s, 2H), 3.63-3.40 (m, 16H), 2.79 (t, 2H, J = 6.0 Hz), 2.11 (s, 6H), 1.28-1.22 (t, 6H, J = 5.8 Hz), 1.20-1.18 (m, 12H).

### Synthesis Example 19. synthesis of Compound 19

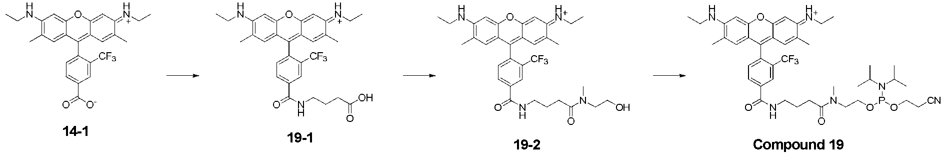

### synthesis of Intermediate 19-1

A 100 mL single-neck round-bottom flask was charged with Intermediate 14-1 (3.0 g, 6.7 mmol), HSTU (N,N,N',N'-Tetramethyl-O-(N-succinimidyl)uronium hexafluorophosphate) (3.6 g, 10.0 mmol), triethylamine (1.8 mL, 13.4 mmol), and dimethylformamide (30 mL), and the mixture was stirred at room temperature for 1 hour. 4-Aminobutyric acid (0.5 g, 4.5 mmol) was added and stirred at room temperature for 12 hours. After concentration, water was added to the reaction mixture, which was vigorously stirred and extracted with dichloromethane. The organic layer was dried over anhydrous sodium sulfate for 5 minutes and filtered. The filtrate was concentrated and purified by column chromatography to obtain Intermediate 19-1.

### synthesis of Intermediate 19-2

A 100 mL single-neck round-bottom flask was charged with Intermediate 19-1 (2.5 g, 3.5 mmol), HSTU (1.9 g, 5.3 mmol), triethylamine (1.0 mL, 7.0 mmol), and dimethylformamide (25 mL), and the mixture was stirred at room temperature for 1 hour. 2-(Methylamino)ethanol (0.3 g, 3.5 mmol) was added and stirred at room temperature for 2 hours. After concentration, water was added to the reaction mixture, which was vigorously stirred and extracted with dichloromethane. The organic layer was dried over anhydrous sodium sulfate for 5 minutes and filtered. The filtrate was concentrated and purified by column chromatography to obtain Intermediate 19-2.

### synthesis of Compound 19

A 25 mL single-neck round-bottom flask was charged with Intermediate 19-2 (1.0 g, 1.3 mmol), 2-cyanoethyl N,N'-diisopropylchlorophosphoramidite (0.40 g, 1.7 mmol), triethylamine (0.54 mL, 3.9 mmol), and dichloromethane (10 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 19 (0.7 g).

The ¹H-NMR data of the obtained Compound 19 is as follows:
¹H-NMR (400 MHz, DMSO-d₆) δ 8.71 (bs, 1H), 7.94 (s, 1H), 7.84 (m, 1H), 7.79 (t, 2H, J = 6.0 Hz), 7.38 (d, 1H, J = 7.6 Hz), 6.90 (s, 2H), 6.84 (s, 2H), 3.66-3.38 (m, 17H), 2.78 (t, 2H, J = 6.0 Hz), 2.42 (t, 2H, J = 6.0 Hz), 2.10 (s, 6H), 1.29-1.23 (m, 8H), 1.20-1.16 (m, 12H).

### Synthesis Example 20. synthesis of Compound 20

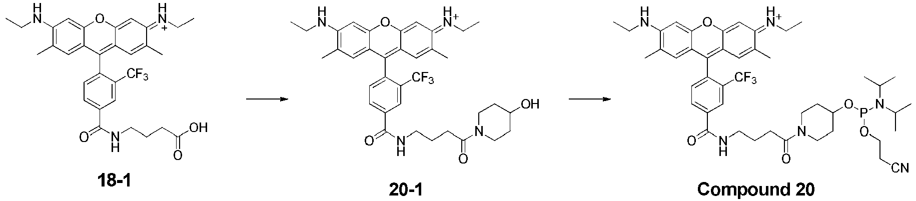

### synthesis of Intermediate 20-1

A 100 mL single-neck round-bottom flask was charged with Intermediate 18-1 (2.0 g, 2.8 mmol), HSTU (N,N,N',N'-Tetramethyl-O-(N-succinimidyl)uronium hexafluorophosphate) (1.5 g, 4.2 mmol), triethylamine (0.8 mL, 5.6 mmol), and dimethylformamide (20 mL), and the mixture was stirred at room temperature for 1 hour. 4-Hydroxypiperidine (0.2 g, 2.8 mmol) was added and stirred at room temperature for 1 hour. After concentration, water was added to the reaction mixture, which was vigorously stirred and extracted with dichloromethane. The organic layer was dried over anhydrous sodium sulfate for 5 minutes and filtered. The filtrate was concentrated and purified by column chromatography to obtain Intermediate 20-1.

### synthesis of Compound 20

A 25 mL single-neck round-bottom flask was charged with Intermediate 20-1 (1.0 g, 1.3 mmol), 2-cyanoethyl N,N'-diisopropylchlorophosphoramidite (0.40 g, 1.6 mmol), triethylamine (0.52 mL, 3.8 mmol), and dichloromethane (10 mL), and the mixture was stirred at room temperature for 1 hour. Water was added, and the mixture was vigorously stirred, followed by extraction with dichloromethane. The organic layer was dried over anhydrous sodium sulfate for 5 minutes and filtered. The filtrate was concentrated and purified by column chromatography to obtain Compound 20 (0.9 g).

The ¹H-NMR data of the obtained Compound 20 is as follows:
¹H-NMR (400 MHz, DMSO-d₆) δ 8.70 (bs, 1H), 7.91 (s, 1H), 7.87 (m, 1H), 7.75 (t, 2H, J = 6.0 Hz), 7.37 (d, 1H, J = 7.6 Hz), 6.92 (s, 2H), 6.83 (s, 2H), 4.13 (m, 1H), 3.76-3.48 (m, 14H), 2.77 (t, 2H, J = 6.0 Hz), 2.41 (t, 2H, J = 6.0 Hz), 2.10 (s, 6H), 2.07-1.77 (m, 4H), 1.29-1.23 (m, 8H), 1.20-1.16 (m, 12H).

### Synthesis Example 21. synthesis of Compound 21

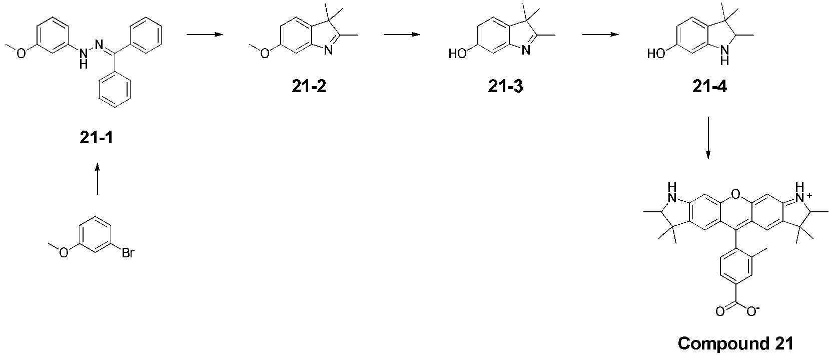

### synthesis of Intermediate 21-1

A 3 L four-neck round-bottom flask was charged with 3-bromoanisole (150 g, 0.802 mol), benzophenone hydrazone (189 g, 0.962 mol), sodium tert-butoxide (108 g, 1.12 mol), Pd₂(dba)₃ (29.4 g, 0.032 mol), BINAP (20.0 g, 0.032 mol), and toluene (1500 mL), and the mixture was stirred at 100°C for 24 hours. After cooling, the reaction mixture was filtered through celite. The filtrate was concentrated and purified by column chromatography to obtain Intermediate 21-1.

### synthesis of Intermediate 21-2

A 3 L four-neck round-bottom flask was charged with Intermediate 21-1 (200 g, 0.661 mol), 3-methyl-2-butanone (85.5 g, 0.992 mol), concentrated hydrochloric acid (400 mL), and ethanol (1600 mL), and the mixture was stirred at 100°C for 24 hours. After cooling and concentration, the product was purified by column chromatography to obtain Intermediate 21-2.

### synthesis of Intermediate 21-3

A 1 L four-neck round-bottom flask was charged with Intermediate 21-2 (40.0 g, 0.211 mol), boron tribromide (40.7 mL, 0.423 mol), and dichloromethane (400 mL), and the mixture was stirred at room temperature for 4 hours. The reaction mixture was poured into 5% aqueous sodium bicarbonate solution (1 L) and vigorously stirred. The organic layer was separated, dried over anhydrous sodium sulfate for 5 minutes, and filtered. The filtrate was concentrated and purified by column chromatography to obtain Intermediate 21-3.

### synthesis of Intermediate 21-4

A 1 L four-neck round-bottom flask was charged with Intermediate 21-3 (40.0 g, 0.211 mol), boron tribromide (40.7 mL, 0.423 mol), and dichloromethane (400 mL), and the mixture was stirred at room temperature for 4 hours. The reaction mixture was poured into 5% aqueous sodium bicarbonate solution (1 L) and vigorously stirred. The organic layer was separated, dried over anhydrous sodium sulfate for 5 minutes, and filtered. The filtrate was concentrated and purified by column chromatography to obtain Intermediate 21-4.

### synthesis of Compound 21

A 250 mL single-neck round-bottom flask was charged with Intermediate 21-4 (27.9 g, 0.157 mol), 4-formyl-3-methylbenzoic acid (12.9 g, 0.079 mol), and 70% sulfuric acid (90 mL), and the mixture was stirred at 130°C for 3 hours. The reaction mixture was poured into cold water and neutralized. The resulting solid was filtered, dried, and then refluxed in methanol (1 L) with chloranil (9.7 g, 0.039 mol) for 1 hour. After cooling, the mixture was concentrated, and the resulting solid was filtered and vacuum dried at 60°C to obtain Compound 21 (12.0 g).

The ¹H-NMR data of the obtained Compound 21 is as follows:
¹H-NMR (400 MHz, DMSO-d₆) δ 8.01 (s, 1H), 7.93 (d, 1H, J = 7.8 Hz), 7.37 (d, 1H, J = 7.8 Hz), 6.91 (s, 2H), 3.81 (q, 2H, J = 6.0 Hz), 2.06 (s, 3H), 1.20 (s, 12H), 1.04 (s, 6H).

### Synthesis Example 22. synthesis of Compound 22

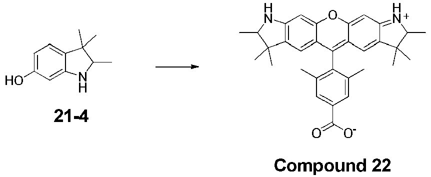

### synthesis of Compound 22

A 50 mL single-neck round-bottom flask was charged with Intermediate 21-4 (5.0 g, 0.028 mol), 4-formyl-3,5-methylbenzoic acid (2.51 g, 0.014 mol), and 70% sulfuric acid (10 mL), and the mixture was stirred at 130°C for 3 hours. The reaction mixture was poured into cold water and neutralized. The resulting solid was filtered, dried, and then refluxed in methanol (50 mL) with chloranil (1.7 g, 0.007 mol) for 1 hour. After cooling, the mixture was concentrated, and the resulting solid was filtered and vacuum dried at 60°C to obtain Compound 22 (2.3 g).

The ¹H-NMR data of the obtained Compound 22 is as follows:
¹H-NMR (400 MHz, DMSO-d₆) δ 8.05 (s, 2H), 6.90 (s, 2H), 6.87 (s, 2H), 3.81 (q, 2H, J = 6.0 Hz), 2.07 (s, 6H), 1.20 (s, 12H), 1.04 (s, 6H).

### Synthesis Example 23. synthesis of Compound 23

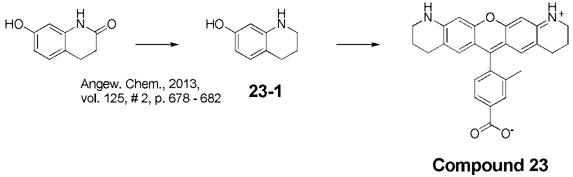

### synthesis of Intermediate 23-1

Intermediate 23-1 was synthesized with reference to Angew. Chem., 2013, vol. 125, # 2, p. 678-682.

### synthesis of Compound 23

Compound 23 was synthesized with reference to the method described in Synthesis Example 21.

The ¹H-NMR data of the obtained Compound 23 is as follows:
¹H-NMR (400 MHz, DMSO-d₆) δ 8.03 (s, 1H), 7.95 (d, 1H, J = 7.8 Hz), 7.36 (d, 1H, J = 7.8 Hz), 6.91 (s, 2H), 6.84 (s, 2H), 3.56 (m, 4H), 2.70 (m, 4H), 2.07 (s, 3H), 1.78 (m, 4H).

### Synthesis Example 24. synthesis of Compound 24

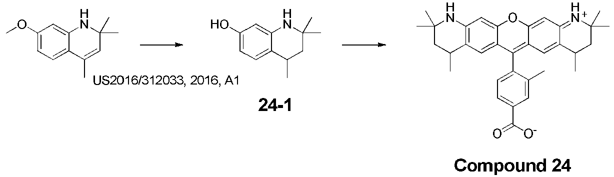

### synthesis of Intermediate 24-1

Intermediate 24-1 was synthesized with reference to US2016/312033, 2016, A1.

### synthesis of Compound 24

Compound 24 was synthesized with reference to the method described in Synthesis Example 21.

The ¹H-NMR data of the obtained Compound 24 is as follows:
¹H-NMR (400 MHz, DMSO-d₆) δ 8.03 (s, 1H), 7.92 (d, 1H, J = 7.8 Hz), 7.34 (d, 1H, J = 7.8 Hz), 6.90 (s, 2H), 6.82 (s, 2H), 2.86 (m, 2H), 2.05 (s, 3H), 1.73-1.55 (m, 4H), 1.30 (s, 6H), 1.24 (d, 6H, J = 6.4 Hz), 1.15 (s, 6H).

### Synthesis Example 25. synthesis of Compound 25

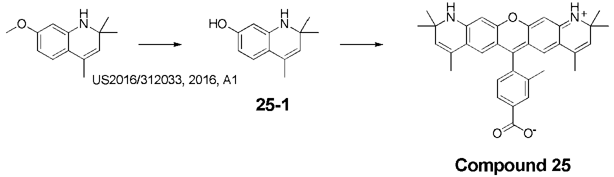

### synthesis of Intermediate 25-1

Intermediate 25-1 was synthesized with reference to US2016/312033, 2016, A1.

### synthesis of Compound 25

Compound 25 was synthesized with reference to the method described in Synthesis Example 21.

The ¹H-NMR data of the obtained Compound 25 is as follows:
¹H-NMR (400 MHz, DMSO-d₆) δ 8.05 (s, 1H), 7.97 (d, 1H, J = 7.8 Hz), 7.39 (d, 1H, J = 7.8 Hz), 6.94 (s, 2H), 6.87 (s, 2H), 5.31 (s, 2H), 2.07 (s, 3H), 1.94 (s, 6H), 1.26 (s, 12H).

### Synthesis Example 26. synthesis of Compound 26

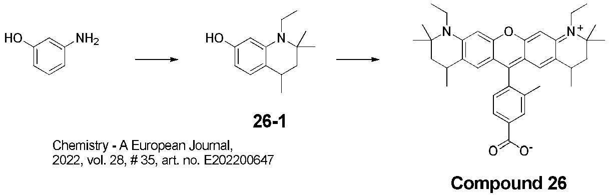

### synthesis of Intermediate 26-1

Intermediate 26-1 was synthesized with reference to Chemistry - A European Journal, 2022, vol. 28, # 35, art. no. E202200647.

### synthesis of Compound 26

Compound 26 was synthesized with reference to the method described in Synthesis Example 21.

The ¹H-NMR data of the obtained Compound 26 is as follows:
¹H-NMR (400 MHz, DMSO-d₆) δ 8.01 (s, 1H), 7.93 (d, 1H, J = 7.8 Hz), 7.31 (d, 1H, J = 7.8 Hz), 6.92 (s, 2H), 6.80 (s, 2H), 3.86-3.50 (m, 4H), 2.86 (m, 2H), 2.05 (s, 3H), 1.73-1.55 (m, 4H), 1.36 (t, 6H, J = 6.8 Hz), 1.29 (s, 6H), 1.22 (d, 6H, J = 6.4 Hz), 1.17 (s, 6H).

### Synthesis Example 27. synthesis of Compound 27

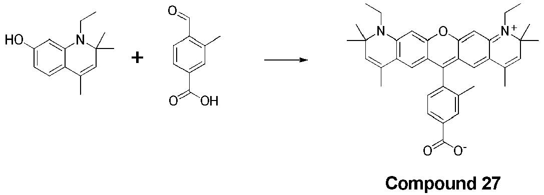

### synthesis of Compound 27

Compound 27 was synthesized with reference to the method described in Synthesis Example 21.

The ¹H-NMR data of the obtained Compound 27 is as follows:
¹H-NMR (400 MHz, DMSO-d₆) δ 8.07 (s, 1H), 7.96 (d, 1H, J = 7.8 Hz), 7.33 (d, 1H, J = 7.8 Hz), 6.92 (s, 2H), 6.86 (s, 2H), 5.33 (s, 2H), 3.88-3.54 (m, 4H), 2.05 (s, 3H), 1.97 (s, 6H), 1.35 (t, 6H, J = 6.8 Hz), 1.22 (s, 12H).

### Synthesis Example 28. synthesis of Compound 28

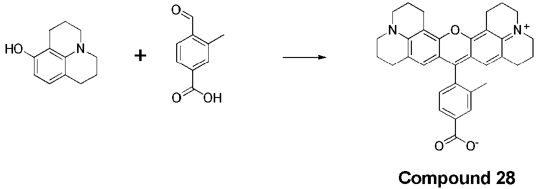

### synthesis of Compound 28

Compound 28 was synthesized with reference to the method described in Synthesis Example 21.

The ¹H-NMR data of the obtained Compound 28 is as follows:
¹H-NMR (400 MHz, DMSO-d₆) δ 7.91 (s, 1H), 7.83 (d, J = 7.60 Hz, 1H), 7.21 (d, J = 8.00 Hz, 1H), 6.65 (s, 2H), 3.58-3.48 (m, 8H), 3.09-3.06 (m, 4H), 2.82-2.76 (m, 4H), 2.21-1.85 (m, 8H), 2.08 (s, 3H).

### Synthesis Example 29. synthesis of Compound 29

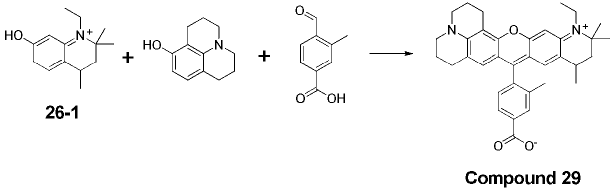

### synthesis of Compound 29

Compound 29 was synthesized with reference to the method described in Synthesis Example 21.

The ¹H-NMR data of the obtained Compound 29 is as follows:
¹H-NMR (400 MHz, DMSO-d₆) δ 8.03 (s, 1H), 7.93 (d, 1H, J = 7.8 Hz), 7.31 (d, 1H, J = 7.8 Hz), 6.92 (s, 1H), 6.79 (s, 1H), 6.65 (s, 1H), 3.86-3.50 (m, 6H), 3.09-2.86 (m, 3H), 3.09-3.06 (m, 2H), 2.82-2.76 (m, 2H), 2.21-1.85 (m, 2H), 2.05 (s, 3H), 1.73-1.55 (m, 2H), 1.36 (t, 3H, J = 6.8 Hz), 1.29 (s, 3H), 1.22 (d, 3H, J = 6.4 Hz), 1.17 (s, 3H).

### Synthesis Example 30. synthesis of Compound 30

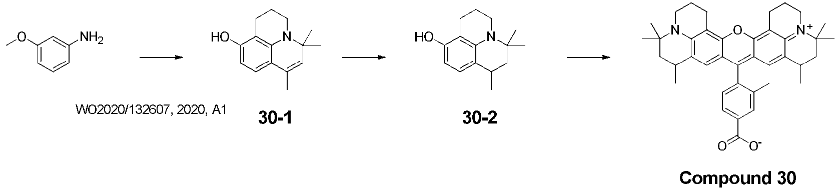

### synthesis of Intermediate 30-1

Intermediate 30-1 was synthesized with reference to WO2020/132607, 2020, A1.

### synthesis of Intermediate 30-2

A 500 mL single-neck flask was charged with Intermediate 30-1 (10 g, 0.04 mol), 10% Pd/C (0.5 g), and MeOH (100 mL), and the reaction mixture was stirred at room temperature under a hydrogen stream for 24 hours. The reaction mixture was filtered through Celite, and the filtrate was concentrated to afford Intermediate 30-2.

### synthesis of Compound 30

A 50 mL single-neck flask was charged with Intermediate 30-2 (3.0 g, 0.013 mol), 4-formyl-3-methylbenzoic acid (1.06 g, 0.0065 mol), and 70% sulfuric acid (7 mL), and the reaction mixture was stirred at 130°C for 3 hours. The reaction mixture was poured into cold water, neutralized, and the resulting solid was filtered. The solid was dried and then refluxed with MeOH (30 mL) and chloranil (0.8 g, 0.005 mol) for 1 hour. After cooling, the mixture was concentrated, and the resulting solid was filtered and dried under vacuum at 60°C to afford Compound 30 (1.7 g).

The ¹H-NMR data of the obtained Compound 30 is as follows:
¹H-NMR (400 MHz, DMSO-d₆) δ 8.03 (s, 1H), 7.94 (d, 1H, J = 7.8 Hz), 7.33 (d, 1H, J = 7.8 Hz), 6.81 (s, 2H), 3.59-3.47 (m, 4H), 3.10-3.06 (m, 2H), 2.81-2.75 (m, 4H), 2.20-1.83 (m, 7H), 1.74-1.51 (m, 4H), 1.29 (s, 6H), 1.22 (d, 6H, J = 6.4 Hz), 1.14 (s, 6H).

### Synthesis Example 31. synthesis of Compound 31

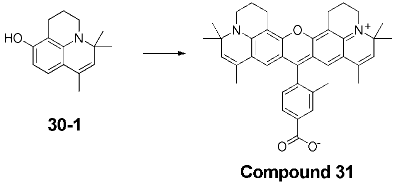

### synthesis of Compound 31

Compound 31 was synthesized with reference to the method described in Synthesis Example 21.

The ¹H-NMR data of the obtained Compound 31 is as follows:
¹H-NMR (400 MHz, DMSO-d₆) δ 8.07 (s, 1H), 7.96 (d, 1H, J = 7.8 Hz), 7.33 (d, 1H, J = 7.8 Hz), 6.64 (s, 2H), 5.33 (s, 2H), 3.88-3.54 (m, 4H), 3.56-3.27 (m, 2H), 3.09-3.06 (m, 2H), 2.82-2.76 (m, 2H), 2.21-1.85 (m, 2H), 2.05 (s, 3H), 1.97 (s, 6H), 1.22 (s, 12H).

### Synthesis Example 32. synthesis of Compound 32

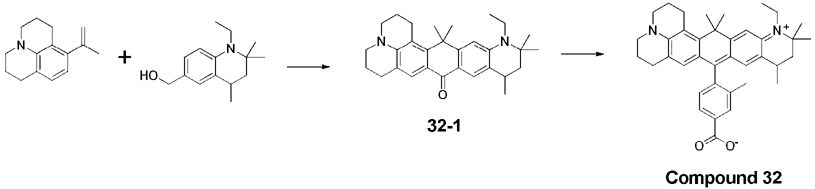

### synthesis of Intermediate 32-1

Intermediate 32-1 was synthesized with reference to Org. Lett. 2020, 2, 381-385.

### synthesis of Compound 32

A 100 mL three-neck flask was charged with Intermediate 32-1 (2.58 g, 0.0102 mol) and tetrahydrofuran (26 mL), and the mixture was stirred under a nitrogen atmosphere at -78°C for 5 minutes. n-Butyllithium (2.5 M, 4 mL, 0.0102 mol) was slowly added dropwise to the reaction mixture, which was stirred at -78°C for 1 hour. Intermediate 32-1 (1.5 g, 0.0034 mol) dissolved in tetrahydrofuran (30 mL) was added dropwise, and the mixture was stirred at room temperature for 2 hours. After concentration, 6 N aqueous hydrochloric acid (38 mL) was added, and the mixture was stirred at 80°C for 16 hours. The reaction mixture was concentrated, and the product was purified by column chromatography to afford Compound 32 (2.1 g).

The ¹H-NMR data of the obtained Compound 32 is as follows:
¹H-NMR (400 MHz, DMSO-d₆) δ 8.06 (s, 1H), 7.35 (s, 1H), 7.33 (s, 1H), 6.81 (s, 1H), 6.79 (s, 1H), 6.75 (s, 1H), 3.86-3.50 (m, 6H), 3.09-2.86 (m, 3H), 3.09-3.06 (m, 2H), 2.82-2.76 (m, 2H), 2.21-1.85 (m, 2H), 2.05 (s, 3H), 1.96 (s, 6H), 1.73-1.55 (m, 2H), 1.36 (t, 3H, J = 6.8 Hz), 1.29 (s, 3H), 1.22 (d, 3H, J = 6.4 Hz), 1.17 (s, 3H).

### Synthesis Example 33. synthesis of Compound 33

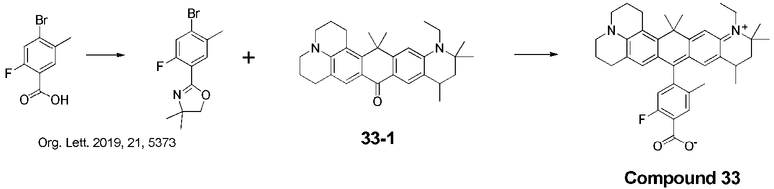

### synthesis of Intermediate 33-1

Intermediate 33-1 was synthesized with reference to Org. Lett. 2019, 21, 5373.

### synthesis of Compound 33

A 100 mL three-neck flask was charged with Intermediate 33-1 (1.13 g, 4 mmol) and tetrahydrofuran (8 mL), and the mixture was stirred under a nitrogen atmosphere at -78°C for 5 minutes. n-Butyllithium (2.5 M, 1.6 mL, 4 mmol) was slowly added dropwise to the reaction mixture, which was stirred at -78°C for 1 hour. Intermediate 30-1 (0.7 g, 1.6 mmol) dissolved in tetrahydrofuran (10 mL) was added dropwise, and the mixture was stirred at room temperature for 2 hours. After concentration, 6 N aqueous hydrochloric acid (20 mL) was added, and the mixture was stirred at 80°C for 16 hours. The reaction mixture was concentrated, and the product was purified by column chromatography to afford Compound 33 (0.6 g).

The ¹H-NMR data of the obtained Compound 33 is as follows:
¹H-NMR (400 MHz, DMSO-d₆) δ 8.08 (s, 1H), 7.33 (s, 1H), 7.30 (s, 1H), 6.80 (s, 1H), 6.75 (s, 1H), 6.71 (s, 1H), 3.88-3.49 (m, 6H), 3.09-2.86 (m, 3H), 3.09-3.06 (m, 2H), 2.82-2.76 (m, 2H), 2.21-1.85 (m, 2H), 2.06 (s, 3H), 1.96 (s, 6H), 1.75-1.50 (m, 2H), 1.34 (t, 3H, J = 6.8 Hz), 1.30 (s, 3H), 1.21 (d, 3H, J = 6.4 Hz), 1.16 (s, 3H).

### Synthesis Example 34. synthesis of Compound 34

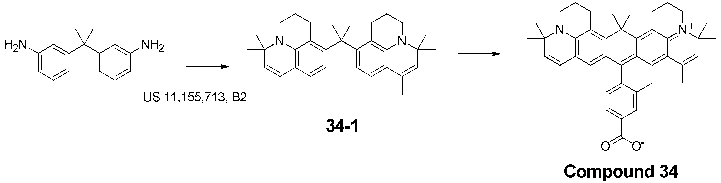

### synthesis of Intermediate 34-1

Intermediate 34-1 was synthesized with reference to US 11,155,713, B2.

### synthesis of Compound 34

A 250 mL single-neck flask was charged with Intermediate 34-1 (12.0 g, 0.0257 mol), 4-formyl-3-methylbenzoic acid (4.22 g, 0.0257 mol), and 70% sulfuric acid (20 mL), and the mixture was stirred at 130°C for 3 hours. After cooling, the reaction mixture was poured into cold water and neutralized. The resulting solid was collected by filtration. The solid was dried and refluxed in methanol (1 L) containing chloranil (3.1 g, 0.0128 mol) for 1 hour, cooled, and concentrated. The resulting solid was filtered and vacuum-dried at 60°C to afford Compound 34 (1.3 g).

The ¹H-NMR data of the obtained Compound 34 is as follows:
¹H-NMR (400 MHz, DMSO-d₆) δ 8.09 (s, 1H), 7.95 (d, 1H, J = 7.8 Hz), 7.35 (d, 1H, J = 7.8 Hz), 6.65 (s, 2H), 5.31 (s, 2H), 3.89-3.52 (m, 4H), 3.56-3.27 (m, 2H), 3.09-3.06 (m, 2H), 2.82-2.76 (m, 2H), 2.20-1.84 (m, 2H), 2.06 (s, 3H), 1.96 (s, 6H), 1.88 (s, 6H), 1.21 (s, 12H).

### Synthesis Example 35. synthesis of Compound 35

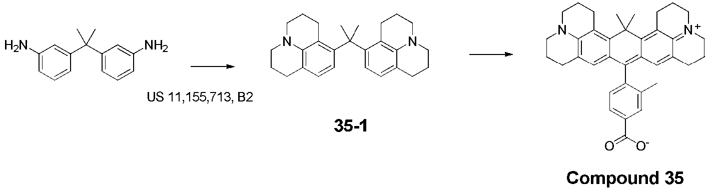

### synthesis of Intermediate 35-1

Intermediate 35-1 was synthesized with reference to US 11,155,713, B2.

### synthesis of Compound 35

A 250 mL single-neck flask was charged with Intermediate 35-1 (7.0 g, 0.018 mol), 4-formyl-3-methylbenzoic acid (2.95 g, 0.018 mol), and 70% sulfuric acid (100 mL), and the mixture was stirred at 130°C for 3 hours. After cooling, the reaction mixture was poured into cold water and neutralized. The resulting solid was filtered and dried. The dried solid was refluxed in methanol (1 L) containing chloranil (1.1 g, 0.005 mol) for 1 hour, cooled, and concentrated. The resulting solid was filtered and vacuum-dried at 60°C to afford Compound 35 (1.6 g).

The ¹H-NMR data of the obtained Compound 35 is as follows:
¹H-NMR (400 MHz, DMSO-d₆) δ 8.03 (s, 1H), 7.78 (d, 1H, J = 7.60 Hz), 7.30 (d, 1H, J = 8.00 Hz), 6.52 (s, 2H), 3.45-3.23 (m, 8H), 3.02-2.92 (m, 4H), 2.50-2.39 (m, 4H), 2.13 (s, 3H), 2.04 (s, 3H), 2.00-1.88 (m, 7H), 1.86 (m, 4H).

### Synthesis Example 36. synthesis of Compound 36

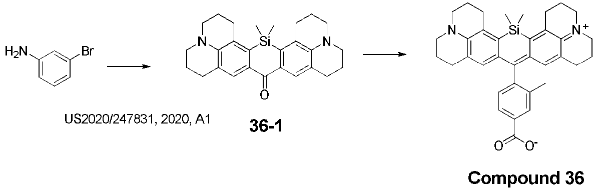

### synthesis of Intermediate 36-1

Intermediate 36-1 was synthesized with reference to US2020/247831, 2020, A1.

### synthesis of Compound 36

A 100 mL three-neck flask was charged with Intermediate 36-1 (2.58 g, 0.0102 mol) and tetrahydrofuran (26 mL), and the mixture was stirred under a nitrogen atmosphere at -78°C for 5 minutes. n-Butyllithium (2.5 M, 4 mL, 0.0102 mol) was added dropwise, and the mixture was stirred at -78°C for 1 hour. Intermediate 33-1 (1.5 g, 0.0034 mol) dissolved in tetrahydrofuran (30 mL) was added dropwise, and the mixture was stirred at room temperature for 2 hours. After concentration, 6 N aqueous hydrochloric acid (38 mL) was added, and the mixture was stirred at 80°C for 16 hours. The reaction mixture was concentrated, and the product was purified by column chromatography to afford Compound 36 (3.0 g).

The ¹H-NMR data of the obtained Compound 36 is as follows:
¹H-NMR (400 MHz, DMSO-d₆) δ 7.93 (s, 1H), 7.81 (d, J = 7.60 Hz, 1H), 7.20 (d, J = 8.00 Hz, 1H), 6.66 (s, 2H), 3.68-3.47 (m, 8H), 3.10-3.06 (m, 4H), 2.87-2.74 (m, 4H), 2.24-1.84 (m, 8H), 2.08 (s, 3H), 0.59 (s, 6H).

### Synthesis Example 37. synthesis of Compound 37

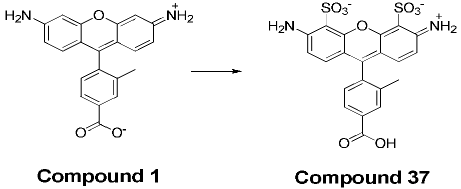

### synthesis of Compound 37

A 50 mL single-neck flask was charged with Compound 1 (1.0 g, 2.9 mmol) and 20% fuming sulfuric acid (6 mL), and the mixture was stirred at room temperature for 12 hours. The reaction mixture was poured into ethyl ether (60 mL) and vigorously stirred. The upper layer was discarded, and the product was dissolved in water and purified by reverse-phase chromatography to afford Compound 37 (0.2 g).

The ¹H-NMR data of the obtained Compound 37 is as follows:
¹H-NMR (400 MHz, D₂O) δ 8.67 (s, 1H), 8.29 (d, 1H, J = 8.0 Hz), 7.41 (d, 1H, J = 8.0 Hz), 7.00 (d, 2H, J = 8.0 Hz), 6.79 (d, 2H, J = 8.0 Hz), 2.07 (s, 3H).

### Synthesis Example 38. synthesis of Compound 38

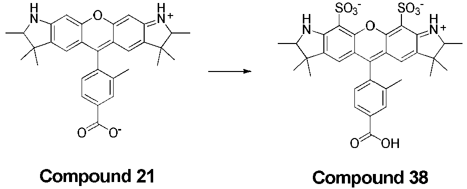

### synthesis of Compound 38

A 50 mL single-neck flask was charged with Compound 21 (1.0 g, 2.1 mmol) and 20% fuming sulfuric acid (6 mL), and the mixture was stirred at room temperature for 12 hours. The reaction mixture was poured into ethyl ether (60 mL) and vigorously stirred. The upper layer was discarded, and the product was dissolved in water and purified by reverse-phase chromatography to afford Compound 38 (0.5 g).

The ¹H-NMR data of the obtained Compound 38 is as follows:
¹H-NMR (400 MHz, D₂O) δ 8.65 (s, 1H), 8.27 (d, 1H, J = 7.8 Hz), 7.37 (d, 1H, J = 7.8 Hz), 6.85 (s, 2H), 3.83 (q, 2H, J = 6.0 Hz), 2.06 (s, 3H), 1.19 (s, 12H), 1.06 (s, 6H).

### Synthesis Example 39. synthesis of Compound 39

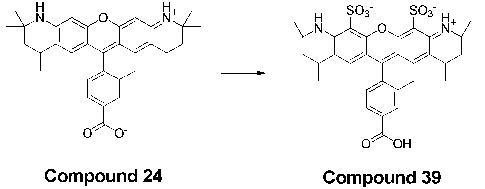

### synthesis of Compound 39

A 50 mL single-neck flask was charged with Compound 24 (1.0 g, 1.9 mmol) and 20% fuming sulfuric acid (6 mL), and the mixture was stirred at room temperature for 12 hours. The reaction mixture was poured into ethyl ether (60 mL) and vigorously stirred. The upper layer was discarded, and the product was dissolved in water and purified by reverse-phase chromatography to afford Compound 39 (0.2 g).

The ¹H-NMR data of the obtained Compound 39 is as follows:
¹H-NMR (400 MHz, D₂O) δ 8.60 (s, 1H), 8.17 (d, 1H, J = 7.8 Hz), 7.35 (d, 1H, J = 7.8 Hz), 6.75 (s, 2H), 2.93 (m, 2H), 2.09 (s, 3H), 1.78-1.57 (m, 4H), 1.33 (s, 6H), 1.26 (d, 6H, J = 6.4 Hz), 1.17 (s, 6H).

### Synthesis Example 40. synthesis of Compound 40

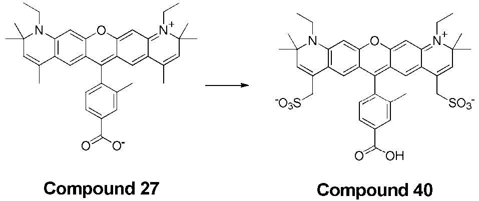

### synthesis of Compound 40

A 50 mL single-neck flask was charged with Compound 27 (1.0 g, 1.8 mmol) and 20% fuming sulfuric acid (6 mL), and the mixture was stirred at room temperature for 12 hours. The reaction mixture was poured into ethyl ether (60 mL) and vigorously stirred. The upper layer was discarded, and the product was dissolved in water and purified by reverse-phase chromatography to afford Compound 40 (0.3 g).

The ¹H-NMR data of the obtained Compound 40 is as follows:
¹H-NMR (400 MHz, D₂O) δ 8.67 (s, 1H), 8.13 (d, 1H, J = 7.8 Hz), 7.33 (d, 1H, J = 7.8 Hz), 6.98 (s, 2H), 6.88 (s, 2H), 5.33 (s, 2H), 3.78-3.54 (m, 4H), 2.08 (s, 3H), 2.03 (m, 4H), 1.33 (t, 6H, J = 6.8 Hz), 1.20 (s, 12H).

### Synthesis Example 41. synthesis of Compound 41

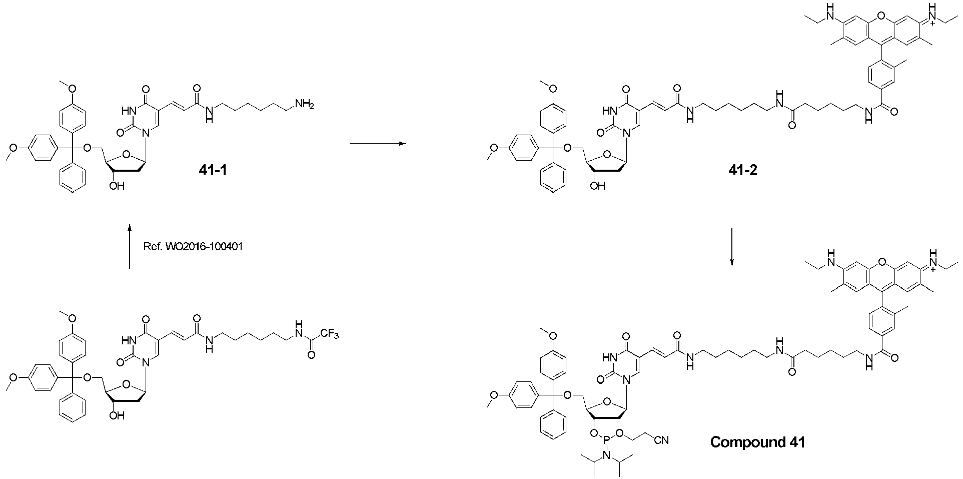

### synthesis of Compound 41

Intermediate 41-1 was synthesized with reference to WO2016/100401.

### synthesis of Intermediate 41-2

A 100 mL single-neck flask was charged with Intermediate 41-1 (2.21 g, 0.0032 mol), Compound 10 (2.0 g, 0.0026 mol), triethylamine (1.1 mL, 0.0079 mol), tetrahydrofuran (30 mL), and dichloromethane (30 mL). The mixture was stirred at room temperature for 1 hour. Water was added, and the mixture was vigorously stirred and extracted with dichloromethane. Sodium sulfate was added, stirred for 5 minutes, and filtered. The filtrate was concentrated and purified by column chromatography to afford Intermediate 41-2.

### synthesis of Compound 41

A 50 mL single-neck flask was charged with Intermediate 41-2 (1.0 g, 0.0007 mol), 2-cyanoethyl N,N'-diisopropylchlorophosphoramidite (0.23 g, 0.001 mol), triethylamine (1.1 mL, 0.0022 mol), and dichloromethane (15 mL). The mixture was stirred at room temperature for 1 hour. Water was added, and the mixture was vigorously stirred and extracted with dichloromethane. Sodium sulfate was added, stirred for 5 minutes, and filtered. The filtrate was concentrated and purified by column chromatography to afford Compound 41 (0.5 g).

The ¹H-NMR data of the obtained Compound 41 is as follows:
¹H-NMR (400 MHz, CDCl₃) δ 8.68 (m, 1H), 8.09-7.77 (m, 6H), 7.48-7.03 (m, 13H), 6.95 (s, 2H), 6.87-6.82 (m, 6H), 6.21-6.11 (m, 1H), 4.47 (m, 1H), 4.26 (m, 1H), 3.71 (m, 8H), 3.66-3.43 (m, 7H), 3.27-3.02 (m, 6H), 2.76-2.61 (m, 2H), 2.16-2.03 (m, 19H), 1.81 (m, 2H), 1.41 (m, 4H), 1.26 (m, 11H), 1.17-0.96 (m, 15H).

### Synthesis Example 42. synthesis of Compound 42

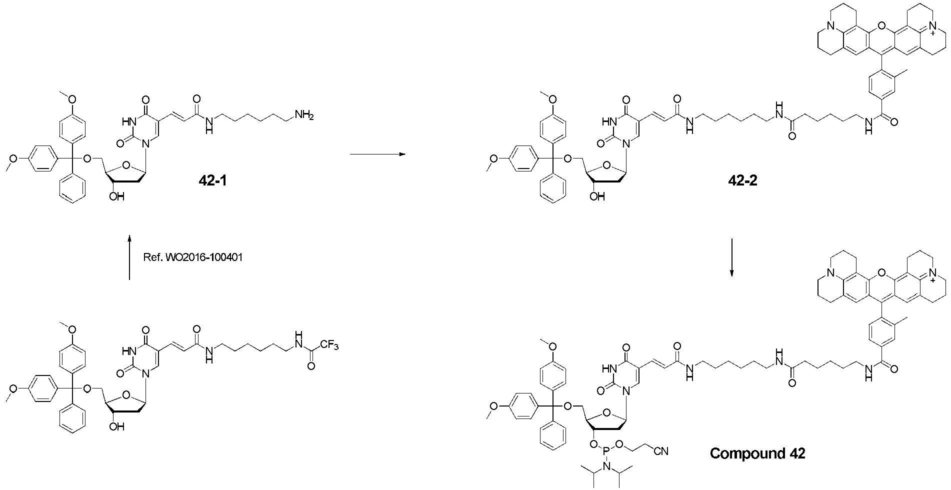

### synthesis of Compound 42

Intermediate 42-1 was synthesized with reference to WO2016/100401.

### synthesis of Intermediate 42-2

A 250 mL single-neck flask was charged with Intermediate 42-1 (3.83 g, 0.0055 mol), Compound 28 (3.8 g, 0.0046 mol), triethylamine (1.7 mL, 0.0114 mol), tetrahydrofuran (57 mL), and dichloromethane (57 mL). The mixture was stirred at room temperature for 2 hours. Water was added, and the mixture was vigorously stirred and extracted with dichloromethane. Sodium sulfate was added, stirred for 5 minutes, and filtered. The filtrate was concentrated and purified by column chromatography to afford Intermediate 42-2.

### synthesis of Compound 42

A 50 mL single-neck flask was charged with Intermediate 42-2 (1.0 g, 0.0007 mol), 2-cyanoethyl N,N'-diisopropylchlorophosphoramidite (0.4 g, 0.0017 mol), N-methylmorpholine (0.2 mL, 0.00176 mol), and dichloromethane (20 mL). The mixture was stirred at room temperature for 1 hour. Water was added, and the mixture was vigorously stirred and extracted with dichloromethane. Sodium sulfate was added, stirred for 5 minutes, and filtered. The filtrate was concentrated and purified by column chromatography to afford Compound 42 (0.6 g).

The ¹H-NMR data of the obtained Compound 42 is as follows:
¹H-NMR (400 MHz, DMSO-d₆) δ 11.60 (s, 1H), 8.60-8.70 (m, 1H), 7.80-8.10 (m, 5H), 7.08-7.40 (m, 13H), 6.80-6.88 (m, 4H), 6.56 (s, 2H), 6.10-6.20 (m, 1H), 4.40 (s, 1H), 3.95-4.05 (m, 1H), 3.72 (s, 7H), 3.40-3.60 (m, 11H), 3.32-3.33 (m, 4H), 3.00-2.18 (m, 8H), 2.71-2.80 (m, 1H), 2.59-2.70 (m, 5H), 2.15-2.22 (m, 2H), 1.98-2.10 (m, 9H), 1.72-1.91 (m, 6H), 1.31-1.42 (m, 4H), 1.15-1.30 (m, 4H), 1.08-1.15 (m, 12H), 0.97 (d, 2H, J = 6.80 Hz).

### Preparation Example 1. Synthesis of Dual-Labeled Probes (Oligonucleotides)

Using Universal UnyLinker Support (Chemgene, 500 Å), the forward primer 5'-SEQ 1-3' and reverse primer 5'-SEQ 2-3' for BQCV (black queen cell virus) were each synthesized on a 1 µmol scale, and the primers were cleaved from the support through a deprotection process using ammonium hydroxide. The resulting primers were purified by RP-HPLC.

First, single-labeled probes, 5'-SEQ 3-Quencher 1-3' and 5'-SEQ 3-Quencher 2-3', were synthesized using Quencher 1-CPG and Quencher 2-CPG (synthesized with reference to Korean Patent Publication No. 10-2019-0062162 and 10-2020-0067733). Then, dual-labeled probes were synthesized by labeling the 5' terminus with the compounds defined herein (Compound 3, Compound 22, Compound 30, and Compound 35) as reporters in phosphoramidite form, as well as commercially available fluorescent dyes Cy5, ROX, HEX, and FAM, using a MerMade^{™} 48X DNA Synthesizer.

The structures of Quencher 1 and Quencher 2 used for synthesizing the dual-labeled probes are shown below.

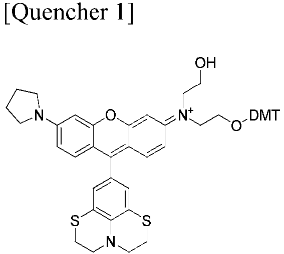

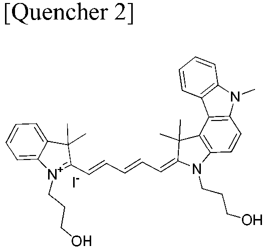

The synthesized dual-labeled probes were cleaved from CPG and purified by RP-HPLC. The forms of the synthesized dual-labeled probes are shown in Table 1 below.

**[Table 1]**

| Classification | Dual-Labeled Probe |
|---|---|
| EX 1-1 | 5'-Dye 35-SEQ 3-Quencher 2-3' |
| EX 1-2 | 5'-Cy5-SEQ 3-Quencher 2-3' |
| EX 1-3 | 5'-Dye 30-SEQ 3-Quencher 2-3' |
| EX 1-4 | 5'-ROX-SEQ 3-Quencher 2-3' |
| EX 1-5 | 5'-Dye 22-SEQ 3-Quencher 1-3' |
| EX 1-6 | 5'-HEX-SEQ 3-Quencher 1-3' |
| EX 1-7 | 5'-Dye 3-SEQ 3-Quencher 1-3' |
| EX 1-8 | 5'-FAM-SEQ 3-Quencher 1-3' |

| | |
|---|---|
| *SEQ 1 : GCGGGAGATGATATGGACTT (Real-Time PCR forward primer for BQCV (black queen cell virus) plasmid DNA) *SEQ 2 : CCGTCTGAGATGCATGAATAC (Real-Time PCR reverse primer for BQCV (black queen cell virus) plasmid DNA) *SEQ 3 : CCATCTTTATCGGTACGCCGCCC (Real-Time PCR dual labeled probe for BQCV(black queen cell virus) plasmid DNA) | |

### Preparation Example 2. Synthesis of Dual-Labeled Probes (Oligonucleotides)

Using Universal UnyLinker Support (Chemgene, 500 Å), the forward primer 5'-SEQ 1-3' and reverse primer 5'-SEQ 2-3' for BQCV (black queen cell virus) were each synthesized on a 1 µmol scale, and the primers were cleaved from the support through a deprotection process using ammonium hydroxide. The resulting primers were purified by RP-HPLC.

First, single-labeled probes, 5'-SEQ 3-Quencher 1-3' and 5'-SEQ 3-Quencher 2-3', were synthesized using Quencher 1-CPG and Quencher 2-CPG (synthesized with reference to Korean Patent Publication Nos. 10-2019-0062162 and 10-2020-0067733). Then, dual-labeled probes were synthesized using a MerMade^{™} 48X DNA Synthesizer by labeling the 5' terminus with the compounds defined herein (Compound 3, Compound 22, Compound 30, and Compound 35) as reporters in phosphoramidite form, and with Dye A, Dye B, Dye C, and Dye D designed as control dyes.

The structures of Dye A to Dye D, designed as control dyes, are shown below.

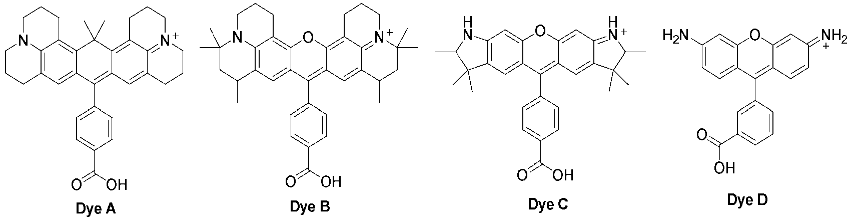

The synthesized dual-labeled probes were cleaved from CPG and purified by RP-HPLC. The forms of the synthesized dual-labeled probes are shown in Table 2 below.

**[Table 2]**

| Classification | Dual-Labeled Probe |
|---|---|
| EX 2-1 | 5'-Dye 35-SEQ 3-Quencher 2-3' |
| EX 2-2 | 5'-Dye A-SEQ 3-Quencher 2-3' |
| EX 2-3 | 5'-Dye 30-SEQ 3-Quencher 2-3' |
| EX 2-4 | 5'-Dye B-SEQ 3-Quencher 2-3' |
| EX 2-5 | 5'-Dye 22-SEQ-3-Quencher 1-3' |
| EX 2-6 | 5'-Dye C-SEQ 3-Quencher 1-3' |
| EX 2-7 | 5'-Dye 3-SEQ 3-Quencher 1-3' |
| EX 2-8 | 5'-Dye D-SEQ 3-Quencher 1-3' |

| | |
|---|---|
| *SEQ 1 : GCGGGAGATGATATGGACTT (Real-Time PCR forward primer for BQCV (black queen cell virus) plasmid DNA) *SEQ 2 : CCGTCTGAGATGCATGAATAC (Real-Time PCR reverse primer for BQCV (black queen cell virus) plasmid DNA) *SEQ 3 : CCATCTTTATCGGTACGCCGCCC (Real-Time PCR dual labeled probe for BQCV(black queen cell virus) plasmid DNA) | |

### Experimental Example 1. Real-Time PCR experiment using dual-labeled probe

Real-Time PCR for BQCV (black queen cell virus) plasmid DNA was performed twice using each dual-labeled probe synthesized according to Preparation Example 1, with the composition shown in Table 3 below (CFX-96^{™} Touch, Bio-Rad). PCR Protocol : 95 °C, 3min - [95 °C, 10s - 60 °C, 30s] x 45cycles

**[Table 3]**

| Classification | Content (µl) |
|---|---|
| (Bioline)SensiFAST^{™} Probe No-ROX Mix (2X) | 10 |
| BQCV plasmid DNA (5000 copies/µl) | 3 |
| BQCV F/R primer mix (5 pmole/µl) | 1 |
| BQCV Dual-labeled probe (5 pmole/µl) | 1 |
| DEPC Water | 5 |

The results of the Real-Time PCR are shown in Table 4.

**[Table 4]**

| Classification | DNA (fg/ul) | Copy number (Log) | Ct | Norm. RFU |
|---|---|---|---|---|
| EX 1-1 | 1000 | 5.48 | 22.49 | 1.00 |
| | 100 | 4.48 | 25.98 | |
| | 10 | 3.48 | 29.08 | |
| | 1 | 2.48 | 32.57 | |
| EX 1-2 | 1000 | 5.48 | 24.66 | 0.36 |
| | 100 | 4.48 | 27.92 | |
| | 10 | 3.48 | 31.14 | |
| | 1 | 2.48 | 34.64 | |
| EX 1-3 | 10000 | 6.48 | 20.43 | 1.00 |
| | 1000 | 5.48 | 23.80 | |
| | 100 | 4.48 | 27.20 | |
| | 10 | 3.48 | 30.47 | |
| EX 1-4 | 10000 | 6.48 | 22.46 | 0.37 |
| | 1000 | 5.48 | 25.76 | |
| | 100 | 4.48 | 29.12 | |
| | 10 | 3.48 | 32.51 | |
| EX 1-5 | 1000 | 5.48 | 21.07 | 1.00 |
| | 100 | 4.48 | 24.22 | |
| | 10 | 3.48 | 27.60 | |
| | 1 | 2.48 | 31.00 | |
| EX 1-6 | 1000 | 5.48 | 21.69 | 0.62 |
| | 100 | 4.48 | 24.76 | |
| | 10 | 3.48 | 28.17 | |
| | 1 | 2.48 | 31.78 | |
| EX 1-7 | 1000 | 5.48 | 22.11 | 1.00 |
| | 100 | 4.48 | 25.47 | |
| | 10 | 3.48 | 28.75 | |
| | 1 | 2.48 | 32.18 | |
| EX 1-8 | 1000 | 5.48 | 22.75 | 0.70 |
| | 100 | 4.48 | 26.17 | |
| | 10 | 3.48 | 29.56 | |
| | 1 | 2.48 | 32.91 | |

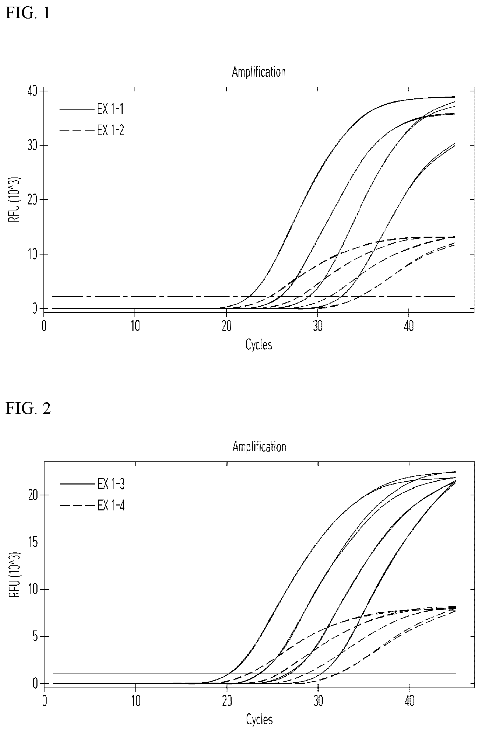

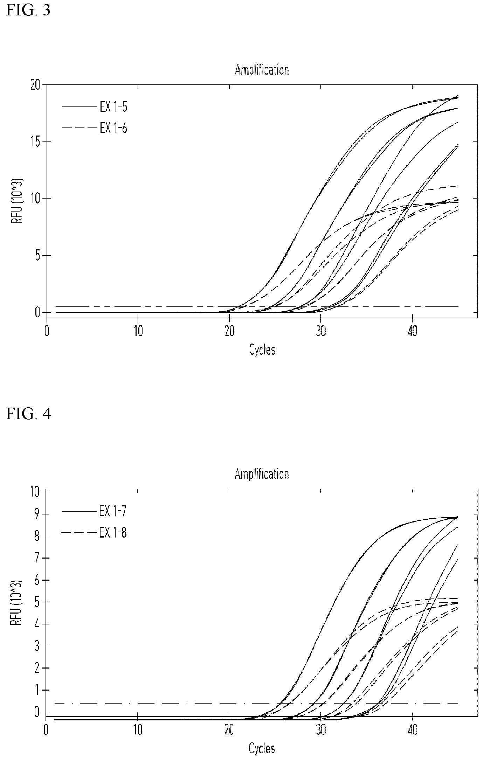

Referring to Table 4 and Figures 1 to 4, in the cases of EX 1-1, 1-3, 1-5, and 1-7, in which the compound defined herein was used as a reporter, relatively lower Ct and higher RFU were exhibited compared to EX 1-2, 1-4, 1-6, and 1-8, in which commercially available fluorescent substances were used as reporters.

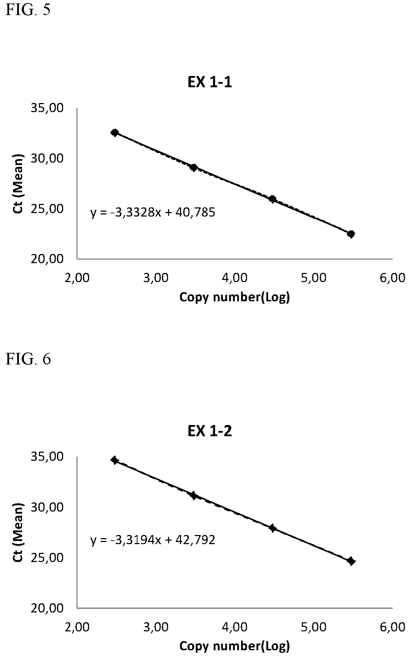

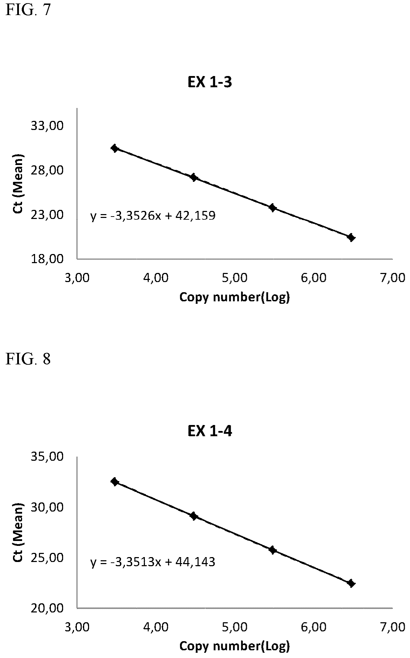

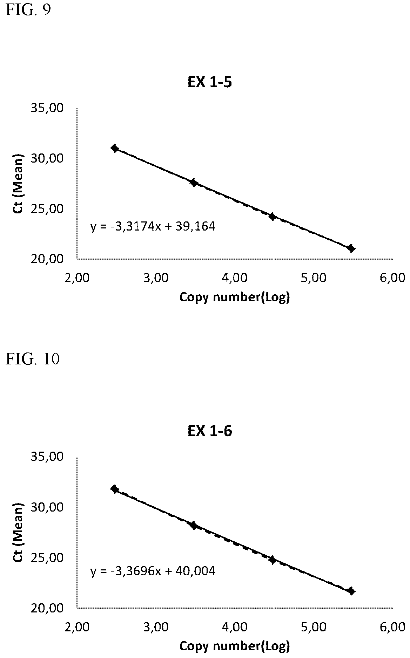

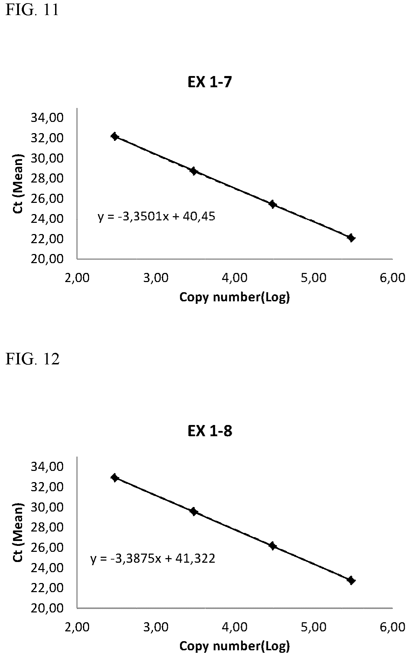

In addition, referring to the results of Figures 5 to 12, which present standard curves prepared using Ct values and concentrations, it can be confirmed that the R² values of EX 1-1, 1-3, 1-5, and 1-7 are all 0.98 or higher, maintaining linearity.

Further, considering the molecular diagnostic aspect, since the limit of detection (LoD) of the compound defined herein is lower than that of the conventional commercially available fluorescent substances, when the compound defined herein is used as a reporter (fluorescent substance) of a dual-labeled probe, it is expected that the detectability and accuracy will be higher than those of the commercially available fluorescent substances even when the target gene is present in a sample at a relatively low concentration.

Accordingly, the reporter for nucleic acid labeling defined herein can be applied to the existing nucleic acid labeling and detection field (for example, PCR experiments, etc.) or sufficiently replace the existing commercially available fluorescent substances.

### Experimental Example 2. Real-Time PCR experiment using dual-labeled probe

Real-Time PCR for BQCV (black queen cell virus) plasmid DNA was performed twice using each dual-labeled probe synthesized according to Preparation Example 2, with the composition shown in Table 5 below (CFX-96^{™} Touch, Bio-Rad). PCR Protocol : 95 °C, 3min - [95 °C, 10s - 60 °C, 30s] x 45cycles

**[Table 5]**

| Classification | Content (µl) |
|---|---|
| (Bioline)SensiFAST^{™} Probe No-ROX Mix (2X) | 10 |
| BQCV plasmid DNA (5000 copies/µl) | 3 |
| BQCV F/R primer mix (5 pmole/µl) | 1 |
| BQCV Dual-labeled probe (5 pmole/µl) | 1 |
| DEPC Water | 5 |

The results of the Real-Time PCR are shown in Table 6.

**[Table 6]**

| Classification | DNA (fg/ul) | Copy number (Log) | Ct | Norm. RFU |
|---|---|---|---|---|
| EX 2-1 | 100 | 4.48 | 25.61 | 1.00 |
| | 10 | 3.48 | 29.16 | |
| | 1 | 2.48 | 32.37 | |
| | 0.1 | 1.48 | 36.31 | |
| EX 2-2 | 100 | 4.48 | 26.69 | 0.57 |
| | 10 | 3.48 | 30.20 | |
| | 1 | 2.48 | 33.56 | |
| | 0.1 | 1.48 | 36.74 | |
| EX 2-3 | 1000 | 5.48 | 22.04 | 1.00 |
| | 100 | 4.48 | 25.43 | |
| | 10 | 3.48 | 28.94 | |
| | 1 | 2.48 | 32.38 | |
| EX 2-4 | 1000 | 5.48 | 22.53 | 0.79 |
| | 100 | 4.48 | 26.13 | |
| | 10 | 3.48 | 29.36 | |
| | 1 | 2.48 | 32.77 | |
| EX 2-5 | 1000 | 5.48 | 21.63 | 1.00 |
| | 100 | 4.48 | 25.02 | |
| | 10 | 3.48 | 28.23 | |
| | 1 | 2.48 | 31.5 | |
| EX 2-6 | 1000 | 5.48 | 22.17 | 0.62 |
| | 100 | 4.48 | 25.66 | |
| | 10 | 3.48 | 29.02 | |
| | 1 | 2.48 | 32.56 | |
| EX 2-7 | 1000 | 5.48 | 22.28 | 1.00 |
| | 100 | 4.48 | 25.75 | |
| | 10 | 3.48 | 29.18 | |
| | 1 | 2.48 | 32.64 | |
| EX 2-8 | 1000 | 5.48 | 22.52 | 0.86 |
| | 100 | 4.48 | 26.04 | |
| | 10 | 3.48 | 29.42 | |
| | 1 | 2.48 | 32.95 | |

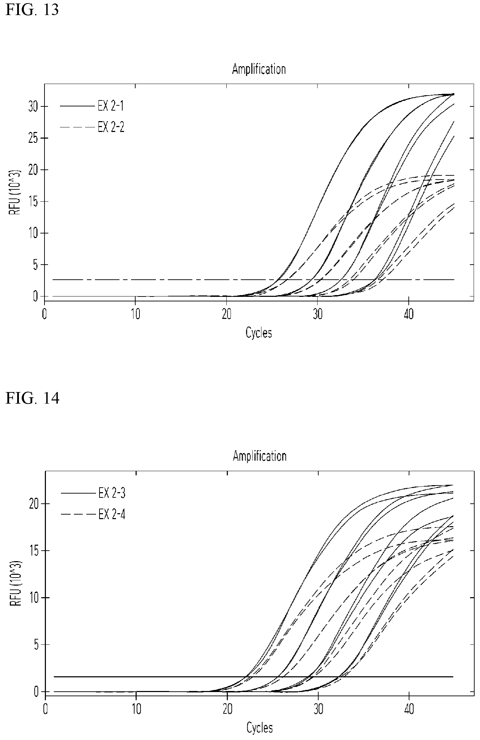

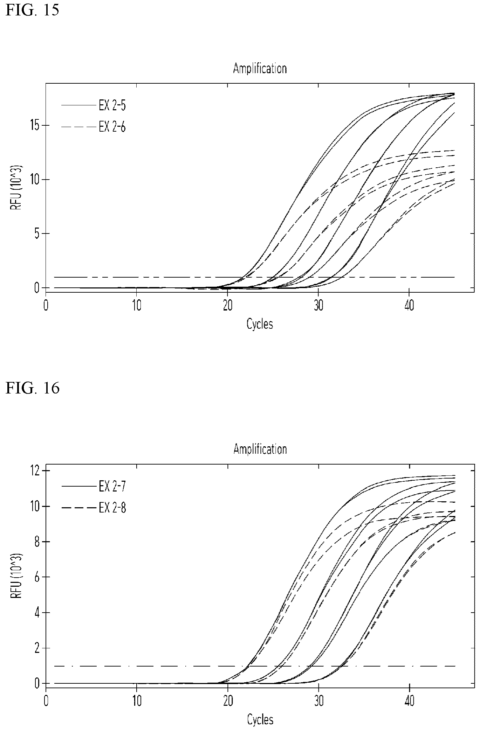

Referring to Table 6 and Figures 13 to 16, in the cases of EX 2-1, 2-3, 2-5, and 2-7, in which the compound defined herein was used as a reporter, relatively lower Ct and higher RFU were exhibited compared to Dyes A to D, in which A1 and A2 in Chemical Formula 1 are both hydrogen.

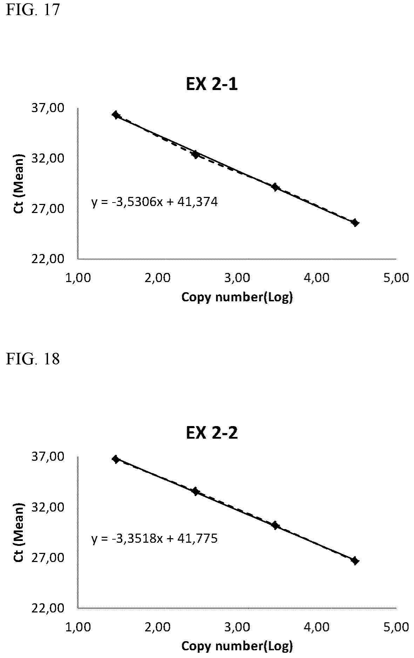

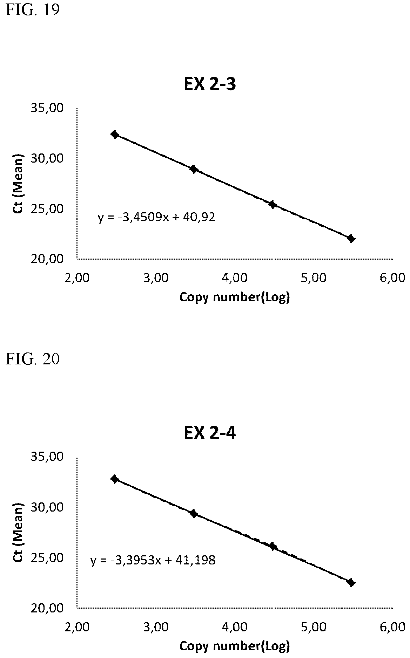

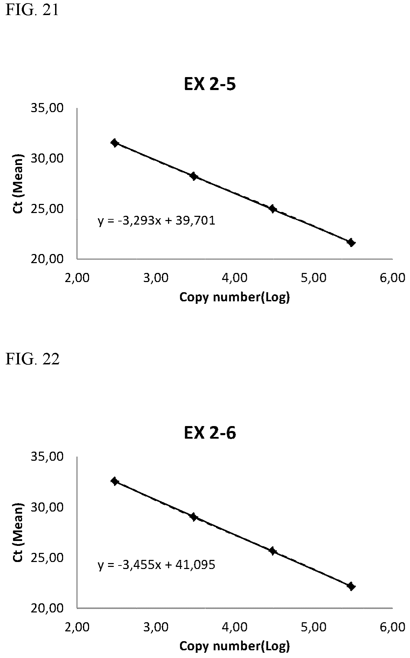

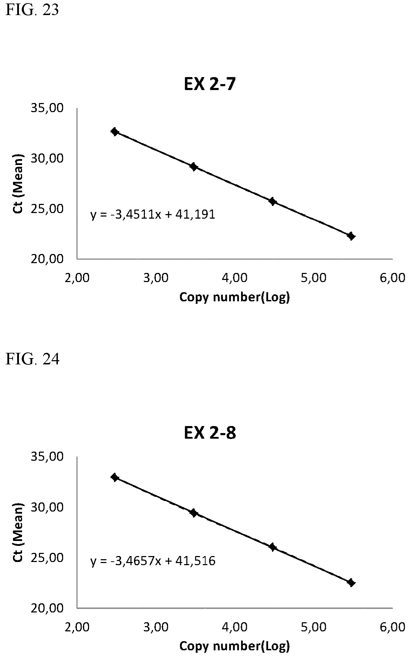

In addition, referring to the results of Figures 17 to 24, which present standard curves prepared using Ct values and concentrations, it can be confirmed that the R² values of EX 2-1, 2-3, 2-5, and 2-7 are all 0.98 or higher, maintaining linearity. From these results, it can be confirmed that the utility as a reporter is enhanced when at least one of A1 and A2 in Chemical Formula 1 is not hydrogen.

Accordingly, the compound defined herein is expected to be applicable as a reporter for nucleic acid labeling in the existing nucleic acid labeling and detection field (for example, PCR experiments, etc.) or to sufficiently replace the existing commercially available fluorescent substances.

### Experimental Example 3. Evaluation of Optical Properties of Reporter for Nucleic Acid Labeling

**[Table 7]**

| Classfication | UV (nm) | PL (nm) | Absorption coefficient (ε) | Quantum Yield (Φ) |
|---|---|---|---|---|
| Dye B | 596 | 620 | 80,000 | 0.60 |
| Compound 30 | 598 | 620 | 87,000 | 0.89 |

Referring to Table 7, in the case of xanthene-based fluorescent dyes in which a phenyl group is substituted, such as Dye B used in Production Example 2, when a substituent is present at the meta or para position, it can be confirmed that a functional defect occurs in which the fluorescent properties of the dye are impaired due to the failure to block rotation of the phenyl group.

On the other hand, it can be confirmed that a compound having a substituent at the ortho position, such as Compound 30, induces steric hindrance to block the rotation of the phenyl group, and as a result, significantly improves fluorescent properties.

In addition, due to the substituent effect at the ortho position, the compound has an effect of fine-tuning the absorption or emission spectra, which provides the possibility of developing fluorescent compounds in various wavelength ranges.

Although several embodiments of the present invention have been described above, those skilled in the art can modify and change the present invention in various ways by adding, changing, or deleting components without departing from the spirit of the present invention as set forth in the claims, and such modifications are also included within the scope of the rights of the present invention.

## Claims

1. A compound represented by Chemical Formula 1 below: 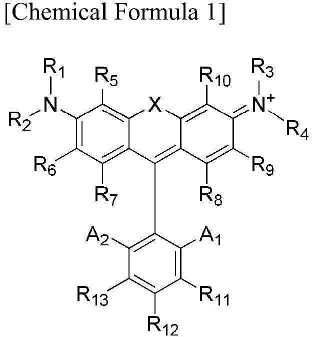 wherein,
R₁ to R₄ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C3-C40 cycloalkyloxy, optionally substituted C5-C40 aryloxy, optionally substituted C2-C40 heteroaryloxy, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, Rₓ and Rₛ,
R₅ to R₁₃ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C40 cycloalkyloxy, optionally substituted C5-C40 aryloxy, optionally substituted C2-C40 heteroaryloxy, optionally substituted C5-C50 aryl, optionally substituted C2-C50 heteroaryl, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, Rₓ and Rₛ,
optionally, any two adjacent groups among R₁ to R₁₃ are bonded to each other to form an optionally substituted aliphatic ring, an optionally substituted aromatic ring, or an optionally substituted aliphatic-aromatic mixed ring,
A₁ and A₂ are each independently selected from hydrogen, halogen, optionally substituted C1-C10 alkyl, optionally substituted C3-C10 cycloalkyl, optionally substituted C1-C10 heterocycloalkyl, optionally substituted C1-C10 heteroalkyl, optionally substituted C2-C10 alkenyl, optionally substituted C2-C10 alkynyl, optionally substituted C1-C10 alkoxy, optionally substituted C5-C40 aryloxy, and cyano, provided that A₁ and A₂ are not simultaneously hydrogen,
Rₓ is a reactive group, or a group comprising a backbone including a hydrocarbon having 1 to 40 carbon atoms to which at least one reactive group is bound,
the reactive group being selected from carboxyl, carboxyl derivatives, carboxylate (-CO₂⁻), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkyne, haloacetamide, imido ester, glycol, halotriazine, hydrazine, acyl halide, alkyl halide, and aryl halide,
Rₛ is a carrier molecule, or a group comprising a backbone including a hydrocarbon having 1 to 40 carbon atoms to which at least one carrier molecule is bound,
at least one of R₁ to R₁₃ is Rₓ or Rₛ,
X is O, S, CRₐR_{b}, NRₐ, or SiRₐR_{b},
and Rₐ and R_{b} are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted C5-C50 aryl, and optionally substituted C2-C50 heteroaryl, or Rₐ and R_{b} are bonded to each other to form an optionally substituted aliphatic ring, an optionally substituted aromatic ring, or an optionally substituted aliphatic-aromatic mixed ring.

2. The compound of claim 1, wherein Rₓ or Rₛ comprises the following Structure 1: 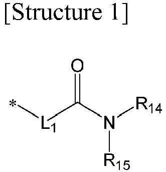 wherein
* denotes a position at which Structure 1 is bonded to the compound represented by Chemical Formula 1,
R₁₄ and R₁₅ are each independently selected from hydrogen, optionally substituted C1-C20 alkyl, optionally substituted C1-C20 heteroalkyl, optionally substituted C2-C20 alkenyl, optionally substituted C2-C20 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C20 cycloalkyloxy, optionally substituted C5-C20 aryloxy, optionally substituted C2-C20 heteroaryloxy, optionally substituted C5-C20 aryl, optionally substituted C2-C20 heteroaryl, thiol, optionally substituted C5-C20 aralkyl, optionally substituted C1-C20 alkylthio, optionally substituted C5-C20 arylthio, optionally substituted C3-C20 cycloalkylthio, optionally substituted C2-C20 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, and thioether,
or R₁₄ and R₁₅ are bonded to each other to form an optionally substituted aliphatic ring, an optionally substituted aromatic ring, or an optionally substituted aliphatic-aromatic mixed ring,
and L₁ is a single bond or a linker comprising a hydrocarbon having 1 to 20 carbon atoms.

3. The compound of claim 2, wherein at least one of R₁₄ and R₁₅ comprises a reactive group selected from carboxyl, carboxyl derivatives, carboxylate (-CO₂⁻), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkyne, haloacetamide, imido ester, glycol, halotriazine, hydrazine, acyl halide, alkyl halide, and aryl halide.

4. The compound of claim 1, wherein Rₓ or Rₛ comprises the following Structure 2: 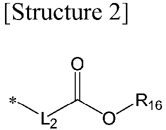 wherein
* denotes a position at which Structure 2 is bonded to the compound represented by Chemical Formula 1,
R₁₆ is selected from hydrogen, optionally substituted C1-C20 alkyl, optionally substituted C1-C20 heteroalkyl, optionally substituted C2-C20 alkenyl, optionally substituted C2-C20 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C20 cycloalkyloxy, optionally substituted C5-C20 aryloxy, optionally substituted C2-C20 heteroaryloxy, optionally substituted C5-C20 aryl, optionally substituted C2-C20 heteroaryl, thiol, optionally substituted C5-C20 aralkyl, optionally substituted C1-C20 alkylthio, optionally substituted C5-C20 arylthio, optionally substituted C3-C20 cycloalkylthio, optionally substituted C2-C20 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, and thioether,
and L₂ is a linker comprising a hydrocarbon having 1 to 20 carbon atoms.

5. The compound of claim 1, wherein at least one Rₓ or at least one Rₛ is present in the compound, and at least one Rₓ or at least one Rₛ is protected with a protecting group.

6. The compound of claim 1, wherein at least one of A₁ and A₂ is selected from optionally substituted C1-C3 alkyl and optionally substituted C1-C5 alkoxy.

7. The compound of claim 1, wherein at least one pair selected from R₁ and R₅, R₂ and R₆, R₃ and R₁₀, and R₄ and R₉ are bonded to each other to form an optionally substituted aliphatic ring, an optionally substituted aromatic ring, or an optionally substituted aliphatic-aromatic mixed ring.

8. The compound of claim 1, wherein at least one pair selected from R₁ and R₂, and R₃ and R₄ are bonded to each other to form an optionally substituted aliphatic ring, an optionally substituted aromatic ring, or an optionally substituted aliphatic-aromatic mixed ring.

9. A compound represented by Chemical Formula 2 below: 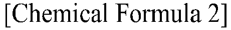 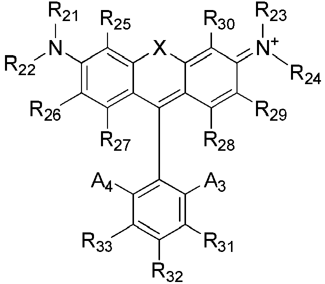 wherein
R₂₁ to R₂₄ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C3-C40 cycloalkyloxy, optionally substituted C5-C40 aryloxy, optionally substituted C2-C40 heteroaryloxy, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, and -L₃-R₃₆,
R₂₅ to R₃₃ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxy, oxido (-O⁻), optionally substituted C1-C40 alkoxy, optionally substituted C3-C40 cycloalkyloxy, optionally substituted C5-C40 aryloxy, optionally substituted C2-C40 heteroaryloxy, optionally substituted C5-C50 aryl, optionally substituted C2-C50 heteroaryl, thiol, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, sulfo group, optionally substituted ammonium, substituted sulfonate ester, optionally substituted sulfonamide, substituted ester, optionally substituted aminocarbonyl, nitroso (-N=O), halogen, optionally substituted silyl, optionally substituted amide, optionally substituted carbamate, phospho group, nitrile, acetal, ketal, optionally substituted amino, aminothiocarbonyl, aminocarbonyl-oxy, aminosulfonyl-oxy, amidino, sulfonyl-oxy, isonitrile, cyanate, imide, iminium, ether, thioether, and -L₃-R₃₆,
optionally, any two adjacent groups among R₂₁ to R₃₃ are bonded to each other to form an optionally substituted aliphatic ring, an optionally substituted aromatic ring, or an optionally substituted aliphatic-aromatic mixed ring,
A₃ and A₄ are each independently selected from hydrogen, halogen, optionally substituted C1-C10 alkyl, optionally substituted C3-C10 cycloalkyl, optionally substituted C1-C10 heterocycloalkyl, optionally substituted C1-C10 heteroalkyl, optionally substituted C2-C10 alkenyl, optionally substituted C2-C10 alkynyl, optionally substituted C1-C10 alkoxy, optionally substituted aryloxy, and cyano, provided that A₃ and A₄ are not simultaneously hydrogen,
X is O, S, CRₐR_{b}, NRₐ, or SiRₐR_{b},
Rₐ and R_{b} are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted C5-C50 aryl, and optionally substituted C2-C50 heteroaryl, or Rₐ and R_{b} are bonded to each other to form an optionally substituted aliphatic ring, an optionally substituted aromatic ring, or an optionally substituted aliphatic-aromatic mixed ring,
at least one of R₂₁ to R₃₃ is -L₃-R₃₆,
L₃ is a linker comprising a hydrocarbon having 1 to 40 carbon atoms,
R₃₆ is a nucleoside selected from Chemical Formulas 3 to 5: 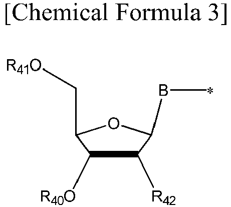 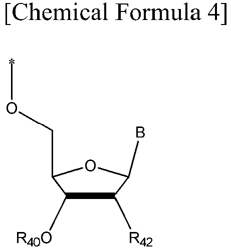 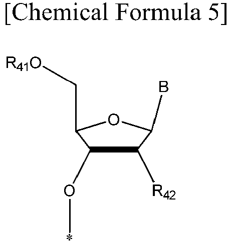 wherein
* denotes a position at which the nucleoside is bonded to L₃,
B is a nucleobase,
R₄₀ is selected from hydrogen, -P(OR₄₃)(N(R₄₄R₄₅)), and -L₄-R₄₆,
R₄₁ is an alcohol protecting group, hydrogen, or -P(OR₄₃)(N(R₄₄R₄₅)), or a nucleic acid or an optionally substituted support,
R₄₃ to R₄₅ are each independently selected from hydrogen, optionally substituted C1-C10 alkyl, and optionally substituted C1-C10 heteroalkyl,
L₄ is a single bond, or selected from an internucleotide phosphodiester bond, optionally substituted C1-C10 alkyl, and optionally substituted C1-C10 heteroalkyl,
R₄₆ is hydroxy, -P(OR₄₃)(N(R₄₄R₄₅)), or a nucleic acid or an optionally substituted support,
R₄₂ is selected from hydrogen, hydroxy, alkoxy, and -ORₚ,
and Rₚ is a protecting group.

10. The compound of claim 9, wherein the optionally substituted support or nucleic acid is a support or nucleic acid substituted with at least one selected from coumarin, cyanine, BODIPY, fluorescein, rhodamine, pyrene, carbopyronine, oxazine, xanthene, thioxanthene, acridine, derivatives thereof, and Chemical Formula 1.

11. The compound of any one of claims 1 to 10, wherein the compound is a labeling reporter.

12. A conjugate comprising the compound of any one of claims 1 to 10 as a labeling reporter.

13. The conjugate of claim 12, wherein the conjugate is a nucleotide conjugate.

14. The conjugate of claim 12, wherein the conjugate is a probe or a primer.

15. The conjugate of claim 12, wherein the conjugate further comprises a minor groove binder (MGB).

16. A conjugate comprising:
a compound of any one of claims 1 to 10; and
a quencher.

17. A conjugate comprising:
a compound of any one of claims 1 to 10; and
two or more quenchers.

18. The conjugate of claim 16, wherein the quencher is at least one selected from azo, coumarin, cyanine, BODIPY, fluorescein, rhodamine, pyrene, carbopyronine, oxazine, xanthene, thioxanthene, acridine, and derivatives thereof.

19. A composition for detecting nucleic acid comprising the conjugate of claim 12.

20. A support for detecting nucleic acid comprising:
a compound of any one of claims 1 to 10;
a support; and
a linker connecting the reporter and the support.

21. The support for nucleic acid detection of claim 20, wherein the support is glass, cellulose, nylon, acrylamide gel, dextran, polystyrene, or resin.

22. The support for nucleic acid detection of claim 20, wherein the linker is selected from optionally substituted C1-C30 alkyl, optionally substituted C3-C30 cycloalkyl, optionally substituted C2-C30 heteroalkyl, optionally substituted C2-C30 heterocycloalkyl, optionally substituted C2-C30 alkenyl, optionally substituted C5-C50 aryl, optionally substituted C2-C50 heteroaryl, amide (-CONH-), ester (-COO-), ketone (-CO-), nucleoside, and any combination thereof.

23. A method for detecting nucleic acid comprising:
(a) preparing a reaction mixture comprising a target nucleic acid, reagents necessary to amplify the target nucleic acid, and the nucleotide conjugate of claim 13;
(b) amplifying the target nucleic acid in the reaction mixture; and
(c) measuring fluorescence intensity of the reaction mixture.

24. The method of claim 23,
wherein step (b) comprises:
(b-1) extending, by a polymerase, the nucleotide conjugate hybridized to the target nucleic acid;
(b-2) separating a reporter and a quencher of the nucleotide conjugate from the target nucleic acid by exonuclease activity of the polymerase; and
(b-3) allowing the reporter separated from the quencher to emit fluorescence.

25. The method of claim 23,
further comprising:
(d) determining the amount of amplification of the target nucleic acid based on the fluorescence intensity measured in step (c).

26. The method of claim 23, wherein step (b) is performed by a method selected from strand displacement amplification (SDA), polymerase chain reaction (PCR), reverse transcription polymerase chain reaction (RT-PCR), real-time polymerase chain reaction, allele-specific polymerase chain reaction, ligase chain reaction (LCR), rolling circle amplification (RCA), isothermal multiple displacement amplification (IMDA), recombinase polymerase amplification (RPA), self-sustained sequence replication (3SR), single primer isothermal amplification (SPIA), multiple displacement amplification (MDA), whole genome amplification (WGA), cross-priming amplification (CPA), signal mediated amplification of RNA technology (SMART), transcription mediated amplification (TMA), nucleic acid sequence based amplification (NASBA), loop-mediated isothermal amplification (LAMP), and helicase dependent amplification (HDA).
